(19) Europäisches Patentamt
European Patent Office
Office européen des brevets

(11) EP 3 915 580 A1

(12) EUROPEAN PATENT APPLICATION

(43) Date of publication:
01.12.2021 Bulletin 2021/48

(51) Int Cl.:
A61K 39/395 (2006.01)    C07K 16/28 (2006.01)
C07K 16/30 (2006.01)

(21) Application number: 20177337.1

(22) Date of filing: 29.05.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Numab Therapeutics AG
8820 Wädenswil (CH)

(72) Inventor: The designation of the inventor has not
yet been filed

(74) Representative: Virnekäs, Bernhard
Wallinger Ricker Schlotter Tostmann
Patent- und Rechtsanwälte Partnerschaft mbB
Zweibrückenstrasse 5-7
80331 München (DE)

(54) MULTISPECIFIC ANTIBODY

(57) The present invention relates to a multispecific antibody comprising two antibody-based binding domains, which specifically binds to mesothelin (MSLN-BD); and at least one antibody-based binding domain, which specifically binds to CD3 (CD3-BD); wherein said multispecific antibody does not comprise an immunoglobulin Fc region polypeptide, and wherein each of said MSLN-BD binds to mesothelin (MSLN) with a monovalent dissociation constant ($K_D$) in the range of from 0.5 to 20 nM, when measured by SPR. The present invention further relates to nucleic acid sequence(s) encoding said multispecific antibody, vector(s) comprising said nucleic acid sequence(s), host cell(s) comprising said nucleic acid sequence(s) or said vector(s), and a method of producing said multispecific antibody. Additionally, the present invention relates to pharmaceutical compositions comprising said multispecific antibody and methods of use thereof.

## Figure 11:

**Description**

**FIELD OF THE INVENTION**

The present invention relates to a multispecific antibody comprising two antibody-based binding domains, which specifically bind to mesothelin (MSLN-BD); and at least one antibody-based binding domain, which specifically binds to CD3 (CD3-BD); wherein said multispecific antibody does not comprise an immunoglobulin Fc region polypeptide, and wherein each of said MSLN-BD binds to mesothelin (MSLN) with a monovalent dissociation constant ($K_D$) in the range of from 0.5 to 20 nM, when measured by SPR. The present invention further relates to nucleic acid sequence(s) encoding said multispecific antibody, vector(s) comprising said nucleic acid sequence(s), host cell(s) comprising said nucleic acid sequence(s) or said vector(s), and a method of producing said multispecific antibody. Additionally, the present invention relates to pharmaceutical compositions comprising said multispecific antibody and methods of use thereof.

**BACKGROUND OF THE INVENTION**

Cancer continues to pose a major unmet medical need, despite the considerable progress made in its treatment. Some of the most substantial progress made in cancer treatment in recent years has come with the advent of immunotherapies of various molecular classes, including, but not limited to: monoclonal antibodies (mAbs), bispecific antibodies (bsAbs), recombinant proteins, and chimeric antigen receptor-T cell (CAR-T cell) therapies. Such therapies induce anti-tumor immunity by: a) actively directing immune-effector cells to tumor-resident cells and/or b) stimulating immune-effector cells and/or c) relieving tumor-mediated immune-suppression. These immunotherapies commonly exploit the overexpression of specific antigens by tumor-resident cells (*e. g.*, malignant cells, cells of the tumor vasculature, stromal cells, immune cells, etc.) - as compared to extratumoral loci - to target their pharmacological activity to tumors. Among these antigens, tumor-associated antigens (TAAs) comprise cell-surface proteins selectively overexpressed by malignant cells. By binding to TAAs with high affinity, immunotherapies can restrict their immunomodulatory activity to immunological synapses between tumor cells and immune effector cells to a degree.

A common class of TAA-binding immunotherapies are mAbs that elicit anti-tumor immunity by opsonizing tumor-cells and triggering antibody-dependent cell-mediated cytotoxicity (ADCC) by Fcγ receptor (FcγR)-expressing cells, primarily natural killer (NK) cells. Other TAA-binding immunotherapies leverage cytotoxic T lymphocytes (CTLs) to induce targeted depletion of malignant cells, such as CAR-T cells as well as bsAbs that simultaneously engage the T cell antigen CD3 (TAA/CD3 bsAbs).

While the therapeutic utility of TAA-(re)directed CTLs and conventional TAA/CD3 bsAbs have been clinically validated, dose-limiting toxicities (DLTs) often preclude administration at maximally effective doses (MEDs) or lead to discontinuation of treatment, resulting in limited efficacy.

One reason for the DLTs is that conventional TAA/CD3 bsAbs are also commonly associated with cytokine release syndrome (CRS), putatively due to excessive activity of anti-CD3 domains. Extratumoral activity of immunotherapies results in the secretion of pro-inflammatory cytokines in healthy tissues, which can result in undesirable safety profiles. Furthermore, while TAA/CD3 bsAbs potently deplete TAA-overexpressing cells, they do so by recruiting and stimulating CTLs regardless of whether such cells express a T cell receptor (TCR) that recognizes a tumor-antigen(s) (*i. e.*, tumor-reactive T cell). Therefore, rather than stimulating or reactivating the host's native anti-tumor immunity, TAA/CD3 bsAbs somewhat indiscriminately stimulate CTLs, potentially posing safety risks.

Although the exact pathways by which such DLTs arise can vary, the risk of immunotherapy-related toxicities can typically be minimized or eliminated by enhancing the tumor-localization of pharmacological activity.

TAAs that are almost exclusively expressed on cancer cells, such as oncofetal tumor antigens, are referred to as clean TAAs. TAA that are also expressed on normal, non-cancer cells - typically at lower levels compared to cancer cells - are considered non-clean TAAs. Due to the very high potency of TAA/CD3 bsAbs approaches, non-clean TAAs are a challenge as they damage non-tumor cells that also express the TAA. Mesothelin (MSLN) is an example of a non-clean TAA; it is not only expressed on tumor cells but also in various other tissues, albeit at a lower level. Therefore, when targeting non-clean TAAs, novel therapies that improve the selectivity of TAA/CD3 bsAb approaches for tumor tissues and minimize off tumor/on target effects are needed. This particularly applies to MSLN/CD3 bsAb approaches.

Mesothelin (MSLN) has been proposed as a tumor-associated antigen (TAA) that can be targeted to treat MSLN$^+$ solid cancers such as mesothelioma. Many other types of cancers are also MSLN$^+$, including certain forms of ovarian cancer and pancreatic cancer, as well as triple negative breast cancer. The current standard of care for mesothelioma includes tumor resection, chemotherapy, and radiation therapy, as well as palliative measures such as fluid reduction and pain management. Immunotherapies for tumors that continue growing include the use of PD-1/PD-L1 blockers such as pembrolizumab and nivolumab, or anti-CTLA4 antibodies such as ipilimumab to stimulate the immune system, as well as VEGF inhibitors such as bevacizumab to block blood vessel angiogenesis. While these therapies have achieved clinical success, they come with a higher risk of systemic side effects. Therefore, a need exists for a MSLN-targeted, specific approach.

A number of preclinical and early stage clinical studies have been performed or are underway to assess the feasibility of targeting MSLN via a few different approaches, which include antibody-based drugs and CAR-T cells. Antibody-based drugs include targeting MSLN-expressing cells with: the anti-MSLN fragment SS1P immunotoxin together with chemotherapeutics such as pemetrexed and cisplatin or coupled with PE38; amatuximab, a chimeric monoclonal antibody to induce ADCC; antibody drug conjugates such as anetumab ravtansine (BAY 94-9343: anti-MSLN + tubulin inhibitor DM4) or DMOT4039A (anti-MSLN + anti-mitotic monomethyl auristatin E) to inhibit tumor growth. HPN536, a multispecific engager (anti-MSLN + anti-CD3 + anti-albumin) with an improved half-life appears to redirect T cells to kill MSLN-expressing targets *in vitro* and *in vivo* and seems to be well tolerated by cynomolgous monkeys. Several anti-MSLN chimeric antigen receptor (CAR)-T therapies have also been well tolerated, including transient mRNA-transfected CAR-T (RNA CARTmeso) and CAR-T that have been engineered with a suicide gene (iCasp9m28z). Responses to most anti-MSLN therapies thus far have been modest, indicating the challenges associated with treating solid tumors.

For anti-MSLN therapies, this problem is further compounded by the fact that MSLN is shed into the serum of cancer patients, where it is referred to as soluble mesothelin-related protein (SMRP). Antibodies with a high affinity to MSLN also strongly bind to SMRP, which significantly counteracts their activity reducing the effective dose on cancer cells.

Therefore, there is a need for novel molecules that are able to effectively localize to tumors and promote T cell responses in the presence of SMRP.

Multispecific antibodies having at least three binding domains, of which two specifically bind to mesothelin (MSLN-BD) and one specifically binds to CD3 (CD3-BD), wherein the binding affinity of the two MSLN-BD is in a well balanced range, could theoretically address many of the foregoing limitations with respect to safety and efficacy. Such multispecific antibodies are theoretically capable of eliciting a high tumor localization and improved selectivity, which could provide safer and more effective therapies for a variety of cancers. Additionally, such molecules would further limit the need for co-administration of additional immunotherapies to boost patient responses, supporting ease-of-development and minimizing treatment costs. However, implementation of multispecific antibodies for therapeutic use has been complicated due to issues with their molecular architecture, the properties of their component antigen-binding domains, their producability and/or poor biophysical properties. In summary, there remains a clear need for novel multi-specific antibodies that exhibit increased tumor cell localization and elicit effective T cell activation with a tolerable toxicological profile and that have biophysical properties rendering them suitable for pharmaceutical development.

In addition, despite the fact that numerous antibodies already exist that are specific for MSLN and/or CD3, the complex and specific requirements of such multispecific antibodies require the development of novel antibody domains with tailor-made properties.

Thus, in spite of numerous treatment options for patients suffering from cancer, there remains a need for effective and safe therapeutic agents and a need for their preferential use in a more targeted manner. Immune-modulating biologics offer promising approaches in treatment of cancers due to their modes of action, however global immunostimulation and lack of any restriction of this immunomodulation to pathologically relevant cells and sites causes numerous side effects and significant toxicities, which potentially may lead to increased morbidity and mortality of patients. It is therefore an object of the present invention to provide a medicament to improve treatment of a proliferative disease, particularly a cancer.

## SUMMARY OF THE INVENTION

It is an object of the present invention to provide a medicament to improve treatment of a proliferative disease, particularly a cancer. In particular, it was an object of the present invention to provide a medicament having increased on target efficacy thereby improving the toxicological profile.

In a first aspect, the present invention relates to a multispecific antibody comprising:

a) two antibody-based binding domains, which specifically bind to mesothelin (MSLN-BD); and
b) at least one antibody-based binding domain, which specifically binds to CD3 (CD3-BD);

wherein said multispecific antibody does not comprise an immunoglobulin Fc region polypeptide, and wherein each of said MSLN-BD binds to mesothelin (MSLN) with a monovalent dissociation constant ($K_D$) in the range of from 0.5 to 20 nM, when measured by SPR.

In a second aspect, the present invention relates to a specific MSLN-binding domain.

In a third aspect, the present invention relates to a nucleic acid sequence or two nucleic acid sequences encoding the multispecific antibody or the specific MSLN-binding domain of the present invention.

In a fourth aspect, the present invention relates to a vector or two vectors comprising the nucleic acid sequence or the two nucleic acid sequences of the present invention.

In a fifth aspect, the present invention relates to a host cell or host cells comprising the vector or the two vectors of the present invention.

In a sixth aspect, the present invention relates to a method for producing the multispecific antibody or a specific binding domain of the present invention, comprising (i) providing the nucleic acid sequence or the two nucleic acid sequences of the present invention, or the vector or the two vectors of the present invention, expressing said nucleic acid sequence or nucleic acid sequences, or said vector or vectors, and collecting said multispecific antibody or said specific binding domain from the expression system, or (ii) providing a host cell or host cells of the present invention, culturing said host cell or said host cells; and collecting said multispecific antibody or said specific binding domain, from the cell culture.

In a seventh aspect, the present invention relates to a pharmaceutical composition comprising the multispecific antibody of the present invention and a pharmaceutically acceptable carrier.

In an eighth aspect, the present invention relates to a multispecific antibody of the present invention for use in the treatment of a disease, particularly a human disease, more particularly a human disease selected from cancer, an inflammatory and an autoimmune disease, wherein said multispecific antibody is a single-chain protein comprising three or four binding domains.

In a ninth aspect, the present invention relates to a multispecific antibody of the present invention for use in the treatment of a disease, particularly a human disease, more particularly a human disease selected from cancer, an inflammatory and an autoimmune disease, wherein said multispecific antibody is a hetero-dimeric protein comprising three or four binding domains.

In a tenth aspect, the present invention relates to a method for the treatment of a disease, particularly a human disease, more particularly a human disease selected from cancer, an inflammatory and an autoimmune disease, comprising the step of administering the above defined single-chain multispecific antibody of the present invention, said single-chain multispecific antibody comprising three or four binding domains.

In an eleventh aspect, the present invention relates to a method for the treatment of a disease, particularly a human disease, more particularly a human disease selected from cancer, an inflammatory and an autoimmune disease, comprising the step of administering the above defined hetero-dimeric multispecific antibody of the present invention, said hetero-dimeric multispecific antibody comprising three or four binding domains.

The aspects, advantageous features and preferred embodiments of the present invention summarized in the following items, respectively alone or in combination, further contribute to solving the object of the invention:

1. A multispecific antibody comprising:

   a) two antibody-based binding domains, which specifically bind to mesothelin (MSLN-BDs); and
   b) at least one antibody-based binding domain, which specifically binds to CD3 (CD3-BD);

   wherein said multispecific antibody does not comprise an immunoglobulin Fc region polypeptide, and wherein each of said MSLN-BDs binds to mesothelin (MSLN) with a monovalent dissociation constant ($K_D$) in the range of from 0.5 to 20 nM, when measured by SPR.

2. The multispecific antibody of item 1, wherein the $EC_{50}$ of said multispecific antibody for killing target cells, which have a 6 to 8-fold higher MSLN expression level than MeT-5A cells (ATCC CRL-9444) as determined by flow cytometry, does not increase by more than 20-fold in the presence of at least 200 ng/ml, particularly of at least 300 ng/ml, particularly of at least 400 ng/ml, particularly of at least 500 ng/ml soluble mesothelin, as determined in a T-cell driven cytotoxicity assay against said target cells.

3. The multispecific antibody of item 1, wherein said multispecific antibody is capable of killing target cells, which have a 6 to 8-fold higher MSLN expression level than MeT-5A cells (ATCC CRL-9444) as determined by flow cytometry, with an $EC_{50}$ that is at least 100 fold, particularly at least 250 fold, particularly at least 500 fold, particularly at least 750 fold, particularly at least 1000 fold, particularly at least 1250 fold, particularly at least 1500 fold smaller than the $EC_{50}$ for killing said MeT-5A cells, as determined in a T-cell driven cytotoxicity assay against said target cells and said MeT-5A cells.

4. The multispecific antibody of any one of items 1 to 3, wherein each of said MSLN-BDs binds to mesothelin (MSLN) with a monovalent dissociation constant ($K_D$) in the range of from 0.5 to 15 nM, particularly in the range of from 0.6 to 10 nM, particularly in the range of from 0.7 to 5 nM, when measured by SPR.

5. The multispecific antibody of any one of the preceding items, wherein each of said MSLN-BDs specifically binds to human mesothelin.

6. The multispecific antibody of any one of the preceding items, wherein each of said MSLN-BDs binds to Region I, Region II and/or Region III of MSLN, preferably to Region I and/or Region II of MSLN, in particular to Region I of MSLN.

7. The multispecific antibody of any one of items 1 to 6, wherein the two MSLN-BDs bind to the same epitope on MSLN.

8. The multispecific antibody of any one of the preceding items, wherein said MSLN-BD comprises

   (i) the HCDR1, HCDR2, and HCDR3 sequences of SEQ ID NOs: 1, 2 (or 10) and 3, respectively, and the LCDR1, LCDR2, and LCDR3 sequences of SEQ ID NOs: 4, 5 and 6, respectively; or the HCDR1, HCDR2, and HCDR3 sequences of SEQ ID NOs: 11, 12 and 13, respectively, and the LCDR1, LCDR2, and LCDR3 sequences of SEQ ID NOs: 14, 15 and 16, respectively; and
   (ii) VH3 or VH4 domain framework sequences FR1 to FR4; particularly VH3 domain framework sequences FR1 to FR4; and
   (iii) a VL domain comprising a VL framework comprising Vκ frameworks FR1, FR2 and FR3, particularly Vκ1 or Vκ3 FR1 to FR3, particularly Vκ1 FR1 to FR3, and a framework FR4, which is selected from a Vκ FR4, and a Vλ FR4, particularly a Vλ FR4 comprising an amino acid sequence having at least 70, 80, or 90 percent identity to any of SEQ ID NO: 66 to SEQ ID NO: 73, more particularly Vλ FR4 selected from any of SEQ ID NO: 66 to SEQ ID NO: 73, particularly Vλ FR4 according to SEQ ID NO: 66 or 73.

9. The multispecific antibody of any one of the preceding items, wherein said MSLN-BD comprises

   a.1) HCDR1, HCDR2, and HCDR3 sequences of SEQ ID NOs: 1,2 (or 10) and 3, respectively,
   b.1) LCDR1, LCDR2, and LCDR3 sequences of SEQ ID NOs: 4, 5 and 6, respectively,
   c.1) a VH sequence at least 80, 85, 90, 91, 92, 93, 94, 95, 96, 97, 98 or 99 percent identical to the amino acid sequence SEQ ID NO: 7, and
   d.1) a VL sequence at least 80, 85, 90, 91, 92, 93, 94, 95, 96, 97, 98 or 99 percent identical to the amino acid sequence SEQ ID NO: 9;
   or
   a.2) HCDR1, HCDR2, and HCDR3 sequences of SEQ ID NOs: 1, 2 (or 10) and 3, respectively,
   b.2) LCDR1, LCDR2, and LCDR3 sequences of SEQ ID NOs: 4, 5 and 6, respectively,
   c.2) a VH sequence at least 80, 85, 90, 91, 92, 93, 94, 95, 96, 97, 98 or 99 percent identical to the amino acid sequence SEQ ID NO: 8, and
   d.2) a VL sequence at least 80, 85, 90, 91, 92, 93, 94, 95, 96, 97, 98 or 99 percent identical to the amino acid sequence SEQ ID NO: 9;
   or
   a.3) HCDR1, HCDR2, and HCDR3 sequences of SEQ ID NOs: 11, 12 and 13, respectively,
   b.3) LCDR1, LCDR2, and LCDR3 sequences of SEQ ID NOs: 14, 15 and 16, respectively,
   c.3) a VH sequence at least 80, 85, 90, 91, 92, 93, 94, 95, 96, 97, 98 or 99 percent identical to the amino acid sequence SEQ ID NO: 17, and
   d.3) a VL sequence at least 80, 85, 90, 91, 92, 93, 94, 95, 96, 97, 98 or 99 percent identical to the amino acid sequence SEQ ID NO: 18.

10. The multispecific antibody of any one of items 8 or 9, wherein said VH domain comprises a C51 amino acid residue (AHo numbering) and said VL domain comprises a C141 amino acid residue (AHo numbering).

11. The multispecific antibody of any one of items 5 to 10, wherein each of said MSLN-BDs is cross reactive with Macaca fascicularis (Cynomolgus) MSLN, in particular binds to Cynomolgus MSLN with a monovalent $K_D$ in the range of 3 to 75 nM, particularly in the range of 3 to 60 nM, particularly of 4 to 50 nM, particularly of 5 to 40 nM, as measured by SPR.

12. The multispecific antibody of any one of the preceding items, wherein said CD3-BD is binding to CD3ε.

13. The multispecific antibody of any one of the preceding items, wherein said antibody comprises one or two CD3-BD, particularly one CD3-BD.

14. The multispecific antibody of any one of the preceding items, wherein said CD3-BD binds CD3ε with a monovalent $K_D$ of less than 50 nM, particularly with a monovalent $K_D$ of 0.5 to 50 nM, particularly of 1 to 40 nM, particularly of 2 to 35 nM, particularly of 3 to 30 nM, as measured by SPR.

15. The multispecific antibody of any one of the preceding items, wherein said CD3-BD comprises

(i) HCDR1, HCDR2, and HCDR3 sequences of SEQ ID NOs: 27, 28 and 29, respectively in a human antibody VH framework, particularly a VH3 framework, and
(ii) LCDR1, LCDR2, and LCDR3 sequences of SEQ ID NOs: 30, 31 and 32, respectively in a human antibody VL framework, wherein the VL framework comprises Vκ frameworks FR1, FR2 and FR3, particularly Vκ1 frameworks, and a framework FR4, which is selected from a Vκ FR4, and a Vλ framework 4.

16. The multispecific antibody of item 15, wherein said CD3-BD comprises

(i) a VH domain comprising the amino acid sequence of SEQ ID NO: 33, and
(ii) a VL domain comprising the amino acid sequence of SEQ ID NO: 34.

17. The multispecific antibody of any one of the preceding items, wherein said antibody further comprises at least one human serum albumin binding domain (hSA-BD), particularly one hSA-BD.

18. The multispecific antibody of item 17, wherein said hSA-BD comprises

(i) HCDR1, HCDR2, and HCDR3 sequences of SEQ ID NOs: 35, 36 and 37, respectively in a human antibody VH framework, particularly a VH3 framework, and
(ii) LCDR1, LCDR2, and LCDR3 sequences of SEQ ID NOs: 38, 39 and 40, respectively in a human antibody VL framework, wherein the VL framework comprises Vκ frameworks FR1, FR2 and FR3, particularly Vκ1 frameworks, and a framework FR4, which is selected from a Vκ FR4, particularly Vκ1 FR4, and a Vλ framework 4; or
(i) HCDR1, HCDR2, and HCDR3 sequences of SEQ ID NOs: 45, 46 and 47, respectively in a human antibody VH framework, particularly a VH3 framework, and
(ii) LCDR1, LCDR2, and LCDR3 sequences of SEQ ID NOs: 48, 49 and 50, respectively in a human antibody VL framework, wherein the VL framework comprises Vκ frameworks FR1, FR2 and FR3, particularly Vκ1 frameworks, and a framework FR4, which is selected from a Vκ FR4, particularly Vκ1 FR4, and a Vλ framework 4.

19. The multispecific antibody of items 17 or 18, wherein said hSA-BD comprises

(i) a VH domain comprising the amino acid sequence of SEQ ID NO: 41 and a VL domain comprising the amino acid sequence of SEQ ID NO: 42; or
(ii) a VH domain comprising the amino acid sequence of SEQ ID NO: 51 and a VL domain comprising the amino acid sequence of SEQ ID NO: 52.]

20. The multispecific antibody of item 19, wherein said VH domain comprises a C51 amino acid residue (AHo numbering) and said VL domain comprises a C141 amino acid residue (AHo numbering).

21. The multispecific antibody of any one of the preceding items, wherein said binding domains are independently selected from the group consisting of a Fab, an Fv, an scFv, dsFv, a scAb, and a STAB.

22. The multispecific antibody of item 21, wherein each of said binding domains is independently selected from

(a) a cognate pair of a VL domain and a VH domain (Fv fragment); or
(b) a cognate pair of a VL domain and a VH domain linked by an oligo- or polypeptide linker (scFv fragment).

23. The multispecific antibody of any one of the preceding items, wherein said multispecific antibody is in a format selected from the group consisting of: a tandem scDb (Tandab), a linear dimeric scDb (LD-scDb), a circular dimeric scDb (CD-scDb), a tandem tri-scFv, a tribody (Fab-(scFv)2), Fab-Fv2, triabody, scDb-scFv, tetrabody, di-diabody, CODV, tandem-di-scFv, tandem tri-scFv, Fab-(scFv)2, Fab-Fv2, or CODV fused to the N- and/or the C-terminus of a heterodimerization domain other than heterodimeric Fc domains, and MATCH.

24. The multispecific antibody of any one of items 1 to 23, wherein said antibody does not comprise CH1 and/or CL regions.

25. The multispecific antibody of any one of the preceding items, wherein said antibody is a scDb-scFv, tribody, MATCH, in particular wherein said multispecific antibody is in a MATCH or tribody format, more particularly wherein said multispecific antibody is in a MATCH format, more particularly wherein said multispecific antibody is a MATCH3 or a MATCH4.

26. The multispecific antibody of items 1 to 25, wherein said multispecific antibody is a single-chain protein.

27. The multispecific antibody of item 26, wherein said single-chain protein comprises an amino acid sequence consisting of:

> (i) a first VL domain,
> (ii) a first polypeptide linker,
> (iii) a first VH domain,
> (iv) a second polypeptide linker,
> (v) a second VL domain,
> (vi) a third polypeptide linker, and
> (vii) a second VH domain,
> arranged one after another in the stated order,
> wherein said first VL domain associates with said second VH domain to form a first binding domain, and said second VL domain associates with said first VH domain to form a second binding domain,
> and wherein said single-chain protein further comprises
> (viii) a third binding domain, which is formed by a third VL domain and a third VH domain that are connected via a fourth polypeptide linker, where said third binding domain is fused C-terminally or N-terminally via a fifth polypeptide linker to said amino acid sequence,
> wherein said three binding domains have the following specificities:
>
>> a) the first binding domain specifically binds to human CD3 (CD3-BD); and
>> b) the second and third binding domains specifically bind to mesothelin (MSLN-BD).

28. The multispecific antibody of item 27, wherein said single-chain protein further comprises a hSA-BD, which is formed by a fourth VL domain and a fourth VH domain that are connected via a sixth polypeptide linker, where said hSA-BD is fused C-terminally or N-terminally via a seventh polypeptide linker to said amino acid sequence.

29. The multispecific antibody of items 1 to 25, wherein said multispecific antibody is a hetero-dimeric protein comprising a first and a second single-chain protein, wherein said first single-chain protein comprises a first amino acid sequence consisting of (from the N- to the C-terminus):

> (viii) a first VL domain,
> (ix) a first polypeptide linker, and
> (x) a second VL domain, and

wherein said second single-chain protein comprises a second amino acid sequence consisting of (from the N- to the C-terminus):

> (ib) a first VH domain,
> (iib) a second polypeptide linker, and
> (iiib) a second VH domain, and

wherein said first VL domain associates with either said first or said second VH domain to form a first binding domain, and said second VL domain associates with the other of said VH domains to form a second binding domain, and wherein at least one of said first and said second single-chain proteins further comprises

> (iv) a third binding domain, which is formed by a third VL domain and a third VH domain that are connected via a third polypeptide linker, where said third binding domain is fused via a fourth polypeptide linker to said first or said second amino acid sequence,

and wherein optionally at least one of said first and said second single-chain proteins further comprises

(v) a fourth binding domain, which is formed by a fourth VL domain and a fourth VH domain that are connected via a fifth polypeptide linker, where said fourth binding domain is fused via a sixth polypeptide linker to said first or said second amino acid sequence,
wherein said three, or optionally four, binding domains have the following specificities:

a) one antibody-based binding domain specifically binds to human CD3 (CD3-BD);
b) another two antibody-based binding domains specifically bind to mesothelin (MSLN-BD), and, when the optional fourth binding domain is present,
c) the remaining binding domain specifically binds to human serum albumin (hSA-BD).

30. The multispecific antibody of item 29, wherein the optional fourth domain is present and wherein one of said first and second binding domain is a CD3-BD and the other one of said first and second binding domain is a hSA-BD.

31. The multispecific antibody of item 29 or 30, wherein the optional fourth domain is present and wherein the third binding domain is fused to either the first or the second amino acid sequence, and the fourth binding domain is fused to the other one of the two said amino acid sequences.

32. The multispecific antibody of any one of the items 29 to 31, wherein any binding domains comprised in said hetero-dimeric protein exclusively consist of immunoglobulin variable domains, arranged in said first and second single-chain protein.

33. The multispecific antibody of any one of the items 29 to 32, wherein said hetero-dimeric protein does not comprise a cognate pair of a first and a second proteinaceous interaction domain, other than said first and second VL and VH domains, wherein said first proteinaceous interaction domain is comprised in said first single-chain protein and wherein said second proteinaceous interaction domain is comprised in said second single-chain protein.

34. The multispecific antibody of any one of items 29 to 33, wherein said first single-chain protein and said second single-chain protein hetero-dimerize in a parallel orientation, *i. e.* said first VL domain associates with said first VH domain and said second VL domain associates with said second VH domain.

35. The multispecific antibody of any one of items 29 to 33, wherein said first single-chain protein and said second single-chain protein hetero-dimerize in an anti-parallel orientation, *i. e.* said first VL domain associates with said second VH domain and said second VL domain associates with said first VH domain.

36. The multispecific antibody of any one of items 29 to 35, wherein
said first single-chain protein comprises an amino acid sequence having at least 90, 95, 96, 97, 98 or 99 percent identity to the amino acid sequence of SEQ ID NO: 55, more particularly comprises the amino acid sequence of SEQ ID NO: 55, especially consists of the amino acid sequence SEQ ID NO: 55 and
said second single-chain protein comprises an amino acid sequence having at least 90, 95, 96, 97, 98 or 99 percent identity to the amino acid sequence of SEQ ID NO: 56, more particularly comprises the amino acid sequence of SEQ ID NO: 56, especially consists of the amino acid sequence SEQ ID NO: 56; or
said first single-chain protein comprises an amino acid sequence having at least 90, 95, 96, 97, 98 or 99 percent identity to the amino acid sequence of SEQ ID NO: 57, more particularly comprises the amino acid sequence of SEQ ID NO: 57, especially consists of the amino acid sequence SEQ ID NO: 57 and
said second single-chain protein comprises an amino acid sequence having at least 90, 95, 96, 97, 98 or 99 percent identity to the amino acid sequence of SEQ ID NO: 58, more particularly comprises the amino acid sequence of SEQ ID NO: 58, especially consists of the amino acid sequence SEQ ID NO: 58.

37. The multispecific antibody of any one of the preceding items, wherein at least one of said antibody variable domains comprises CDR regions derived from a parental rabbit antibody.

38. The multispecific antibody of any one of the preceding items, wherein at least one of the MSLN-BDs and the CD3-BD are capable of binding to their respective antigens simultaneously, particularly wherein both MSLN-BDs and the CD3-BD are capable of binding to their respective antigens simultaneously.

39. A MSLN-binding domain as defined in any one of items 8 to 10.

40. A nucleic acid sequence or two nucleic acid sequences encoding the multispecific antibody of any one of items 1 to 38 or the MSLN-binding domain of item 39.

41. A vector or two vectors comprising the nucleic acid sequence or the two nucleic acid sequences of item 40.

42. A host cell or host cells comprising the vector or the two vectors of item 41.

43. A method for producing the multispecific antibody of any one of items 1 to 38, or the MSLN-binding domain of item 39, comprising (i) providing the nucleic acid sequence or the two nucleic acid sequences of item 40, or the vector or the two vectors of item 41, expressing said nucleic acid sequence or nucleic acid sequences, or said vector or vectors, and collecting said multispecific antibody or said binding domain from the expression system, or (ii) providing a host cell or host cells according to item 42, culturing said host cell or said host cells; and collecting said multispecific antibody or said binding domain from the cell culture.

44. A pharmaceutical composition comprising the multispecific antibody of any one of items 1 to 38 and a pharmaceutically acceptable carrier.

45. The multispecific antibody of any one of items 1 to 28 and 37 to 38 for use in the treatment of a disease, particularly a human disease, more particularly a human disease selected from cancer, an inflammatory and an autoimmune disease, wherein said multispecific antibody is a single-chain protein comprising three or four binding domains.

46. The multispecific antibody of any one of items 1 to 25 and 29 to 38 for use in the treatment of a disease, particularly a human disease, more particularly a human disease selected from cancer, an inflammatory and an autoimmune disease, wherein said multispecific antibody is a hetero-dimeric protein comprising three or four binding domains.

47. The multispecific antibody of any one of items 1 to 38 for use in the treatment of a disease according to items 45 and 46, wherein said disease is a cancer, particularly a cancer selected from mesothelioma, pancreatic cancer, and ovarian cancer.

48. A method for the treatment of a disease, particularly a human disease, more particularly a human disease selected from cancer, an inflammatory and an autoimmune disease, comprising the step of administering the multispecific antibody of any one of items 1 to 28 and 37 to 38, wherein said multispecific antibody is a single-chain protein comprising three or four binding domains.

49. A method for the treatment of a disease, particularly a human disease, more particularly a human disease selected from cancer, an inflammatory and an autoimmune disease, comprising the step of administering the multispecific antibody of any one of items 1 to 25 and 29 to 38, wherein said multispecific antibody is a hetero-dimeric protein comprising three or four binding domains.

50. The method of items 48 or 49, wherein said disease is a cancer, particularly a cancer selected from mesothelioma, pancreatic cancer, and ovarian cancer.

## BRIEF DESCRIPTION OF THE DRAWINGS

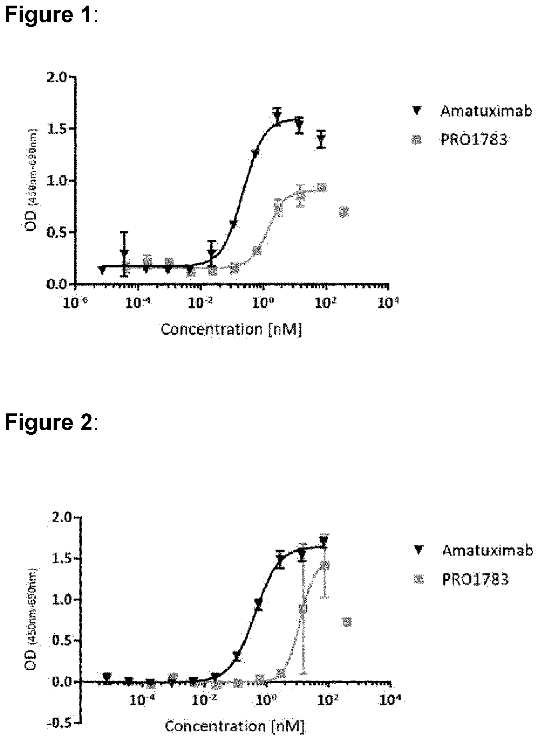

**FIG. 1** shows the binding of anti-MSLN scFv PRO1783 and reference antibody Amatuximab to the plasma membrane of cells from a H226 cell line expressing high levels of human MSLN. Binding of PRO1783 and Amatuximab to H226 cell line was tested in competition ELISA ("cELISA"). HRP-coupled Protein L and HRP-coupled anti-human IgG antibody were used to detect PRO1783 and Amatuximab bound to H226 cells in cELISA, respectively. The optical density ($OD_{450nm-690nm}$) is represented as a function of antibody concentration in nM. Note that only concentrations with increasing values were fitted. The $EC_{50}$ value of PRO1783 is roughly six times higher than the value found for reference antibody Amatuximab.

**FIG. 2** shows the binding of anti-MSLN scFv PRO1783 and reference antibody Amatuximab to the plasma membrane of cells from a CHO cell line expressing cynomolgus monkey MSLN. Plasma-membranous binding of PRO1783 and Amatuximab to CHO cell line expressing cynomolgus monkey MSLN was tested in cELISA. Binding of PRO1783 and Amatuximab was detected by HRP-coupled Protein L and HRP-coupled anti-human IgG antibody, respectively. The optical density ($OD_{450nm-690nm}$) is represented as function of antibody concentration in nM. Amatuximab as well as PRO1783 demonstrated binding to cynomolgus monkey MSLN.

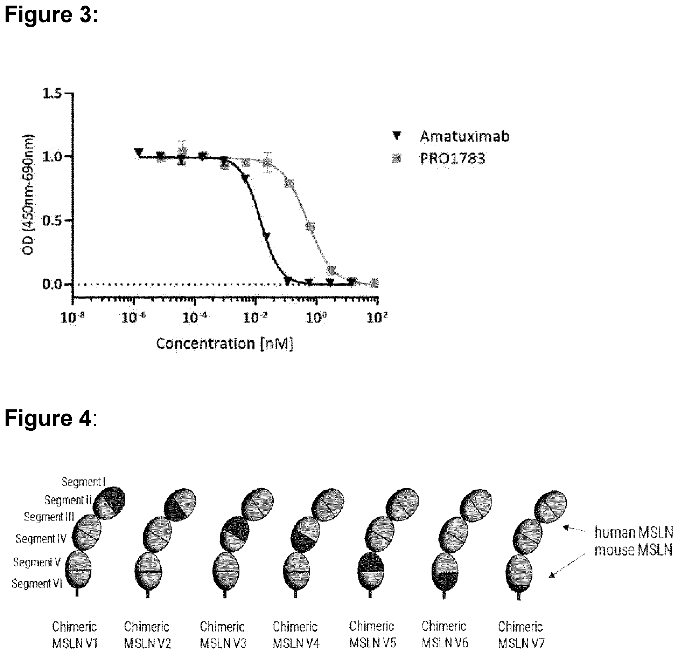

**FIG. 3** shows the blockade of human MSLN/MUC16 interaction by anti-MSLN scFv PRO1783. The potency of PRO1783 and Amatuximab to block human MSLN/MUC16 interaction was tested in cELISA. The optical density

($OD_{450nm-690nm}$) is represented as function of antibody concentration in nM. The $IC_{50}$ Of PRO1783 was 0.5 nM, whereas reference antibody Amatuximab neutralized MSLN/MUC16 interaction more potently ($IC_{50}$ = 0.014 nM).

**FIG. 4** shows chimeric variants of hMSLN/mMSLN. Domain highlighted in dark grey is the segment of the human MSLN sequence replaced by the corresponding mouse sequence. Segment VI corresponds to the C-terminal part of MSLN extracellular domain being the closest to the plasma membrane. Segments I and II correspond to Region I of MSLN; Segments III and IV correspond to Region II of MSLN; and Segments V and VI correspond to Region III of MSLN.

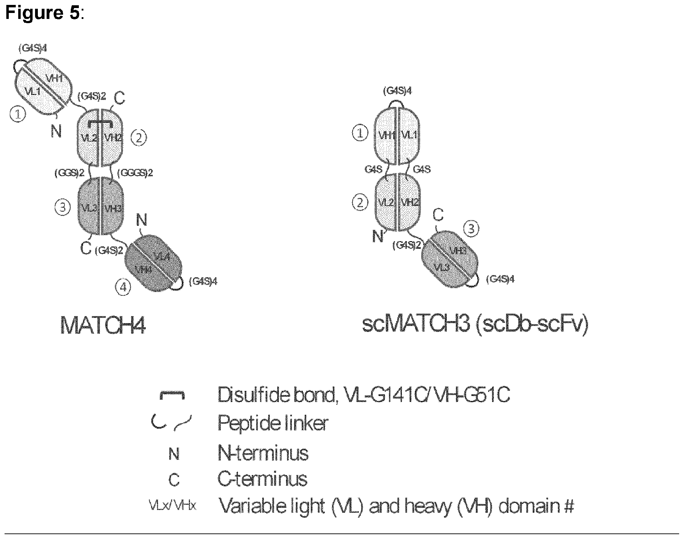

**FIG. 5** shows different MATCH formats. (left) Architecture of the anti-parallel MATCH4 format in which the split, heterodimer-forming variable domains on each chain are organized in the opposite N-terminus-to-C-terminus order as their cognate variable domains on the complementary MATCH chain. (right) Exemplary architecture of the scMATCH3 format in which split variable domains are located on a single peptide chain, which assembles into trispecific molecules. Alternative arrangements such as VL2-VL1-VH1-VH2-scFv are also within the scope of the scMATCH3 format concept. Gly-Ser linkers which were used to connect the domains are indicated by lines. Table 15 describes the domains comprised in the different molecules produced and their positioning within the molecules (Domains 1 to 4). The domain numbering does not correlate with the binding-domain numbering, as defined in the claims.

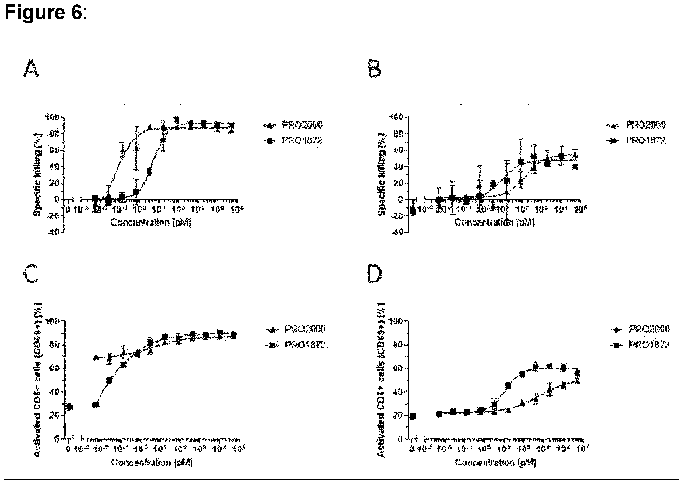

**FIG. 6** shows the cytotoxic activity and effect on CD8+ T cell activation of PRO2000 and PRO1872 in the presence of human serum albumin. (A) Specific killing of high MSLN expressing cancer cells (H226 cells). On cancer cells expressing high levels of mesothelin, the target cell killing potency observed for PRO2000 is 75-fold better than for PRO1872. (B) Specific killing of low MSLN expressing cancer cells (MeT-5A cells). On cells derived from healthy mesothelial tissue (MeT-5A; ATCC CRL-9444) expressing low mesothelin levels the monovalent mesothelin binding protein PRO1872 shows the best killing potency. (C) CD8+ T cell activation in presence of H226 cells, and (D) CD8+ T cell activation in presence of MeT-5A cells. Similar data were observed for CD8+ T cell activation. PBMCs from donor #1 were used. Target cells and CD8+ T cells were analyzed by flow cytometry 40 h after the beginning of their incubation with the respective molecules and data were fitted using sigmoidal 4PL fit (GraphPad Prism).

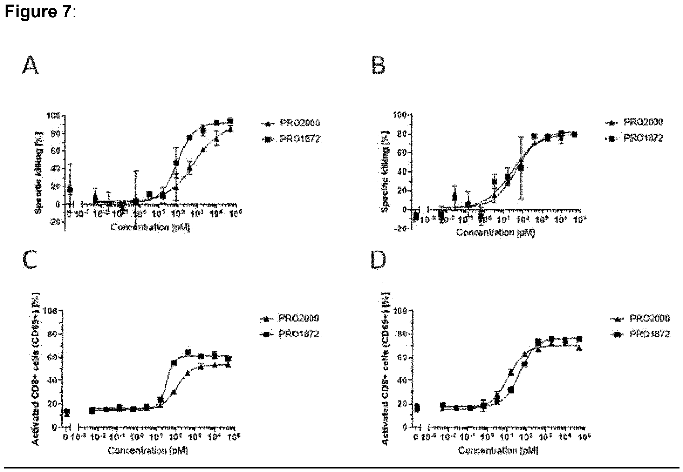

**FIG. 7** shows the cytotoxic activity and effect on CD8+ T cell activation of PRO2000 and PRO1872 in the presence of human serum albumin. (A) Specific killing of low MSLN expressing cancer cells (H292 cells). On cancer cells expressing low levels of mesothelin, the target cell killing potency observed for PRO1872 is 7-fold better than for PRO2000. (B) Specific killing of intermediate MSLN expressing cancer cells (HPAC cells). On cancer cells expressing intermediate mesothelin levels both monovalent and bivalent mesothelin binders PRO1872 and PRO2000 show similar killing potency. (C) CD8+ T cell activation in presence of H292 cells, and (D) CD8+ T cell activation in presence of HPAC cells. Similar data were observed for CD8+ T cells activation, except that PRO2000 was clearly more potent than PRO1872 in presence of HPAC cells (4-fold). PBMCs from donor #1 were used. Target cells and CD8+ T cells were analyzed by flow cytometry 40 h after the beginning of their incubation with the respective molecules and data were fitted using sigmoidal 4PL fit (GraphPad Prism).

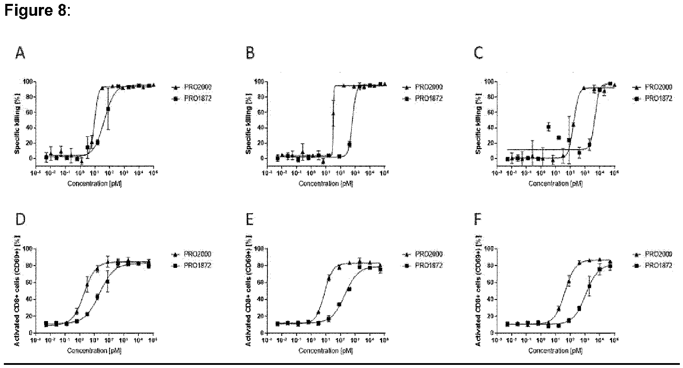

**FIG. 8** shows the cytotoxic activity and effect on CD8+ T cell activation of PRO2000 and PRO1872 in absence or presence of sMSLN. (A to C) Cytotoxic activity of PRO2000 and PRO1872 on H226 target cells. Specific killing of H226 cells in absence of sMSLN (A), in presence of 50 ng/ml sMSLN (B), or in presence of 500 ng/ml sMSLN (C). PRO2000 killing potency is less affected by increasing concentrations of sMSLN as compared to PRO1872 (D to F) Similar data are observed for CD8+ T cell activation in the corresponding conditions. CD8+ T cell activation in presence of H226 cells without sMSLN (D), in presence of 50 ng/ml sMLSN (E), or in presence of 500 ng/ml sMLSN (F). PBMCs from donor #2 were used. Target cells and CD8+ T cells were analyzed by flow cytometry 40 h after the beginning of their incubation with the respective molecules and data were fitted using sigmoidal 4PL fit (GraphPad Prism).

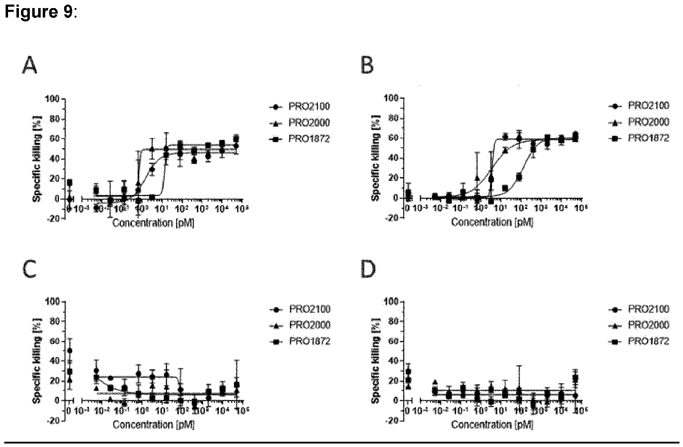

**FIG. 9** shows the cytotoxic activity of PRO2000, PRO2100 and PRO1872 in absence or presence of 100 ng/ml sMSLN: Cytotoxic activity on H226 target cells in absence of sMSLN (A) or presence of 100 ng/ml sMSLN (B); cytotoxic activity on Met-5A target (ATCC CRL-9444) cells in absence of sMSLN (C) or presence of 100 ng/ml sMSLN (D). PBMCs from donor #3 were used. Target cells were analyzed by flow cytometry 40 h after the beginning of their incubation with the respective molecules and data were fitted using sigmoidal 4PL fit (GraphPad Prism).

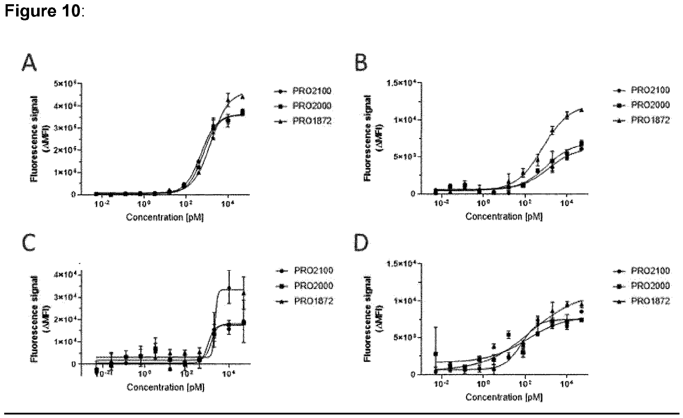

**FIG. 10** shows binding of MATCH molecules to target cell lines expressing different mesothelin cell surface levels. Binding of PRO2000, PRO2100 and PRO1872 to (A) high mesothelin expressing H226 cells, (C) intermediate mesothelin expressing HPAC cells, (B) low mesothelin expressing H292 cancer cells and (D) low mesothelin expressing mesothelial cells, MeT-5A (ATCC CRL-9444) was assessed by flow cytometry and data were fitted using sigmoidal 4PL fit (GraphPad Prism).

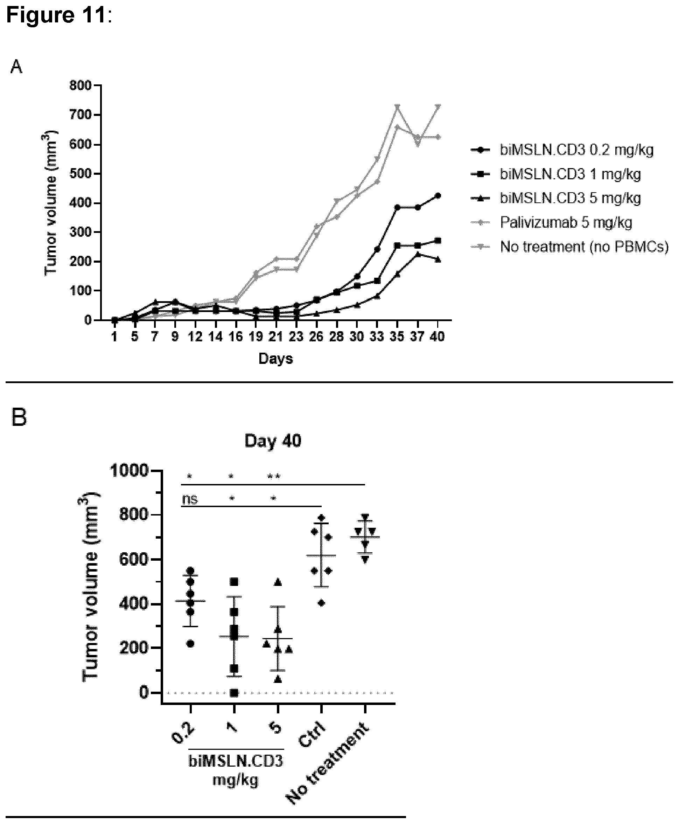

**FIG. 11** shows that treatment with molecule PRO2000 (biMSLN.CD3) results in tumor growth inhibition relative to control conditions. (A) Longitudinal analysis of tumor growth in the presence or absence of treatment. The lines depict the median. Animals were subcutaneously co-implanted with 1 x $10^7$ H292 cells and 1 x $10^7$ PBMCs, and treatment was administered intravenously starting on day 5 and repeated every 5 days until the end of the experiment. (B) Day 40 data displayed as a scatter plot. Each point corresponds to one animal, and the data are displayed with the mean and standard deviation. After a two-way repeated measures ANOVA, the Tukey's multiple comparisons test was performed and the significance of each data set is depicted relative to palivizumab control (Ctrl, lower line), or no treatment (upper line). ns = not significant; *, $p < 0.05$; **, $p < 0.01$, ***, $p < 0.001$. The spark line in gray indicates 0 on the y-axis.

## DETAILED DESCRIPTION OF THE INVENTION

Known MSLN/CD3 bsAbs-based immunotherapies typically suffer from dose-limiting toxicities and limited *in vivo* efficacy. There is thus a need in the medical field for novel MSLN/CD3 bsAbs-based immunotherapies, which have lower or no dose-limiting toxicities and higher efficacy than the currently available approaches.

The present invention provides a multispecific antibody comprising a combination of two mesothelin binding domains (MSLN-BD) and at least one binding domain for CD3 (CD3-BD), wherein the binding affinity of the MSLN-BD to MSLN is tuned such that it allows efficient localization on high MSLN expressing target cells while low MSLN expressing healthy mesothelial cells are much less affected. Due to the presence of two MSLN-BD, which are embedded in well-defined and compact multi-domain antibody architecture that is devoid of immunoglobulin Fc region polypeptides, in combination with well balanced MSLN and CD3 binding affinities, these multispecific antibodies show high on-target potency while exhibiting low off-tumor side effects. The compact bivalent design of multispecific antibodies of the present invention, which cannot be achieved for bivalent multispecific antibodies that are based on classical IgG-architecture, as well as their well tuned MSLN and CD3 binding affinities are crucial features for achieving the desired selectivity and efficacy profile.

The multispecific antibodies of the present invention are capable of binding to target cells via the two MSLN-BD in a highly antigen-density dependent manner by taking advantage of avidity effects. Simultaneously, the multispecific antibodies of the present invention are capable of inducing T-cell activation and tumor cell killing by binding to CD3 via the CD3-BD. Due to their enhanced selectivity for high MSLN expressing cells that leads to efficient tumor localization, the multispecific antibodies of the present invention enable treatments without dose-limiting toxicities caused by non-specific activation of T cells.

In addition, it has been surprisingly found that the potency of killing high MSLN expressing target cells is not significantly reduced in the presence of high levels of soluble mesothelin that is often observed in patient sera. Furthermore, the multispecific antibodies of the present invention comprising (a) two MSLN binding domains, and (b) at least one CD3-BD and having the above defined design antigen-binding affinities demonstrated further beneficial properties as shown in the Examples and accompanying figures. Furthermore, the optional addition of a half-life-extending anti-hSA domain not only enables convenient dosing, but should also promote delivery of the molecule to tumor microenvironments.

The multispecific antibodies of the present invention thus provide distinct therapeutic advantages over conventional compositions and therapies.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

In one aspect, the present invention relates to a multispecific antibody comprising:

a) two antibody-based binding domains, which specifically bind to mesothelin (MSLN-BDs); and
b) at least one antibody-based binding domain, which specifically binds to CD3 (CD3-BD);

wherein said multispecific antibody does not comprise an immunoglobulin Fc region polypeptide, and wherein each of said MSLN-BD binds to mesothelin (MSLN) with a monovalent dissociation constant ($K_D$) in the range of from 0.1 to 20 nM, when measured by SPR.

The term "antibody" and the like, as used herein, includes whole antibodies or single chains thereof; and any antigen-binding fragment (*i. e.*, "antigen-binding portion") or single chains thereof; and molecules comprising antibody CDRs, VH regions or VL regions (including without limitation multispecific antibodies). A naturally occurring "whole antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDRs), interspersed with regions that are more conserved, termed framework regions (FRs). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e. g., effector cells) and the first component (Clq) of the classical complement system.

The term "immunoglobulin Fc region", as used herein, refers to the CH2 and CH3 domains of the heavy chain constant regions.

The terms "binding domain", "antigen-binding fragment thereof", "antigen-binding portion" of an antibody, and the like, as used herein, refer to one or more fragments of an intact antibody that retain the ability to specifically bind to a given antigen (e. g., MSLN, CD3, hSA). Antigen-binding functions of an antibody can be performed by fragments of an intact antibody. In some embodiments, a binding domain of a multispecific antibody of the present invention is selected from the group consisting of a Fab fragment, a monovalent fragment consisting of the VL, VH, CL and CH1 domains; a F(ab)2 fragment, a bivalent fragment comprising two Fab fragments linked by a disulfide bridge at the hinge region; an Fd fragment consisting of the VH and CH1 domains; an Fv fragment consisting of the VL and VH domains of a single arm of an antibody; a single domain antibody (dAb) fragment (Ward et al., 1989 Nature 341:544-546), which consists of a VH domain; an isolated complementarity determining region (CDR), a single-chain Fv, a dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e. g. f-star technology (F-star's Modular Antibody TechnologyTM)). Suitably, a binding domain of the present invention is a single-chain Fv fragment (scFv) or a single antibody variable domain. In a preferred embodiment, a binding domain of the present invention is a single-chain Fv fragment (scFv). In particular embodiments, the two variable domains of an antigen-binding fragment, as in an Fv or an scFv fragment, are stablized by an interdomain disulfide bond, in particular wherein said VH domain comprises a single cysteine residue in position 51 (AHo numbering) and said VL domain comprises a single cysteine residue in position 141 (AHo numbering).

The term "Complementarity Determining Regions" ("CDRs") refers to amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273, 927-948 ("Chothia" numbering scheme); ImMunoGenTics (IMGT) numbering (Lefranc, M.-P., The Immunologist, 7, 132-136 (1999); Lefranc, M.-P. et al., Dev. Comp. Immunol., 27, 55-77 (2003)) ("IMGT" numbering scheme); and the numbering scheme described in Honegger & Pluckthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present invention, the numbering system suggested by Honegger & Plückthun ("AHo") is used (Honegger & Pluckthun, J. Mol. Biol. 309 (2001) 657-670), unless specifically mentioned otherwise. In particular, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H27-H42; HCDR2 (also referred to as CDR-H2): H57-H76; HCDR3 (also referred to as CDR-H3): H108-H138. For the sake of clarity, the numbering system according to Honegger & Plückthun takes the length diversity into account that is found in naturally occurring antibodies, both in the different VH and VL subfamilies and, in particular, in the CDRs, and provides for gaps in the sequences. Thus, in a given antibody variable domain usually not all positions 1 to 149 will be occupied by an amino acid residue.

The term "binding specificity" as used herein refers to the ability of an individual antibody to react with one antigenic determinant and not with a different antigenic determinant. As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" is referring to the ability of the antibody to discriminate between the target of interest and an unrelated molecule, as determined, for example, in accordance with a specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard colour development (e. g. secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, particularly at least 100-fold difference between a background and signal indicates on specific binding. Typically, determination of binding specificity is performed by using not a single reference molecule, but a set of about three to five unrelated molecules, such as milk powder, transferrin or the like.

Suitably, the antibody of the invention is an isolated antibody. The term "isolated antibody", as used herein, refers to an antibody that is substantially free of other antibodies having different antigenic specificities (e. g., an isolated antibody that specifically binds MSLN and CD3 is substantially free of antibodies that specifically bind antigens other than MSLN and CD3 and an isolated antibody that specifically binds MSLN, CD3 and human serum albumin is substantially free of antibodies that specifically bind antigens other than MSLN, CD3 and human serum albumin). Moreover, an isolated antibody may be substantially free of other cellular material and/or chemicals.

Suitably, the antibody of the invention is a monoclonal antibody. The term "monoclonal antibody" or "monoclonal antibody composition" as used herein refers to antibodies that are substantially identical to amino acid sequence or are derived from the same genetic source. A monoclonal antibody composition displays a binding specificity and affinity for a particular epitope, or binding specificities and affinities for specific epitopes.

Antibodies of the invention include, but are not limited to, the chimeric, human and humanized.

The term "chimeric antibody" (or antigen-binding fragment thereof) is an antibody molecule (or antigen-binding fragment thereof) in which (a) the constant region, or a portion thereof, is altered, replaced or exchanged so that the antigen-binding site (variable region) is linked to a constant region of a different or altered class, effector function and/or species, or an entirely different molecule which confers new properties to the chimeric antibody, e. g., an enzyme, toxin, hormone, growth factor, drug, etc.; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody" (or antigen-binding fragment thereof), as used herein, is intended to include antibodies (and antigen-binding fragments thereof) having variable regions in which both the framework and CDR regions are derived from sequences of human origin. Furthermore, if the antibody contains a constant region, the constant region also is derived from such human sequences, e. g., human germline sequences, or mutated versions of human germline sequences. The human antibodies and antigen-binding fragments thereof of the invention may include amino acid residues not encoded by human sequences (e. g., mutations introduced by random or site-specific mutagenesis *in vitro* or by somatic mutation *in vivo*). This definition of a human antibody specifically excludes a humanized antibody comprising non-human antigen-binding residues. Human antibodies can be produced using various techniques known in the art, including phage-display libraries (Hoogenboom and Winter, J. Mol. Biol, 227:381 (1991); Marks et al, J. Mol. Biol, 222:581 (1991)). Also available for the preparation of human monoclonal antibodies are methods described in Cole et al, Monoclonal Antibodies and Cancer Therapy, Alan R. Liss, p. 77 (1985); Boemer et al, J. Immunol, 147(I):86-95 (1991). See also van Dijk and van de Winkel, Curr. Opin. Pharmacol, 5: 368-74 (2001). Human antibodies can be prepared by administering the antigen to a transgenic animal that has been modified to produce such antibodies in response to antigenic challenge, but whose endogenous loci have been disabled, e. g., immunized xenomice (see, e. g., U.S. Pat. Nos. 6,075,181 and 6,150,584 regarding XENOMOUSE™ technology). See also, for example, Li et al, Proc. Natl. Acad. Sci. USA, 103:3557- 3562 (2006) regarding human antibodies generated via a human B-cell hybridoma technology.

A "humanized" antibody (or antigen-binding fragment thereof), as used herein, is an antibody (or antigen-binding fragment thereof) that retains the reactivity of a non-human antibody while being less immunogenic in humans. This can be achieved, for instance, by retaining the non-human CDR regions and replacing the remaining parts of the antibody with their human counterparts (i. e., the constant region as well as the framework portions of the variable region). Additional framework region modifications may be made within the human framework sequences as well as within the CDR sequences derived from the germline of another mammalian species. The humanized antibodies of the invention may include amino acid residues not encoded by human sequences (e. g., mutations introduced by random or site-specific mutagenesis *in vitro* or by somatic mutation *in vivo*, or a conservative substitution to promote stability or manufacturing). See, e. g., Morrison et al., Proc. Natl. Acad. Sci. USA, 81:6851-6855, 1984; Morrison and Oi, Adv. Immunol.,

44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include, but are not limited to the Xoma technology disclosed in U.S. Pat. No. 5,766,886.

The term "recombinant humanized antibody" as used herein, includes all human antibodies that are prepared, expressed, created or isolated by recombinant means, such as antibodies isolated from a host cell transformed to express the humanized antibody, e. g., from a transfectoma, and antibodies prepared, expressed, created or isolated by any other means that involve splicing of all or a portion of a human immunoglobulin gene, sequences to other DNA sequences.

Suitably, the antibody of the invention or antigen-binding fragment thereof is humanized. Suitably, the antibody of the invention or antigen-binding fragment thereof is humanized and comprises rabbit-derived CDRs.

The term "multispecific antibody" as used herein, refers to an antibody that binds to two or more different epitopes on at least two or more different targets (e. g., MSLN and CD3). The term "multispecific antibody" includes bispecific, trispecific, tetraspecific, pentaspecific and hexaspecific. The term "bispecific antibody" as used herein, refers to an antibody that binds to at least two different epitopes on two different targets (e. g., MSLN and CD3). The term "trispecific antibody" as used herein, refers to an antibody that binds to at least three different epitopes on three different targets (e. g., MSLN, CD3 and hSA).

The term "epitope" means a protein determinant capable of specific binding to an antibody. Epitopes usually consist of chemically active surface groupings of molecules such as amino acids or sugar side chains and usually have specific three-dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein.

The term "linear epitope" refers to an epitope with all of the points of interaction between the protein and the interacting molecule (such as an antibody) occurring linearly along the primary amino acid sequence of the protein (continuous).

The term "recognize" as used herein refers to an antibody antigen-binding fragment thereof that finds and interacts (e. g., binds) with its conformational epitope.

As used herein, the term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the total sum of non-covalent interactions between a single binding site of a molecule (e. g., of an antibody) and its binding partner (e. g., an antigen). Unless indicated otherwise, as used herein, "binding affinity", "bind to", "binds to" or "binding to" refers to intrinsic binding affinity that reflects a 1:1 interaction between members of a binding pair (e. g., an antibody fragment and antigen). The affinity of a molecule X for its partner Y can generally be represented by the dissociation constant ($K_D$). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative and exemplary embodiments for measuring binding affinity, i. e. binding strength are described in the following.

The term "Kassoc", "Ka" or "Kon", as used herein, is intended to refer to the association rate of a particular antibody-antigen interaction, whereas the term "Kdis", "Kd" or "Koff", as used herein, is intended to refer to the dissociation rate of a particular antibody- antigen interaction. In one embodiment, the term "$K_D$", as used herein, is intended to refer to the dissociation constant, which is obtained from the ratio of Kd to Ka (i. e. Kd/Ka) and is expressed as a molar concentration (M). The "$K_D$" or "$K_D$ value" or "KD" or "KD value" according to this invention is in one embodiment measured by using surface-plasmon resonance assays. Affinity to recombinant human mesothelin (human MSLN) and recombinant *Cynomolgus* MSLN (*Cynomolgus* MSLN) was determined by surface plasmon resonance (SPR) measurements as described in section [0163]. Affinity to recombinant human CD3 was measured by SPR as described in section [0188].

Suitably, the multispecific antibody of the present invention is bivalent for MSLN specificity.

Suitably, the multispecific antibody of the present invention is monovalent, bivalentor multivalent for CD3 specificity. In one embodiment, the multispecific antibody of the present invention is bivalent for CD3 specificity. In a preferred embodiment, the multispecific antibody of the present invention is monovalent for CD3 specificity.

The term "multivalent antibody" refers to a single binding molecule with more than one valency, where "valency" is described as the number of antigen-binding moieties that binds to epitopes on identical target molecules. As such, the single binding molecule can bind to more than one binding site on a target molecule. Examples of multivalent antibodies include, but are not limited to bivalent antibodies, trivalent antibodies, tetravalent antibodies, pentavalent antibodies, and the like.

The term "monovalent antibody", as used herein, refers to an antibody that binds to a single epitope on a target molecule, such as CD3. Also, the term "binding domain" or "monovalent binding domain", as used herein, refers to a binding domain that binds to a single epitope on a target molecule such as CD3.

The term "bivalent antibody" as used herein, refers to an antibody that binds to two epitopes on two identical target molecules, such as MSLN target molecules.

The two MSLN-BDs of the multispecific antibodies of the present invention bind to any region of the extracelllar part of MSLN, e.g. to Region I, Region II and/or Region III of MSLN. Preferably, the two MSLN-BDs of the multispecific antibodies of the present invention bind to Region I and/or Region II of MSLN, in particular to Region I of MSLN. Region I is the part of MSLN that is most distal from the cell surface, where MSLN is attached to.

The two MSLN-BDs of the multispecific antibodies of the present invention either bind the same or different epitopes on the MSLN target molecules. Preferably, the two MSLN-BDs of the multispecific antibodies of the present invention bind the same epitopes on the MSLN target molecules. The term "same epitope", as used herein, refers to individual protein determinants on the same protein capable of specific binding to an antibody, where these individual protein determinants are identical, i.e. consist of identical chemically active surface groupings of molecules such as amino acids or sugar side chains having identical three-dimensional structural characteristics, as well as identical charge characteristics. The term "different epitope", as used herein in connection with a specific protein target, refers to individual protein determinants on the same protein capable of specific binding to an antibody, where these individual protein determinants are not identical, i.e. consist of non-identical chemically active surface groupings of molecules such as amino acids or sugar side chains having different three-dimensional structural characteristics, as well as different charge characteristics. These different epitopes can be overlapping or non-overlapping.

The inventors of the present invention have now surprisingly found that the tri-specific molecule PRO2000 (biMSLN$_{high\,KD}$xCD3xhSA) is capable of killing target cells, which have an approximately 7-fold higher MSLN expression level than healthy MeT-5A cells (ATCC CRL-9444), as determined by flow cytometry, with high efficiency and with an $EC_{50}$ that is 2000-fold lower than the $EC_{50}$ for killing said MeT-5A cells, as determined in a T-cell driven cytotoxicity assay against said target cells and said MeT-5A cells (Figure 5). Thus, although PRO2000 exhibits a very high killing potency for high MSLN expressing target cells, the killing potency of PRO2000 towards healthy cells is much lower, indicating a potentially large therapeutic window for treatments using PRO2000. In contrast thereto, the potencies of a tri-specific reference molecule PRO1872 (MSLN$_{lowKD}$xCD3xhSA), which comprises one MSLN-BD having a more than 5-fold better binding affinity ($K_D$) than the MSLN-BDs of PRO2000, for killing said high MSLN expressing target cells and said healthy Met-5A cells does not differ significantly. This finding indicates that the therapeutic window for the use of the multispecific antibodies of the present invention in the treatment of cancer patients is significantly increased. In addition, the inventors of the present invention have surprisingly found that the $EC_{50}$ of the tri-specific molecules PRO2000 and PRO2100 (biMSLN$_{high\,KD}$xCD3xhSA) for killing target cells, which have an approximately 7-fold higher MSLN expression level than said healthy MeT-5A cells, as determined by flow cytometry, does not increase by more than 5-fold in the presence of 100 ng/ml soluble mesothelin (sMSLN), and in the case of PRO2000, by not more than 20-fold in the presence of 500 ng/ml soluble mesothelin (sMSLN), as determined in a T-cell driven cytotoxicity assay against said target cells. On the other hand, the $EC_{50}$ of the tri-specific reference molecule PRO1872 (MSLN$_{low\,KD}$xCD3xhSA) for killing said target cells increased by almost 10-fold in the presence of 100 ng/ml sMSLN and by more than 100-fold in the presence of 500 ng/ml sMSLN. Thus, the high killing potency of the tri-specific molecules PRO2000 and PRO2100 for high MSLN expressing target cells is only marginally affected by high concentrations of sMSLN. On the other hand, the killing potency of the tri-specific molecules PRO2000 and PRO2100 for healthy cells is further decreased in the presence of high concentration of sMSLN, indicating that the therapeutic window for their use in the clinic is even increased by the presence of sMSLN. This is an important finding, since high plasma-levels of soluble mesothelin-related protein (SMRP) are often observed in patients. The inventors obtained similar advantageous results for the CD8+ T cell activation potency of PRO2000. The above findings are even more surprising as it could not *a priori* be expected that all four binding domains remain functional without sterically or otherwise inhibiting each other in a complex multi-target, multi-cell situation.

Suitable MSLN-BDs for use in the multispecific antibody of the invention are binding domains provided in the present disclosure. The mesothelin-BDs of the invention include, but are not limited to, the humanized MSLN-binding domains whose sequences are listed in Table 1.

Suitable CD3-BDs for use in the multispecific antibody of the invention are binding domains provided in the present disclosure. The CD3-BDs of the invention include, but are not limited to, the humanized CD3-binding domains whose sequences are listed in Table 3.

Suitably, the multispecific antibody of the invention has two different specificities (MSLN and CD3). Suitably, the multispecific antibody of the invention is a bispecific antibody, which is bivalent for MSLN. The multispecific antibody of the present invention may comprise a further specificity (trispecific antibody) or specificities (tetraspecific or pentaspecific or hexaspecific antibody). In one embodiment, the multispecific antibody is bispecific (MSLN and CD3). In another embodiment, the multispecific antibody is trispecific (MSLN, CD3 and hSA).

Suitably, the antibody of the invention does not comprise an immunoglobulin Fc region polypeptide.

In order to increase the number of specificities/functionalities at the same or lower molecular weight, it is advantageous to use antibodies comprising antibody fragments, such as Fv, Fab, Fab' and F(ab')2 fragments and other antibody fragments. These smaller molecules retain the antigen-binding activity of the whole antibody and can also exhibit improved tissue penetration and pharmacokinetic properties in comparison to the whole immunoglobulin molecules. Whilst such fragments appear to exhibit a number of advantages over whole immunoglobulins, they also suffer from an increased rate of clearance from serum since they lack the Fc domain that imparts a long half-life *in vivo* (Medasan et al., 1997, J. Immunol. 158:2211-2217). Molecules with lower molecular weights penetrate more efficiently into target tissues (*e. g.* solid cancers) and thus hold the promise for improved efficacy at the same or lower dose.

The inventors have surprisingly found that an addition of human serum albumin binding domain (hSA-BD) to the multispecific antibody of the invention does not interfere with the ability of the other binding domains to bind to their respective targets. This finding is insofar surprising as it cannot *a priori* be expected that all four binding domains remain functional without sterically or otherwise inhibiting each other in a complex multi-target, multi-cell *in vivo* situation.

Suitably, the multispecific antibody of the present invention may comprise a further binding domain having specificity to human serum albumin. In one embodiment, the multispecific antibody comprises: (i) two MSLN-BD; (ii) at least one CD3-BD; and (iii) at least one hSA-BD.

The term "hSA" refers in particular to human serum albumin with UniProt ID number P02768. Human Serum Albumin (hSA) is 66.4 kDa abundant protein in human serum (50 % of total protein) composing of 585 amino acids (Sugio, Protein Eng, Vol. 12, 1999, 439-446). Multifunctional hSA protein is associated with its structure that allowed binding and transporting a number of metabolizes such as fatty acids, metal ions, bilirubin and some drugs (Fanali, Molecular Aspects of Medicine, Vol. 33, 2012, 209-290). HSA concentration in serum is around 3.5-5 g/dL. Albumin binding antibodies and fragments thereof may be used for example, for extending the *in vivo* serum half-life of drugs or proteins conjugated thereto.

In some embodiments, the hSA-BD is derived from a monoclonal antibody or antibody fragment.

Suitable hSA-BDs for use in the multispecific antibody of the invention are binding domains provided in the present disclosure. The hSA-BDs of the invention include, but are not limited to, the humanized hSA-binding domains whose sequences are listed in Table 4.

In particular, the hSA-BDs of the invention specifically bind to human serum albumin.

Other suitable hSA-BD for use in the multispecific antibody of the invention comprises or is derived from an antibody selected from the group consisting of: (i) polypeptides that bind serum albumin (see, for example, Smith et al., 2001, Bioconjugate Chem. 12:750-756; EP0486525; US6267964; WO 2004/001064; WO 2002/076489; and WO 2001/45746); (ii) anti-serum albumin binding single variable domains described in Holt et al., Protein Engineering, Design & Selection, vol 21, 5, pp283-288, WO 2004/003019, WO 2008/096158, WO 2005/118642, WO 2006/0591056 and WO 2011/006915; (iii) anti-serum albumin antibodies described in WO 2009/040562, WO 2010/035012 and WO 2011/086091.

Other variable domains of the invention include amino acid sequences that have been mutated, yet have at least 80, 85, 90, 91, 92, 93, 94, 95, 96, 97, 98 or 99 percent identity in the CDR regions with the CDR regions depicted in the sequences described in Tables 1, 3 and 4. Other variable domains of the invention include mutant amino acid sequences wherein no more than 1, 2, 3, 4 or 5 amino acids have been mutated in the CDR regions when compared with the CDR regions depicted in the sequence described in Tables 1, 3 and 4.

Suitably, the VH domains of the binding domains of the invention belong to a VH3 or VH4 family. In one embodiment, a binding domain of the invention comprises a VH domain belonging to the VH3 family. In the context of the present invention, the term "belonging to VHx family (or VLx family)" means that the framework sequences FR1 to FR3 show the highest degree of homology to said VHx family (or VLx, respectively). Examples of VH and VL families are given in Knappik et al., J. Mol. Biol. 296 (2000) 57-86, or in WO 2019/057787. A specific example of a VH domain belonging to VH3 family is represented by SEQ ID NO: 63, and a specific example of a VH domain belonging to VH4 family is represented by SEQ ID NO: 64. In particular, framework regions FR1 to FR3 taken from SEQ ID NO: 63 belong to VH3 family (Table 7, regions marked in non-bold). Suitably, a VH belonging to VH3 family, as used herein, is a VH comprising FR1 to FR3 having at least 85 %, particularly at least 90 %, more particularly at least 95 % sequence identity to FR1 to FR3 of SEQ ID NO: 63. Alternative examples of VH3 sequences, and examples of VH4 sequences, may be found in Knappik et al., J. Mol. Biol. 296 (2000) 57-86 or in WO 2019/057787. Suitably, the hSA-BD of the invention comprises: Vκ frameworks FR1, FR2 and FR3, particularly Vκ1 or Vκ3 frameworks, particularly Vκ1 frameworks FR1 to 3, and a framework FR4, which is selected from a Vκ FR4, and a Vλ FR4, particularly a Vλ FR4. Suitable Vκ1 frameworks FR1 to 3 as well as an exemplary Vλ FR4 are set forth in SEQ ID NO: 65 (Table 7, FR regions are marked in non-bold). Alternative examples of Vκ1 sequences, and examples of Vκ2, Vκ3 or Vκ4 sequences, may be found in Knappik et al., J. Mol. Biol. 296 (2000) 57-86. Suitable Vκ1 frameworks FR1 to 3 comprise the amino acid sequences having at least 70, 80, 90, 95 percent identity to amino acid sequences corresponding to FR1 to 3 and taken from SEQ ID NO: 65 (Table 7, FR regions are marked in non-bold). Suitable Vλ FR4 are as set forth in SEQ ID NO: 66 to SEQ ID NO: 72 and in SEQ ID NO: 73 comprising a single cysteine residue, particular in a case where a second single cysteine is present in the corresponding VH chain, particularly in position 51 (AHo numbering) of VH, for the formation of an inter-domain disulfide bond. In one embodiment, the VL domains of the present invention comprises Vλ FR4 having at least 70, 80, or 90 percent identity to an amino acid sequence selected from any of SEQ ID NO: 66 to SEQ ID NO: 73, particularly to SEQ ID NO: 66 or 73.

The binding domains of the invention comprises a VH domain listed in Tables 1, 3 and 4. Suitably, a binding domain of the invention comprises a VH amino acid sequence listed in one of Tables 1, 3 and 4, wherein no more than 20 amino acids in a framework sequence (for example, a sequence which is not a CDR) have been mutated (wherein a mutation is, as various non-limiting examples, an addition, substitution or deletion). Suitably, a binding domain of the present invention comprises a VH amino acid sequence listed in one of Tables 1, 3 and 4, wherein no more than 15 amino acids, particularly not more than 10 amino acids, particularly not more than 5 amino acids in a framework sequence (for example, a sequence which is not a CDR) have been mutated (wherein a mutation is, as various non-limiting examples, an addition, substitution or deletion). Other binding domains of the invention include amino acids that have been mutated, yet have at least 80, 85, 90, 91, 92, 93, 94, 95, 96, 97, 98 or 99 percent identity in the VH regions with the VH regions depicted in the corresponding sequences described in one of Tables 1, 3 and 4, including VH domains comprising at least positions 5 to 140 (AHo numbering), particularly at least positions 3 to 145 of one of the sequences shown in Tables 1, 3 and 4.

In particular, a binding domain of the invention comprises a VL domain listed in one of Tables 1, 3 and 4. Suitably, a binding domain of the invention comprises a VL amino acid sequence listed in one of Tables 1, 3 and 4, wherein no more than 20 amino acids in a framework sequence (for example, a sequence which is not a CDR) have been mutated (wherein a mutation is, as various non-limiting examples, an addition, substitution or deletion). Suitably, a binding domain of the invention comprises a VL amino acid sequence listed in one of Tables 1, 3 and 4, wherein no more than 15 amino acids, particularly not more than 10 amino acids, particularly not more than 5 amino acids in a framework sequence (for example, a sequence which is not a CDR) have been mutated (wherein a mutation is, as various non-limiting examples, an addition, substitution or deletion). Other binding domains of the invention include amino acids that have been mutated, yet have at least 80, 85, 90, 91, 92, 93, 94, 95, 96, 97, 98 or 99 percent identity in the VL regions with a VL region depicted in the sequences described in Tables 1, 3 and 4, including VL domains comprising at least positions 5 to 140 (AHo numbering), particularly at least positions 3 to 145 of one of the sequences shown in Tables 1, 3 and 4.

In the context of the present invention, the term "binding domain of the present invention" relates both to a binding domain as such, *i. e.* independent of a multispecific context, and, in particular, to a binding domain comprised in a multispecific construct, *e. g.* one of the binding domains comprised in a bispecific, trispecific or tetraspecific construct.

Suitably, a binding domain of the invention is selected from the group consisting of: a Fab, an Fv, an scFv, dsFv, a scAb, and STAB.

Suitably, a binding domain of the invention is an scFv antibody fragment.

The multispecific antibody of the invention may be in any suitable format.

Suitably, the binding domains of the multispecific antibody are operably linked. The binding domains of the multispecific antibody of the invention are capable of binding to their respective antigens or receptors simultaneously. The term "simultaneously", as used in this connection, refers to the simultaneous binding of at least one of the MSLN-BDs and the CD3-BD. In specific cases, e.g. in cases of target cells having a high density of MSLN on the cell surface, it might also be possible that three binding domains, *i. e.* both MSLN-BD and the CD3-BD, bind simultaneously.

The multispecific antibody of the invention comprises two MSLN-BD, and at least one CD3-BD, wherein said MSLN-BDs, and said CD3-BD are operably linked to each other.

The term "operably linked", as used herein, indicates that two molecules (*e. g.*, polypeptides, domains, binding domains) are attached so as to each retain functional activity. Two molecules can be "operably linked" whether they are attached directly or indirectly (*e. g.*, via a linker, via a moiety, via a linker to a moiety). The term "linker" refers to a peptide or other moiety that is optionally located between binding domains or antibody fragments of the invention. A number of strategies may be used to covalently link molecules together. These include but are not limited to polypeptide linkages between N- and C-termini of proteins or protein domains, linkage via disulfide bonds, and linkage via chemical cross-linking reagents. In one aspect of this embodiment, the linker is a peptide bond, generated by recombinant techniques or peptide synthesis. Choosing a suitable linker for a specific case where two polypeptide chains are to be connected depends on various parameters, including but not limited to the nature of the two polypeptide chains (*e. g.*, whether they naturally oligomerize), the distance between the N- and the C-termini to be connected if known, and/or the stability of the linker towards proteolysis and oxidation. Furthermore, the linker may contain amino acid residues that provide flexibility.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (e. g., 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, $(Gly-Ser)_n$, $(GSGGS)_n$ $(GGGGS)_n$ and $(GGGS)_n$, where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since both of these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single-chain antibodies.

Suitably, the multispecific antibody is in a format selected from any suitable multispecific, e. g. at least bispecific, format known in the art, which do not comprise immunoglobulin Fc region(s), including, by way of non-limiting example, formats based on a tandem scDb (Tandab), a linear dimeric scDb (LD-scDb), a circular dimeric scDb (CD-scDb), a tandem tri-scFv, a tribody (Fab-(scFv)$_2$), Fab-Fv$_2$, triabody, scDb-scFv, tetrabody, di-diabody, CODV, tandem-di-scFv, tandem tri-scFv, Fab-(scFv)$_2$, Fab-Fv$_2$, or CODV fused to the N- and/or the C-terminus of a heterodimerization domain other than heterodimeric Fc domains, and MATCH (described in WO 2016/0202457; Egan T. et al., MABS 9 (2017) 68-84) and DuoBodies (bispecific IgGs prepared by the Duobody technology) (MAbs. 2017 Feb/Mar;9(2):182-212. doi: 10.1080/19420862.2016.1268307). Particularly suitable, the multispecific antibody is a single-chain diabody (scDb)-scFv or a MATCH.

In one embodiment, the multispecific antibody of the invention does not comprise CH1 and/or CL regions.

In another embodiment, the multispecific antibody of the invention is in a format selected from the list consisting of scDb-scFv, triabody, and tribody. Particularly suitable for use herein is a scDb-scFv, in particular wherein one of said MSLN-BDs and said CD3-BD are in the form of a scDb and the second MSLN-BD is an scFv operably linked to said scDb.

The term "diabodies" refers to antibody fragments with two antigen-binding sites, which fragments comprise a VH connected to VL in the same polypeptide chain (VH-VL). By using a linker that is too short to allow pairing between the two domains on the same chain, the domains are forced to pair with the complementary domains of another chain to create two antigen-binding sites. Diabodies may be bivalent or bispecific. Diabodies are described more fully in, for example, EP 404 097, WO 93/01161, Hudson et al., Nat. Med. 9:129-134 (2003), and Hollinger et al., Proc. Natl. Acad. Sci. USA 90: 6444-6448 (1993). Triabodies and tetrabodies are also described in Hudson et al., Nat. Med. 9:129-134 (2003).

The bispecific scDb, in particular the bispecific monomeric scDb, particularly comprises two variable heavy chain domains (VH) or fragments thereof and two variable light chain domains (VL) or fragments thereof connected by linkers L1, L2 and L3 in the order VHA-L1-VLB-L2-VHB-L3-VLA, VHA-L1-VHB-L2-VLB-L3-VLA, VLA-L1-VLB-L2-VHB-L3-VHA, VLA-L1-VHB-L2-VLB-L3-VHA, VHB-L1-VLA-L2-VHA-L3-VLB, VHB-L1-VHA-L2-VLA-L3-VLB, VLB-L1-VLA-L2-VHA-L3-VHB or VLB-L1-VHA-L2-VLA-L3-VHB, wherein the VLA and VHA domains jointly form the antigen-binding site for the first antigen, and VLB and VHB jointly form the antigen-binding site for the second antigen.

The linker L1 particularly is a peptide of 2-10 amino acids, more particularly 3-7 amino acids, and most particularly 5 amino acids, and linker L3 particularly is a peptide of 1-10 amino acids, more particularly 2-7 amino acids, and most particularly 5 amino acids. In particular embodiments, the linker L1 and/or L3 comprises one or two units of four (4) glycine amino acid residues and one (1) serine amino acid residue $(GGGGS)_n$, wherein n=1 or 2, particularly n=1.

The middle linker L2 particularly is a peptide of 10-40 amino acids, more particularly 15-30 amino acids, and most particularly 20-25 amino acids. In particular embodiments, said linker L2 comprises one or more units of four (4) glycine amino acid residues and one (1) serine amino acid residue $(GGGGS)_n$, wherein n=1, 2, 3, 4, 5, 6, 7 or 8, particularly n=4.

In one embodiment, the multispecific antibody of the invention is a scDb-scFv. The term "scDb-scFv" refers to an antibody format, wherein a single-chain Fv (scFv) fragment is fused by a flexible Gly-Ser linker to a single-chain diabody (scDb). In one embodiment, said flexible Gly-Ser linker is a peptide of 2-40 amino acids, e. g., 2-35, 2-30, 2-25, 2-20, 2-15, 2-10 amino acids, particularly 10 amino acids. In particular embodiments, said linker comprises one or more units of four (4) glycine amino acid residues and one (1) serine amino acid residue $(GGGGS)_n$, wherein n=1, 2, 3, 4, 5, 6, 7 or 8, particularly n=2.

In one embodiment of the present invention, the multispecific antibody of the invention is in a MATCH format described in WO 2016/0202457; Egan T., et al., MABS 9 (2017) 68-84. In particular, in this embodiment, the multispecific antibody of the invention is in a MATCH3 or a MATCH4 format.

The multispecific antibody of the invention can be produced using any convenient antibody manufacturing method known in the art (see, e. g., Fischer, N. & Leger, O., Pathobiology 74 (2007) 3-14 with regard to the production of bispecific constructs; Hornig, N. & Farber-Schwarz, A., Methods Mol. Biol. 907 (2012)713-727, and WO 99/57150 with regard to bispecific diabodies and tandem scFvs). Specific examples of suitable methods for the preparation of the bispecific construct of the invention further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, e. g., Zhu et al., Cancer Lett. 86 (1994) 127-134); and Suresh et al., Methods Enzymol. 121 (1986) 210-228).

These methods typically involve the generation of monoclonal antibodies, for example by means of fusing myeloma cells with the spleen cells from a mouse that has been immunized with the desired antigen using the hybridoma technology (see, e. g., Yokoyama et al., Curr. Protoc. Immunol. Chapter 2, Unit 2.5, 2006) or by means of recombinant antibody engineering (repertoire cloning or phage display/yeast display) (see, e. g., Chames & Baty, FEMS Microbiol. Letters 189 (2000) 1-8), and the combination of the antigen-binding domains or fragments or parts thereof of two or more different monoclonal antibodies to give a bispecific or multispecific construct using known molecular cloning techniques.

The multispecific molecules of the invention can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N- succinimidyl-3- (2-pyridyldithio)propionate (SPDP), and sulfosuccinimidyl 4- (N- maleimidomethyl)cyclohaxane-I-carboxylate (sulfo-SMCC) (see e. g., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83), and Glennie et al., 1987 J. Immunol. 139: 2367-2375). Conjugating agents are SATA and sulfo-SMCC, both available from Pierce Chemical Co. (Rockford, 111).

When the binding specificities are antibodies, they can be conjugated by sulfhydryl bonding of the C-terminus hinge regions of the two heavy chains. In a particular embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb X mAb, mAb X Fab, Fab X F (ab')$_2$ or ligand X Fab fusion protein. A multispecific antibody of the invention can be a single-chain molecule comprising one single-chain antibody and a binding determinant, or a single-chain multispecific antibody comprising two binding determinants. Multispecific antibody may comprise at least two single-chain molecules. Methods for preparing multispecific antibodies and molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the multispecific antibodies to their specific targets can be confirmed by, for example, enzyme-linked immunosorbent assay (ELISA), radioimmunoassay (REA), FACS analysis, bioassay (e. g., growth inhibition), or Western Blot assay. Each of these assays generally detects the presence of protein-antibody complexes of particular interest by employing a labeled reagent (e. g., an antibody) specific for the complex of interest.

In a further aspect, the invention provides a nucleic acid encoding the multispecific antibody of the invention or fragments thereof or binding domains thereof. Such nucleic acid sequences can be optimized for expression in mammalian cells.

The term "nucleic acid" is used herein interchangeably with the term "polynucleotide(s)" and refers to one or more deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e. g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The invention provides substantially purified nucleic acid molecules which encode polypeptides comprising segments or domains of the multispecific antibody described above. When expressed from appropriate expression vectors, polypeptides encoded by these nucleic acid molecules are capable of exhibiting antigen-binding capacity or capacities of the multispecific antibody of the present invention.

Also provided in the invention are polynucleotides which encode at least one CDR region and usually all three CDR regions of the binding domains of the multispecific antibody of the present invention set forth in Tables 1, 3 and 4. Because of the degeneracy of the code, a variety of nucleic acid sequences will encode each of the immunoglobulin amino acid sequences.

The polynucleotide sequences can be produced by de novo solid-phase DNA synthesis or by PCR mutagenesis of an existing sequence (*e. g.*, sequences as described in the Examples below) encoding the multispecific antibody of the invention or fragments thereof or binding domains thereof. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetra. Lett., 22: 1859, 1981; and the solid support method of U.S. Pat. No. 4,458,066. Introducing mutations to a polynucleotide sequence by PCR can be performed as described in, *e. g.*, PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the invention are expression vectors and host cells for producing the multispecific antibody of the invention or fragments thereof or binding domains thereof.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e. g.*, bacterial vectors having a bacterial origin of replication and episomal mammalian vectors). Other vectors (*e. g.*, non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e. g.*, replication defective retroviruses, adenoviruses and adenoassociated viruses), which serve equivalent functions. In this particular context, the term "operably linked" refers to a functional relationship between two or more polynucleotide (*e. g.*, DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, *i. e.*, they are cis-acting. However, some transcriptional regulatory sequences, such as enhancers, need not be physically contiguous or located in close proximity to the coding sequences whose transcription they enhance.

Various expression vectors can be employed to express the polynucleotides encoding the multispecific antibody chains or binding fragments. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, *e. g.*, Harrington et al., Nat Genet. 15:345, 1997). For example, nonviral vectors useful for expression of the MSLN-binding polynucleotides and polypeptides in mammalian (*e. g.*, human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adenoassociated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (*e. g.*, enhancers) that are operably linked to the polynucleotides encoding a multispecific antibody chain or a fragment. In one embodiment, an inducible promoter is employed to prevent expression of inserted sequences except under inducing conditions. Inducible promoters include, *e. g.*, arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of a multispecific antibody chain or a fragment. These elements typically include an ATG initiation codon and adjacent ribosome binding site or other sequences. In addition, the efficiency of expression may be enhanced by the inclusion of enhancers appropriate to the cell system in use (see, *e. g.*, Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

The expression vectors may also provide a secretion signal sequence position to form a fusion protein with polypeptides encoded by inserted multispecific antibody of the invention or fragments thereof or binding domains thereof sequences. More often, the inserted multispecific antibody of the invention or fragments thereof or binding domains thereof sequences are linked to signal sequences before inclusion in the vector. Vectors to be used to receive sequences encoding binding domains of the multispecific antibody light and heavy chain variable domains sometimes also encode constant regions or parts thereof.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the multispecific antibody of the invention or fragments thereof or binding domains thereof can be either prokaryotic or eukaryotic. *E. coli* is one prokaryotic host useful for cloning and expressing the polynucleotides of the present invention. Other microbial hosts suitable for use include bacilli, such as *Bacillus subtilis*, and other enterobacteriaceae, such as Salmonella, Serratia, and various Pseudomonas species. In these prokaryotic hosts, one can also make expression vectors, which typically contain expression control sequences compatible with the host cell (*e. g.*, an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express MSLN-binding polypeptides of the invention. Insect cells in combination with baculovirus vectors can also be used.

In one embodiment, mammalian host cells are used to express and produce the multispecific antibody of the invention or fragments thereof or binding domains thereof. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, *e. g.*, Winnacker, FROM GENES TO CLONES, VCH Publishers, N.Y., N.Y., 1987. Expression vectors for mammalian host cells can include expression control sequences, such as an origin of replication, a promoter, and an enhancer (see, *e. g.*, Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP polIII promoter, the constitutive MPS V promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, et al., supra). Other methods include, *e. g.*, electroporation, calcium phosphate treatment, liposome-mediated transformation, injection and microinjection, ballistic methods, virosomes, immunoliposomes, polycatiomnucleic acid conjugates, naked DNA, artificial virions, fusion to the herpes virus structural protein VP22 (Elliot and O'Hare, Cell 88:223, 1997), agent-enhanced uptake of DNA, and ex vivo transduction. For long-term, high-yield production of recombinant proteins, stable expression will often be desired. For example, cell lines which stably express the multispecific antibody of the invention or fragments thereof or binding domains thereof can be prepared using expression vectors of the invention which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present invention thus provides a method of producing the antibody of the invention or antigen-binding fragment thereof, wherein said method comprises the step of culturing a host cell comprising a nucleic acid or a vector encoding the antibody of the invention or antigen-binding fragment thereof, whereby said antibody of the disclosure or a fragment thereof is expressed.

In one aspect, the present invention relates to a method of producing the multispecific antibody of the invention or a binding domain thereof or a fragment thereof, the method comprising the step of culturing a host cell expressing a nucleic acid encoding the multispecific antibody of the invention or a binding domain thereof or a fragment thereof. In particular, the present invention relates to a method of producing the multispecific antibody of the invention or a binding domain thereof or a fragment thereof, the method comprising (i) providing a nucleic acid sequence or two nucleic acid sequences encoding the multispecific antibody of the invention or a binding domain thereof, or a vector or two vectors encoding the multispecific antibody of the invention or a binding domain thereof, expressing said nucleic acid sequence or nucleic acid sequences, or said vector or vectors, and collecting said multispecific antibody or said binding domain from the expression system, or (ii) providing a host cell or host cells expressing a nucleic acid encoding the multispecific antibody of the invention or a binding domain thereof, culturing said host cell or said host cells; and collecting said multispecific antibody or said binding domain from the cell culture.

In a further aspect, the present invention relates to a pharmaceutical composition comprising the multispecific antibody of the invention, and a pharmaceutically acceptable carrier. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

A pharmaceutical composition of the invention can be administered by a variety of methods known in the art. The route and/or mode of administration vary depending upon the desired results. Administration can be intravenous, intramuscular, intraperitoneal, or subcutaneous, or administered proximal to the site of the target. The pharmaceutically acceptable carrier should be suitable for intravenous, intramuscular, subcutaneous, parenteral, spinal or epidermal administration (*e. g.*, by injection or infusion). Depending on the route of administration, the active compound, *i. e.*, the multispecific antibody of the invention, may be coated in a material to protect the compound from the action of acids and other natural conditions that may inactivate the compound.

Pharmaceutical compositions of the invention can be prepared in accordance with methods well known and routinely practiced in the art. See, *e. g.*, Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the multispecific antibody of the invention is employed in the pharmaceutical compositions of the invention. The multispecific antibodies of the invention are formulated into pharmaceutically acceptable dosage forms by conventional methods known to those of skill in the art. Dosage regimens are adjusted to provide the optimum desired response (*e. g.*, a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

The multispecific antibody of the invention is usually administered on multiple occasions. Intervals between single dosages can be weekly, monthly or yearly. Intervals can also be irregular as indicated by measuring blood levels of the multispecific antibody of the invention in the patient. Alternatively, the multispecific antibody of the invention can be administered as a sustained release formulation, in which case less frequent administration is required. Dosage and frequency vary depending on the half-life of the antibody in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In one aspect, the present invention relates to the multispecific antibody of the invention or the pharmaceutical composition of the invention for use as a medicament. In a suitable embodiment, the present invention provides the multispecific antibody or the pharmaceutical composition for use in treatment of a proliferative disease, in particular a cancer in a subject in need thereof.

In another aspect, the present invention provides the multispecific antibody or the pharmaceutical composition for use in a manufacture of a medicament for treatment of a proliferative disease, in particular a cancer.

In another aspect, the present invention relates to the use of the multispecific antibody or the pharmaceutical composition for treating a proliferative disease, in particular a cancer in a subject in need thereof.

In a further aspect, the present invention relates to the use of the multispecific antibody or the pharmaceutical composition in the manufacture of a medicament for treatment of a proliferative disease, in particular a cancer, in a subject in need thereof.

In another aspect, the present invention relates to a method of treating a subject comprising administering to the subject a therapeutically effective amount of the multispecific antibody of the present invention. In a suitable embodiment, the present invention relates to a method of treating a proliferative disease, in particular a cancer in a subject comprising administering to the subject a therapeutically effective amount of the multispecific antibody of the present invention.

The term "subject" includes human and non-human animals. Non-human animals include all vertebrates, *e. g.*, mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, *e. g.*, in a human, and includes: (a) inhibiting the disease, *i. e.*, arresting its development; and (b) relieving the disease, *i. e.*, causing regression of the disease.

The term "therapeutically effective amount" or "efficacious amount" refers to the amount of an agent that, when administered to a mammal or other subject for treating a disease, is sufficient to effect such treatment for the disease. The "therapeutically effective amount" will vary depending on the agent, the disease and its severity and the age, weight, etc., of the subject to be treated.

In one embodiment, the proliferative disease is a cancer. The term "cancer" refers to a disease characterized by the rapid and uncontrolled growth of aberrant cells. Cancer cells can spread locally or through the bloodstream and lymphatic system to other parts of the body. The terms "tumor" and "cancer" are used interchangeably herein, *e. g.*, both terms encompass solid and liquid, *e. g.*, diffuse or circulating, tumors. As used herein, the term "cancer" or "tumor" includes premalignant, as well as malignant cancers and tumors. The term "cancer" is used herein to mean a broad spectrum of tumors, including all solid and hematological malignancies. Examples of such tumors include, but are not limited to: a benign or especially malignant tumor, solid tumors, brain cancer, kidney cancer, liver cancer, adrenal gland cancer, bladder cancer, breast cancer, stomach cancer (*e. g.*, gastric tumors), esophageal cancer, ovarian cancer, cervical cancer, colon cancer, rectum cancer, prostate cancer, pancreatic cancer, lung cancer (*e. g.* non-small cell lung cancer and small cell lung cancer), vaginal cancer, thyroid cancer, melanoma (*e. g.,* unresectable or metastatic melanoma), renal cell carcinoma, sarcoma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (*e. g.*, Philadelphia chromosome-positive chronic myelogenous leukemia), acute lymphoblastic leukemia (*e. g.*, Philadelphia chromosome-positive acute lymphoblastic leukemia), non-Hodgkin's lymphoma, plasma cell myeloma, Hodgkin's lymphoma, a leukemia, and any combination thereof. In a preferred embodiment, the cancer is a cancer selected from mesothelioma, pancreatic cancer, and ovarian cancer.

The multispecific antibody of the present invention, or the composition of the present invention, inhibits the growth of solid tumors, but also liquid tumors. In a further embodiment, the proliferative disease is a solid tumor. The term "solid tumor" especially means a breast cancer, ovarian cancer, colon cancer, rectum cancer, prostate cancer, stomach cancer (especially gastric cancer), cervical cancer, lung cancer (e. g., non-small cell lung cancer and small cell lung cancer), and a tumor of the head and neck. Further, depending on the tumor type and the particular combination used, a decrease of the tumor volume can be obtained. The multispecific antibody of the present invention, or the composition of the present invention, is also suited to prevent the metastatic spread of tumors and the growth or development of micro metastases in a subject having a cancer.

**Sequence listing (mutations designated according to AHo numbering scheme, CDRs defined according to Numab CDR definition)**

*Table 1. Examples of low affinity MSLN binding domains of the present invention.*

| SEQ ID NO: | Ab region | Sequence |
|---|---|---|
| 1 | HCDR1 54-01-G02 | GFSLSSYAMG |
| 2 | HCDR2 54-01-G02 | YISTINNTYYASWAKG |
| 3 | HCDR3 54-01-G02 | REIRSGWVDYGFSI |
| 4 | LCDR1 54-01-G02 | QASQNIYSNLA |
| 5 | LCDR2 54-01-G02 | DASDLAS |
| 6 | LCDR3 54-01-G02 | QQVRSSSDIDNP |
| 7 | VH 54-01-G02-sc01 (PRO1783) | QVQLVESGGGLVQPGGSLRLSCAASGFSLSSYAMGWVRQAPGKGLEWIGYIST INNTYYASWAKGRFTISRDNSKNTVYLQMNSLRAEDTAVYYCAREIRSGWVD YGFSIWGQGTLVTVSS |
| 8 | VH 54-01-G02-sc01 N66A (54-01-G02-sc03) (PRO2197) | QVQLVESGGGLVQPGGSLRLSCAASGFSLSSYAMGWVRQAPGKGLEWIGYIST IANTYYASWAKGRFTISRDNSKNTVYLQMNSLRAEDTAVYYCAREIRSGWVD YGFSIWGQGTLVTVSS |
| 9 | VL 54-01-GO2-sc01 (PRO1783 and PRO2197) | DIQMTQSPSSLSASVGDRVTITCQASQNIYSNLAWYQQKPGKAPKLLIYDASDL ASGVPSRFSGSGSGTDFTLTISSLQPEDFATYYCQQVRSSSDIDNPFGTGTKVTV LG |
| 10 | HCDR2 54-01-G02 N66A | YISTIANTYYASWAKG |
| | | |
| SEQ ID NO: | Ab region | Sequence |
| 11 | HCDR1 54-32-A07 | GFSLSSYAMG |
| 12 | HCDR2 54-32-A07 | YISKIGTTYYASWAKG |
| 13 | HCDR3 54-32-A07 | RGSSSGGYLDDGFDP |
| 14 | LCDR1 54-32-A07 | QASQSISNYLA |
| 15 | LCDR2 54-32-A07 | DASDLAS |

(continued)

| SEQ ID NO: | Ab region | Sequence |
|---|---|---|
| 16 | LCDR3 54-32-A07 | QQVYDSNNVENV |
| 17 | VH 54-32-A07-sc02 (PRO1925) | QSQLVESGGGLVQPGGSLRLSCAVSGFSLSSYAMGWVRQAPGKGLEYIGYISKIGTTYYASWAKGRFTISKDNSKNTVYLQMNSLRAEDTAVYFCARGSSSSGGYLDDGFDPWGQGTLVTVSS |
| 18 | VL 54-32-A07-sc02 (PRO1925) | ALQMTQSPSSLSASVGDRVTITCQASQSISNYLAWYQQKPGKPPKFLIYDASDLASGVSSRFSGSGSGTDFTLTISSLQPEDFATYYCQQVYDSNNVENVFGTGTKVTVLGRGSSSGGGYLDDGFDP |

Table 2. Examples of reference MSLN binding domains.

| SEQ ID NO: | Description | Sequence |
|---|---|---|
| 19 | HCDR1 | GISVSNDYYMC |
| 20 | HCDR2 | CISTYIGNTHYASWAKG |
| 21 | HCDR3 | KNAGYPGYRYAIDL |
| 22 | LCDR1 | QASESIGNYLA |
| 23 | LCDR2 | SASTLAS |
| 24 | LCDR3 | QSTDYGDSYI |
| 25 | VH 54-22-H03-SC01 (PRO1795) | EVQLVESGGGLVQPGGSLRLSCAASGISVSNDYYMCWVRQAPGKGLEWIGCIS TYIGNTHYASWAKGRFTISRDNSKNTVYLQMNSLRAEDTAVYYCAKNAGYPG YRYAIDLWGQGTLVTVSS |
| 26 | VL 54-22-H03-sc01 (PRO1795) | DIQMTQSPSSLSASVGDRVTITCQASESIGNYLAWYQQKPGKAPKLLIYSASTL ASGVPSRFSGSGSGTDFTLTISSLQPEDFATYYCQSTDYGDSYIFGTGTKVTVLG |

*Table 3. Examples of CD3 binding domains of the present invention.*

| SEQ ID NO: | Description | Sequence |
|---|---|---|
| 27 | HCDR1 | GFSLSSYDMS |
| 28 | HCDR2 | ASYASGPTYYASWAKG |
| 29 | HCDR3 | RGGWTGTSHSNI |
| 30 | LCDR1 | QSSQSVFSNNYLA |
| 31 | LCDR2 | SASTLAS |
| 32 | LCDR3 | LGSYACSSADCYV |
| 33 | VH 28-21-D09 sc04 | EVQLVESGGGLVQPGGSLRLSCAASGFSLSSYDMSWVRQAPGKGLAWIGASYASGPTYYASWAKGRFTISRDNSKNTVYLQMNSLRAEDTATYFCARGGWTGTSHSNIWGQGTLVTVSS |
| 34 | VL 28-21-D09 sc04 | DIQMTQSPSSLSASVGDRVTITCQSSQSVFSNNYLAWFQQKPGQSPKRLIYSASTLASGVPSRFSGSGSGTDFTLTISSLQPEDFATYYCLGSYACSSADCYVFGTGTKVTVLG |

**Table 4. Examples of human serum albumin (hSA) binding domains of the present invention.**

| SEQ ID NO: | Description | Sequence |
|---|---|---|
| *Anti-hSA domain 19-01-H04-sc03* | | |
| 35 | HCDR1 | GFSLSSNAMG |
| 36 | HCDR2 | IISVGGFTYYASWAKG |
| 37 | HCDR3 | RDRHGGDSSGAFYL |
| 38 | LCDR1 | QSSESVYSNNQLS |
| 39 | LCDR2 | DASDLAS |
| 40 | LCDR3 | AGGFSSSSDTA |
| 41 | VH$_{19-01-H04-sc03}$ (PRO325) | EVQLVESGGGLVQPGGSLRLSCAASGFSLSSNAMGWVRQAPGKGLEYIGIISVG GFTYYASWAKGRFTISRDNSKNTVYLQMNSLRAEDTATYFCARDRHGGDSSG AFYLWGQGTLVTVSS |
| 42 | VL$_{19-01-H04-sc03}$ (PRO325) | DIQMTQSPSSLSASVGDRVTITCQSSESVYSNNQLSWYQQKPGQPPKLLIYDAS DLASGVPSRFSGSGSGTDFTLTISSLQPEDFATYYCAGGFSSSSDTAFGGGTKLT VLG |
| 43 | VH$_{19-01-H04-sc03 (G51C)}$ (PRO325_Cys) | EVQLVESGGGLVQPGGSLRLSCAASGFSLSSNAMGWVRQAPGKCLEYIGIISVG GFTYYASWAKGRFTISRDNSKNTVYLQMNSLRAEDTATYFCARDRHGGDSSG AFYLWGQGTLVTVSS |
| 44 | VL$_{19-01-H04-sc03 (G141C)}$ (PRO325_Cys) | DIQMTQSPSSLSASVGDRVTITCQSSESVYSNNQLSWYQQKPGQPPKLLIYDAS DLASGVPSRFSGSGSGTDFTLTISSLQPEDFATYYCAGGFSSSSDTAFGCGTKLT VLG |
| *Anti-hSA domain 23-13-A01-sc03* | | |
| 45 | HCDR1 | GFSFSSSYWIC |
| 46 | HCDR2 | CVFTGDGTTYYASWAKG |
| 47 | HCDR3 | RPVSVYYYGMDL |
| 48 | LCDR1 | QASQIISSRSA |
| 49 | LCDR2 | QASKLAS |
| 50 | LCDR3 | QCTYIDSNFGA |

28

(continued)

| | | |
|---|---|---|
| *Anti-hSA domain 23-13-A01-sc03* | | |
| 51 | VH<sub>23-13.A01.sc03</sub> (PRO459) | EVQLVESGGGLVQPGGSLRLSCAASGFSFSSSYWICWVRQAPGKGLEWVGCVF TGDGTTYYASWAKGRFTISRDNSKNTVYLQMNSLRAEDTATYFCARPVSVYY YGMDLWGQGTLVTVSS |
| 52 | VL<sub>23-13-A01-SC03</sub> (PRO459) | DVVMTQSPSSLSASVGDRVTITCQASQIISSRSAWYQQKPGQPPKLLIYQASKL ASGVPSRFSGSGSGTDFTLTISSLQPEDFATYYCQCTYIDSNFGAFGGGTKLTVL G |
| 53 | VH<sub>23-13-A01-sc03 (G51C)</sub> (PRO459_Cys) | EVQLVESGGGLVQPGGSLRLSCAASGFSFSSSYWICWVRQAPGKCLEWVGCVF TGDGTTYYASWAKGRFTISRDNSKNTVYLQMNSLRAEDTATYFCARPVSVYY YGMDLWGQGTLVTVSS |
| 54 | VL<sub>23-13-A01-SC03 (G141C)</sub> (PRO459_Cys) | DVVMTQSPSSLSASVGDRVTITCQASQIISSRSAWYQQKPGQPPKLLIYQASKL ASGVPSRFSGSGSGTDFTLTISSLQPEDFATYYCQCTYIDSNFGAFGCGTKLTVL G |

Table 5. Examples of multispecific molecules of the present invention.

| SEQ ID NO: | Description | Sequence |
|---|---|---|
| **biMSLNlow affinity x CD3 x hSA constructs** | | |
| **PRO2000 (MATCH4)** | | |
| 55 | CHAIN_1PRO2000 | DIQMTQSPSSLSASVGDRVTITCQASQNIYSNLAWYQQKPGKAPKLLIYDASDLA SGVPSRFSGSGSGTDFTLTISSLQPEDFATYYCQQVRSSSDIDNPFGTGTKVTVLG GGGGSGGGGSGGGGSGGGGSQVQLVESGGGLVQPGGSLRLSCAASGFSLSSYA MGWVRQAPGKGLEWIGYISTINNTYYASWAKGRFTISRDNSKNTVYLQMNSLR AEDTAVYYCAREIRSGWVDYGFSIWGQGTLVTVSSGGGGSGGGGSEVQLVESG GGLVQPGGSLRLSCAASGFSLSSYDMSWVRQAPGKGLAWIGASYASGPTYYAS |
| | | WAKGRFTISRDNSKNTVYLQMNSLRAEDTATYFCARGGWTGTSHSNIWGQGTL VTVSSGGGSGGGSEVQLVESGGGLVQPGGSLRLSCAASGFSFSSSYWICWVRQA PGKCLEWVGCVFTGDGTTYYASWAKGRFTISRDNSKNTVYLQMNSLRAEDTAT YFCARPVSVYYYGMDLWGQGTLVTVSS |
| 56 | CHAIN_2PRO2000 | DIQMTQSPSSLSASVGDRVTITCQASQNIYSNLAWYQQKPGKAPKLLIYDASDLA SGVPSRFSGSGSGTDFTLTISSLQPEDFATYYCQQVRSSSDIDNPFGTGTKVTVLG GGGGSGGGGSGGGGSGGGGSQVQLVESGGGLVQPGGSLRLSCAASGFSLSSYA MGWVRQAPGKGLEWIGYISTINNTYYASWAKGRFTISRDNSKNTVYLQMNSLR AEDTAVYYCAREIRSGWVDYGFSIWGQGTLVTVSSGGGGSGGGGSDVVMTQSP SSLSASVGDRVTITCQASQIISSRSAWYQQKPGQPPKLLIYQASKLASGVPSRFSG SGSGTDFTLTISSLQPEDFATYYCQCTYIDSNFGAFGCGTKLTVLGGGSGGGSDIQ MTQSPSSLSASVGDRVTITCQSSQSVFSNNYLAWFQQKPGQSPKRLIYSASTLAS GVPSRFSGSGSGTDFTLTISSLQPEDFATYYCLGSYACSSADCYVFGTGTKVTVL G |

| PRO2100 (MATCH4) | | |
|---|---|---|
| 57 | CHAIN_1<sub>PRO2100</sub> | DIQMTQSPSSLSASVGDRVTITCQASQNIYSNLAWYQQKPGKAPKLLIYDASDLA SGVPSRFSGSGSGTDFTLTISSLQPEDFATYYCQQVRSSSDIDNPFGTGTKVTVLG GGGGSGGGGSGGGGSGGGGSQVQLVESGGGLVQPGGSLRLSCAASGFSLSSYA MGWVRQAPGKGLEWIGYISTIANTYYASWAKGRFTISRDNSKNTVYLQMNSLR AEDTAVYYCAREIRSGWVDYGFSIWGQGTLVTVSSGGGGSGGGGSEVQLVESG GGLVQPGGSLRLSCAASGFSLSSYDMSWVRQAPGKGLAWIGASYASGPTYYAS WAKGRFTISRDNSKNTVYLQMNSLRAEDTATYFCARGGWTGTSHSNIWGQGTL VTVSSGGGSGGGSEVQLVESGGGLVQPGGSLRLSCAASGFSFSSSYWICWVRQA PGKCLEWVGCVFTGDGTTYYASWAKGRFTISRDNSKNTVYLQMNSLRAEDTAT YFCARPVSVYYYGMDLWGQGTLVTVSS |
| 58 | CHAIN_2<sub>PRO2100</sub> | DIQMTQSPSSLSASVGDRVTITCQASQNIYSNLAWYQQKPGKAPKLLIYDASDLA SGVPSRFSGSGSGTDFTLTISSLQPEDFATYYCQQVRSSSDIDNPFGTGTKVTVLG GGGGSGGGGSGGGGSGGGGSQVQLVESGGGLVQPGGSLRLSCAASGFSLSSYA MGWVRQAPGKGLEWIGYISTIANTYYASWAKGRFTISRDNSKNTVYLQMNSLR AEDTAVYYCAREIRSGWVDYGFSIWGQGTLVTVSSGGGGSGGGGSDVVMTQSP SSLSASVGDRVTITCQASQIISSRSAWYQQKPGQPPKLLIYQASKLASGVPSRFSG SGSGTDFTLTISSLQPEDFATYYCQCTYIDSNFGAFGCGTKLTVLGGGSGGSDIQ |
| | | MTQSPSSLSASVGDRVTITCQSSQSVFSNNYLAWFQQKPGQSPKRLIYSASTLAS GVPSRFSGSGSGTDFTLTISSLQPEDFATYYCLGSYACSSADCYVFGTGTKVTVL G |

Table 6. Examples of reference multispecific molecules.

| SEQ ID NO: | Description | Sequence |
|---|---|---|
| Constructs of Example 4: reference anti-MSLN$_{low\ KD}$XCD3xhSA | | |
| PRO1872 (scMATCH3) | | |
| 59 | scDb-scFv | DVVMTQSPSSLSASVGDRVTITCQASQIISSRSAWYQQKPGQPPKLLIYQASKLA SGVPSRFSGSGSGTDFTLTISSLQPEDFATYYCQCTYIDSNFGAFGCGTKLTVLGG GGGSEVQLVESGGGLVQPGGSLRLSCAASGFSLSSYDMSWVRQAPGKGLAWIG ASYASGPTYYASWAKGRFTISRDNSKNTVYLQMNSLRAEDTATYFCARGGWTG TSHSNIWGQGTLVTVSSGGGGSGGGGSGGGGSGGGGSDIQMTQSPSSLSASVGD RVTITCQSSQSVFSNNYLAWFQQKPGQSPKRLIYSASTLASGVPSRFSGSGSGTDF TLTISSLQPEDFATYYCLGSYACSSADCYVFGTGTKVTVLGGGGGSEVQLVESG GGLVQPGGSLRLSCAASGFSFSSSYWICWVRQAPGKCLEWVGCVFTGDGTTYY ASWAKGRFTISRDNSKNTVYLQMNSLRAEDTATYFCARPVSVYYYGMDLWGQ GTLVTVSSGGGGSGGGGSDIQMTQSPSSLSASVGDRVTITCQASESIGNYLAWYQ QKPGKAPKLLIYSASTLASGVPSRFSGSGSGTDFTLTISSLQPEDFATYYCQSTDY GDSYIFGTGTKVTVLGGGGGSGGGGSGGGGSGGGGSEVQLVESGGGLVQPGGS LRLSCAASGISVSNDYYMCWVRQAPGKGLEWIGCISTYIGNTHYASWAKGRFTI SRDNSKNTVYLQMNSLRAEDTAVYYCAKNAGYPGYRYAIDLWGQGTLVTVSS |

32

*Table 7. Other sequences related to the present invention.*

| SEQ ID NO: | Description | Sequence |
|---|---|---|
| *Linkers* | | |
| 60 | Linker | GGGGSGGGGSGGGGSGGGGS |
| 61 | Linker sequence unit | GGGGS |
| 62 | Generic linker sequence | (GmS)n, with m being selected from 2, 3 and 4 and with n being selected from 2, 3, 4, 5 |
| | | and 6 |
| *VH and VL sequences* | | |
| 63 | VH3 | EVQLVESGGGLVQPGGSLRLSCAAS*GFSFSANYYPC*WVRQAPGKGLEWIG*CIYG GSSDITYDANWTK*GRFTISRDNSKNTVYLQMNSLRAEDTAVYYCA*RSAWYSGW GGDL*WGQGTLVTVSS |
| 64 | VH4 | QVQLQESGPGLVKPSETLSLTCKVS*GFSFSNSYWIC*WIRQPPGKGLEWIG*CTFVG SSDSTYYANWAKG*RVTISVDSSKNQFSLKLSSVTAADTAVYYCA*RHPSDAVYGY ANNL*WGQGTLVTVSS |
| 65 | Vkappal | DIQMTQSPSSLSASVGDRVTITC*QASQSINNVLA*WYQQKPGKAPKLLIY*RASTLA SGVPSRFSGSGSGTDFTLTISSLQPEDFATYYC*QSSYGNYGD*FGTGTKVTVLG |
| *Vlambda FR4 sequencesTFGCGTKLTVLG* | | |
| 66 | Vlambda germline-based FR4 Sk17 | FGTGTKVTVLG |
| 67 | Vlambda germline-based FR4 Sk12 | FGGGTKLTVLG |
| 68 | Vlambda germline-based FR4 1 | FGGGTQLIILG |
| 69 | Vlambda germline-based FR4 2 | FGEGTELTVLG |
| 70 | Vlambda germline-based FR4 3 | FGSGTKVTVLG |
| 71 | Vlambda germline-based FR4 4 | FGGGTQLTVLG |
| 72 | Vlambda germline-based FR4 5 | FGGGTQLTALG |
| 73 | Vlambda germline-based FR4 G141C | FGCGTKVTVLG |

33

Throughout the text of this application, should there be a discrepancy between the text of the specification (*e. g.*, Tables 1 to 7) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

## Examples

### Example 1: Generation and pharmacodynamic characterization of anti-MSLN molecules:

In a first step, anti-MSLN antibody fragments that have a medium to low binding affinity to MSLN should be identified. The anti-MSLN antibody fragments should be suitable for use in multispecific antibody formats, in particular the MATCH3 and MATCH4 antibody format.

### Identification, selection and production of the anti-MSLN binding domains of the present invention

The identification, selection, humanization and production of the humanized scFv anti-MSLN binding domains of the present invention were performed analogous to the scFv anti-CD3 binding domains described in patent application PCT/EP2018/064630, which is herewith incorporated by reference.

From several of the identified monoclonal antibodies having the desired properties, in particular the desired affinities, scFv molecules were produced according to the following procedure.

### Humanization and expression:

Rabbit antibodies were humanized by CDR engraftment on a λ-capped Vk1/VH3 Fv scaffold and optional engraftment of specific rabbit framework residues. Each scFv was designed with a N-term-VL-peptide linker-VH-C-term orientation (peptide linker: (G4S)4).

Recombinant amino acid sequences were *de novo* synthesized and expression of scFv constructs was performed in CHO-S cells using CHOgro transient transfection kit (Mirus). Cultures were harvested after 5-7 days (cell viability < 70 %) of expression at 37°C by centrifugation and proteins were purified from clarified culture supernatants by Protein L or A affinity chromatography followed, if needed, by a polishing step by size-exclusion chromatography (SEC) using a Superdex S200 column.

### Quality control:

For the quality control of the manufactured material standard analytical methods, such as SE-HPLC, UV280 and SDS-PAGE were used.

*SE-HPLC*

SE-HPLC analysis Samples were passed through either a Shodex™ (Showa Denko, Cat. #: 554-1740) KW402.5-4F column (for scFv analysis) or a Shodex™ (Showa Denko, Cat. #: 554-1741) KW403- 4F column (MATCH protein analysis) with running buffer (Shodex™ KW402.5-4F: 250 mM NaCl, 50 mM NaOAc (Cat. #: A 1045), pH 6.0; Shodex™ KW403-4F: 35 mM $NaH_2PO_4$ (Cat. #: A 3905), 15 mM $Na_2HPO_4$ (Cat. #: A1372), 300 mM NaCl, pH 6.0) at a flow rate of 0.35 mL/min. Eluted protein was detected by absorbance at λ = 280 nm.

*SDS-PAGE*

Protein identity and degradation was assessed by SDS-PAGE analysis, loading denatured proteins onto Mini-iPROTEAN TGX™ precast gels (Bio-Rad Laboratories, Cat. #: 4569036) and staining electrophoresed protein with Coomassie brilliant blue solution. Molecular weight standard: BioRad Precision™ Plus (Cat. #: 161-03/04).
The manufacturing data of the produced scFv molecules are summarized in Table 8.

**Pharmacodynamic characterization of anti-mesothelin scFv antibody PRO1783 (low affinity)**

The humanized anti-mesothelin scFv antibody PRO1783 was evaluated for its primary pharmacodynamic properties including determination of binding kinetics and affinity to recombinant human and cynomolgus monkey MSLN in SPR, assessment of plasma-membranous binding to human and cynomolgus monkey MSLN expressing cell lines in cELISA and assessment of blockade of MSLN/MUC16 in cELISA.

Affinity to human and cynomolgus monkey MSLN in SPR

Affinity of scFv PRO1783 (derived from monoclonal antibody 54-01-G02) to recombinant human and cynomolgus monkey MSLN was determined by SPR analysis on a T200 device (Biacore, GE Healthcare). In this experiment, recombinant human and cynomolgus monkey MSLN (purchased from Peprotech and Sino Biological, respectively) was immobilized onto a CM5 sensor chip using a standard amine-coupling procedure. Then, scFv antibody PRO1783 was injected into the flow cells for 5 min at concentrations ranging from 90 to 0.12 nM and dissociation of the protein was allowed to proceed for 12 min. The dissociation ($k_d$) and association ($k_a$) rate constants and the equilibrium dissociation constant ($K_D$) were calculated with the Biacore T200 evaluation software (GE Healthcare) using one-to-one Langmuir binding model.
As shown in Table 9, PRO1783 bound to recombinant human MSLN in SPR with an affinity in low nanomolar range ($K_D$ = 2.91 nM). SPR measurement also demonstrated binding of PRO1783 to recombinant cynomolgus monkey MSLN, although with reduced affinity ($K_D$ = 30.06 nM, Table 10).

Binding to MSLN expressing cell lines by cELISA

*Binding to cells expressing human MSLN at high levels (H226 cell line)*

Binding of anti-MSLN scFv antibody PRO1783 to plasma-membranous MSLN was assessed by cELISA on H226 cancer cells. In brief, 20'000 NCI-H226 cells expressing MSLN or HEK293T (MSLN negative) were distributed to flat bottom tissue culture treated 96 well plates. The next day, plates were washed three times in overflow mode with 450 µl wash buffer (PBS, 0.2 % BSA) per well, 50 µl of each point of the serial dilution of PRO1783 and anti-MSLN reference antibody Amatuximab were added and plates were incubated for 1.5 h at room temperature (RT) under gentle agitation. After 3 washes with 450 µl wash buffer, 50 µl of HRP-coupled Protein L or HRP-coupled anti-human IgG antibody were added to each well. After 1 h incubation at RT on a nutating mixer, plates were washed three times with 450 µl of washing buffer per well prior to the addition of 50 µl TMB (3,3',5,5'-tetramethylbenzidine, KPL). After 10 min of development, the enzymatic reaction was stopped by addition of 50 µl of 1 M HCl per well and the plate was read at 450 nm using 690 nm as a reference wavelength.
Results of the experiment assessing the plasma-membranous binding of PRO1783 to H226 cell line expressing high levels of MSLN are shown in Table 11. The $EC_{50}$ for binding of PRO1783 to H226 cell line was found at concentration of 1.44 nM, which is roughly six times worse when compared to the value obtained for the reference antibody Amatuximab (compare rel. $EC_{50}$ values, Table 11). No binding of Amatuximab and PRO1783 was detected when mesothelin negative HEK293T cells were tested in cELISA (data not shown). Concentration-response curves of PRO1783 and Amatuximab in cELISA using H226 cell line are displayed in Figure 1.

Binding to cells expressing cynomolgus monkey MSLN (CHO recombinant cell line)

Cross-reactivity to cynomolgus monkey MSLN of anti-MSLN scFv antibody PRO1783 was tested in cELISA using recombinant CHO cell line expressing cynomolgus monkey MSLN. 20'000 CHO cells expressing cynomolgus monkey MSLN or CHO-K1 cells (cynomolgus monkey MSLN negative) were distributed to flat bottom tissue culture treated 96 well plates. Next day, plates were washed and serial dilutions of PRO1783 and anti-MSLN reference antibody Amatuximab were added as described in cELISA protocol using H226 cell line. After 1.5 h incubation at RT under gentle agitation, plates were washed again and HRP-coupled Protein L or HRP-coupled anti-human IgG antibody were added to detect binding of PRO1783 and Amatuximab, respectively. After 1 h incubation at RT on a nutating mixer, plates were washed and TMB was added to each well. After 10 min of development, the enzymatic reaction was stopped by addition of 50 µl of 1 M HCl per well and plate was read at 450 nm using 690 nm as a reference wavelength.

Results of cELISA using CHO cell line expressing cynomolgus monkey MSLN are shown in Table 12Fehler! Verweisquelle konnte nicht gefunden werden.. $EC_{50}$ of PRO1783 for binding to plasma-membranous cynomolgus monkey MSLN was found at a concentration of 12 nM, which is clearly inferior to the reference antibody Amatuximab (rel. $EC_{50}$ = 0.03). On the other hand, when compared to the binding to plasma-membranous human MSLN an increased half-maximal binding concentration of PRO1783 is obvious, which is in line with the results of SPR analysis demonstrating reduced affinity of PRO1783 to recombinant cynomolgus monkey MSLN protein. Concentration-response curves of PRO1783 and Amatuximab in cELISA using CHO cell line expressing cynomolgus monkey MSLN are displayed in Figure 2. No binding of Amatuximab and PRO1783 was detected when CHO-K1 wild type cells were tested in cELISA (data not shown).

Neutralization of MSLN/MUC16 interaction by competition ELISA

The potency of anti-MSLN scFv antibody PRO1783 to block the MSLN/MUC16 interaction was assessed in a competition ELISA. ELISA plates were coated by adding 50 µl of PBS containing 1 µg/ml MUC16 over night at 4°C. Next day, plates were washed three times in overflow mode with 450 µl wash buffer per well and 300 µl of blocking buffer was added to each well for 1h at RT on a nutating mixer. Then, biotinylated MSLN was diluted in blocking buffer to reach a final concentration of 1 ng/ml. Next, PRO1783 and Amatuximab were titrated in biotinylated MSLN containing blocking buffer and incubated for 1h at RT on a nutating mixer. ELISA plates were washed 3 times in overflow mode with 450 µl wash buffer per well and 50 µl of each concentration of the titration curve of PRO1783 and Amatuximab were added in duplicates to the ELISA plates. Plates were incubated 1.5h at RT under gentle agitation. After three washes with 450 µl of washing buffer per well, 50 µl of 10 ng/ml streptavidin-polyHRP40 was added to each well of the ELISA plate. After 1h incubation at RT, plates were washed three times with 450 µl wash buffer and developed for 5 to 10 minutes after addition of 50 µl TMB. Finally, the enzymatic reaction was stopped by addition of 50 µl of 1M HCl and the plate was read at 450 nm using 690 nm as a reference wavelength.

Results of the competition ELISA are shown in Table 13. The ICso to block human MSLN/MUC16 interaction by PRO1783 was found at a concentration of 0.5 nM, which is inferior to the reference antibody Amatuximab as shown by the rel. ICso value. Hence, PRO1783 is less potent as reference antibody Amatuximab to neutralize human MSLN/MUC16 interaction. Concentration-response curves of PRO1783 and Amatuximab in competition ELISA are displayed in Figure 3.

**Generation and pharmacodynamic characterization of reference anti-MSLN molecule PRO1795:**

The anti-MSLN binding domain PRO1795, which has a high binding affinity to MSLN, is used as reference binding domain.

The identification, selection, humanization and production of the humanized reference anti-MSLN binding domain PRO1795 was performed analogous to the anti-MSLN binding domains of the present invention and anti-CD3 molecule described herein.

Also, PRO1795 was evaluated for its primary pharmacodynamic properties including determination of binding kinetics and affinity to recombinant human and cynomolgus monkey MSLN in SPR, assessment of plasma-membranous binding to human and cynomolgus monkey MSLN expressing cell lines in cELISA and assessment of blockade of MSLN/MUC16 in cELISA. Results are summarized in Tables 9 to 13.

Further details about the generation, production and the pharmacodynamic characterisation of the reference anti-MSLN binding domain PRO1795 are described in the patent application EP20164913.4, which is herewith incorporated by reference.

**Table 8. Manufacture of scFvs.**

| Protein ID | Description | Titer [mg/L] post capure | Polishing step | Final titer [µg protein/mL Expression] | Purity SE-HPLC [% monomer] |
|---|---|---|---|---|---|
| PRO1783 | 54-01-G02-sc01 | 78.5 | SEC | 11 | 97.2 |
| PRO2197 | 54-01-G02-sc03 | 38.2 | NA | 30 | 99.2 |
| PRO1925 | 54-32-A07-sc02 | 34.8 | NA | 26 | 94.7 |

(continued)

| Protein ID | Description | Titer [mg/L] post capure | Polishing step | Final titer [μg protein/mL Expression] | Purity SE-HPLC [% monomer] |
|---|---|---|---|---|---|
| PRO2215 | 54-32-A07-sc04 | 15.9 | NA | 17 | 99.4 |

NA: not available

**Table 9: Binding kinetics and affinity of anti-MSLN scFv PRO1783 and reference anti-MSLN scFv PRO1795 to human MSLN in SPR.**

| Clone ID | Protein ID | Framework | Grafting Strategy | Affinity to human MSLN by SPR | | | |
|---|---|---|---|---|---|---|---|
| | | | | $k_a$ [M$^{-1}$ s$^{-1}$] | $k_d$ [s$^{-1}$] | $K_D$ [M] | Binding level normalized to theoretical Rmax [%] |
| 54-01-G02-sc01 | PRO1783 | VH3 | CDR | 7.80E+05 | 2.27E-03 | 2.91 E-09 | 27.9 |
| 54-22-H03-sc01 | PRO1795 | VH3 | CDR | 5.68E+05 | 1.83E-04 | 3.21E-10 | 36.2 |

**Table 10: Binding kinetics and affinity of anti-MSLN scFv PRO1783 and reference anti-MSLN scFv PRO1795 to cynomolgus monkey MSLN in SPR.**

| Clone ID | Protein ID | Framework | Grafting Strategy | Affinity to cynomolgus monkey MSLN by SPR | | | |
|---|---|---|---|---|---|---|---|
| | | | | $k_a$ [M$^{-1}$ s$^{-1}$] | $k_d$ [s$^{-1}$] | $K_D$ [M] | Binding level normalized to theoretical Rmax [%] |
| 54-01-G02-sc01 | PRO1783 | VH3 | CDR | 1.72E+06 | 5.27E-02 | 3.06E-08 | 17.4 |
| 54-22-H03-sc01 | PRO1795 | VH3 | CDR | 1.02E+06 | 1.10E-03 | 1.08E-09 | 30.9 |

**Table 11: Plasma-membranous binding of anti-MSLN scFv PRO1783 and reference anti-MSLN scFv PRO1795 to H226 cell line expressing high levels of human MSLN.**

| Clone ID | Protein ID | Framework | Grafting Strategy | Plasma-membranous binding to H226 cells by cELISA | | |
|---|---|---|---|---|---|---|
| | | | | $EC_{50}$ [nM] | rel. $EC_{50}$ ($EC_{50,}$ Amatuximab/ $EC_{50,}$ scFv) | rel. Maximum binding ($OD_{450-690nm,}$ scFv / $OD_{450-690nm,}$ Amatuximab) |
| 54-01-G02-sc01 | PRO1783 | VH3 | CDR | 1.44 | 0.16 | 0.57 |
| 54-22-H03-sc01 | PRO1795 | VH3 | CDR | 0.25 | 0.98 | 0.71 |

**Table 12: Plasma-membranous binding of anti-MSLN scFv PRO1783 and reference anti-MSLN scFv PRO1795 to CHO cell line expressing cynomolgus monkey MSLN.**

| Clone ID | Protein ID | Framework | Grafting Strategy | Plasma-membranous binding to CHO cells expressing cynomolgus monkey MSLN | | |
|---|---|---|---|---|---|---|
| | | | | $Ec_{50}$ [nM] | rel. $EC_{50}$ ($EC_{50, Amatuximab}$ / $EC_{50}$, scFv) | rel. Maximum binding ($OD_{450-690nm, scfv}$ / $OD_{450-690nm, Amatuximab}$) |
| 54-01-G02-sc01 | PRO1783 | VH3 | CDR | 12.00 | 0.03 | 0.89 |
| 54-22-H03-sc01 | PRO1795 | VH3 | CDR | 0.76 | 0.7 | 0.81 |

**Table 13: Blockade of human MSLN/MUC16 interaction by anti-MSLN scFv PRO1783 and by reference anti-MSLN scFv PRO1795 in competition ELISA.**

| Clone ID | Protein ID | Framework | Grafting Strategy | Blockade of MSLN/MUC16 interaction in competition ELISA | | |
|---|---|---|---|---|---|---|
| | | | | $IC_{50}$ [nM] | rel. $IC_{50}$ ($IC_{50, Amatuximab}$ / $IC_{50}$, scFv) | Maximum inhibition at highest sample conc. [%] |
| 54-01-G02-sc01 | PRO1783 | VH3 | CDR | 0.50 | 0.03 | 99.2 |
| 54-22-H03-sc01 | PRO1795 | VH3 | CDR | 0.02 | 0.87 | 99.0 |

Epitope mapping of anti-MSLN rabbit IgG clone 54-01-G02 (predecessor clone of low affinity anti-MSLN scFv domain PRO1783):

**Binding to human/mouse MLSN variants by cELISA**

In order to precisely define the binding region of selected anti-MSLN rabbit IgGs, binding level to HEK293T cells transiently transfected with seven human/mouse variants (V5 tagged) of the extracellular domain (ECD) of MSLN was assessed by cELISA (Figure 4). Plates were coated with 25'000 cells per well to flat bottom poly-D lysine treated 96-well plates. Next day, cells were transfected with the corresponding constructs and incubated at 37°C, 5 % $CO_2$. 24 h later, cells were washed with 450 $\mu$l wash buffer (PBS, 0.2 % BSA) and samples were added (250 ng/ml rIgG or anti-V5 tag antibody serial dilution) for 1.5 h at room temperature (RT) under gentle agitation. After 3 washes with 450 $\mu$l wash buffer, 50 $\mu$l of a HRP coupled goat and rabbit IgG antibody was added to each well. After 1 h incubation at RT on a nutating mixer, plates were washed three times with 450 $\mu$l of washing buffer per well prior to the addition of 50 $\mu$l TMB (3,3',5,5'-tetramethylbenzidine, KPL, Cat. No. 53-00-00). After 10 min development the enzymatic reaction was stopped by addition of 50 $\mu$l of 1 M HCl per well and plate was read at 450 nm using 690 nm as a reference wavelength. Binding level relative to binding of the anti-V5 antibody was calculated. A clear reduction of the binding level of the rIgG to a specific variant in comparison to the reference antibody (anti-V5 tag) would indicate localization of the rIgG epitope within the segment of human MSLN replaced by the respective mouse sequence.

When anti-MSLN rabbit IgG clones 54-01-G02 (predecessor clone of low affinity anti-MSLN scFv domain PRO1783) was tested in cELISA, a reduction of binding to the chimeric human/mouse variant V1 (most distal region of the ECD of human MSLN) was observed (reduction to 65 % binding relative to V5 reference antibody, Table 14), whereas the binding of 54-01-G02 to all other variants was above 90 %. These data suggest that V1 region of human MSLN represents an important region for binding of 54-01-G02. However, as 54-01-G02 can still bind substantially to human MSLN even in the absence of V1 region, other regions of the ECD of human MSLN are involved in binding of 54-01-G02 too. Regarding the rabbit IgG 54-22-H03 (predecessor clone of high affinity anti-MSLN scFv domain PRO1795), no binding region could be identified in cELISA using chimeric human/mouse variants of the ECD of human MSLN (Table 14).

**Table 14: Summary of binding to h/m MSLN variant transfected HEK293T cells of rabbit IgG clone 54-01-G02 and 54-22-H03.**

cELISA Transfection variants - Concentration of rIgG tested: 250 ng/mL

| | h/mMSLN-V1 | h/mMSLN-V2 | h/mMSLN-V3 | h/mMSLN-V4 | h/mMSLN-V5 | h/mMSLN-V6 | h/mMSLN-V7 |
|---|---|---|---|---|---|---|---|
| Clone ID | | | Binding (%) | [relative to anti-V5 antibody] | | | |
| 54-01-G02 | 65.0 | 93.7 | 98.8 | 93.3 | 101.0 | 101.3 | 104.0 |

**Example 2: Generation and testing of anti-CD3 molecules:**

The identification, selection, humanization as well as the production and characterization of the humanized anti-CD3 binding domain 28-21-D09 sc04 were performed as described in the patent applicatipon PCT/EP2018/064630, which is herewith incorporated by reference.

**Example 3: Generation and testing of anti-hSA molecules:**

The identification, selection, humanization as well as the production and characterization of the humanized anti-hSA binding domain 19-01-H04-sc03 and 23-13-A01-sc03 were performed as described in the patent applicatipon EP19206959.9, which is herewith incorporated by reference.

**Example 4: Generation and pharmacodynamic characterization of multispecific constructs of the present invention (biMSLN_low affinity x CD3 x hSA constructs):**

**Molecule architecture**

The MATCH is a format invented by Numab that consists solely of variable domains connected by different linkers that allow for the specific pairing of matching domain pairs only (Egan TJ et al., Novel multi-specific heterodimeric antibody format allowing modular assembly of variable domain fragments. MABS 9 (2017) 68-84). This format is particularly well suited for the convenient screening of different combinations of antigen-binding domains for optimal cooperativity. The MATCH can be expressed recombinantly from mammalian cells. For the purification, a conventional affinity chromatography step can be used.

The architecture of MATCH molecules is depicted in Figure 5. The MATCH4 format requires that the dimer subunits consist of a core of 2 split variable domain pairs, each respective subunit possessing either 2 VL domains or 2 VH domains positioned in tandem, thereby driving heterodimerization of the 2 protein chains. The dimer-forming tandem variable domains on the respective MATCH4 chains are organized in anti-parallel N-term-C-term orientation as their counterpart chain. Both chains are co-expressed in mammalian cells into fully functional tetra-specific molecules. Traditional Gly-Ser linkers between the variable domains were used to connect them as indicated in Figure 5. Further, the antiparallel MATCH4 format is amenable to the introduction of a disulfide bridge in one of the core domains as indicated in Figure 5. The corresponding MATCH3 format (not shown) is constructed and organized analogously, except that only one scFv binding domain is attached to the core of two split variable domain pairs, instead of two scFv binding domains as in case of the MATCH4.

Similar to the MATCH4 and MATCH3 format, the scMATCH3 format consists solely of variable domains connected by different linkers as depicted in Figure 5 (right). However, in this format split variable domains are located on a single peptide chain (sc) which assemble into fully functional trispecific molecules as shown in Figure 5 (right). As the MATCH4 and MATCH3 format, also scMATCH3 molecules can be expressed recombinantly in mammalian cells and for their purification, a conventional affinity chromatography step can be used.

Combining two to three rabbit antibodies humanized with a A-capped Fv scaffold, anti-parallel tetraspecific MATCH4 molecules according to the present invention as well as a reference trispecific scMATCH3 molecule, having only one high affinity MSLN-BD, were designed as summarized in Table 15.

**Manufacture**

Expression of MATCH constructs was performed in CHO-S cells using CHOgro transient transfection kit (Mirus). Cultures were harvested after 5-7 days (cell viability < 70 %) of expression at 37°C by centrifugation and proteins were purified from clarified culture supernatants by Protein L or A affinity chromatography followed, if needed, by a polishing step by size-exclusion chromatography (SEC) using a Superdex S200 column. For the quality control of the manufactured material standard analytical methods, such as SE-HPLC, UV280 and SDS-PAGE were used.

The manufacturing details for the produced molecules are summarized in Table 16.

**Pharmacodynamic characterization of multi-specific antibodies**

The following section describes the characterization of multi-specific molecules, which are either monovalent or bivalent for human MSLN, monovalent for human CD3$\epsilon$ and monovalent for human serum albumin (hSA). The bivalent anti-MSLN antibodies PRO2000 and PRO2100 (i.e. biMSLN$_{high\ KD}$XCD3xhSA) and monovalent anti-MSLN antibody PRO1872 (i.e. MSLN$_{low\ KD}$XCD3xhSA) were tested in SPR to assess their binding kinetics and affinities to recombinant human MSLN and human CD3$\epsilon$ in SPR.

Affinity to human MSLN in SPR

Affinity of multi-specific anti-MSLN antibodies to recombinant human MSLN was determined by SPR analysis on a T200 device (Biacore, GE Healthcare). In this experiment, recombinant human MSLN (purchased from Peprotech) was immobilized onto a CM5 sensor chip as described above. Multi-specific antibodies were injected into the flow cells and the binding kinetics as well as the equilibrium dissociation constant ($K_D$) were calculated as described above.

As shown in Table 17 monovalent anti-MSLN antibody PRO1872 bound to recombinant human MSLN in SPR with a binding affinity in sub-nanomolar range ($K_D$ = 0.187 nM). A similar binding affinity to human MSLN was found for the corresponding anti-MSLN scFv antibody PRO1795 ($K_D$ = 0.321 nM, domain 54-22-H03-sc01, data not shown). Bivalent anti-MSLN antibody PRO2000 showed a binding affinity to recombinant human MSLN in low nanomolar range ($K_D$ = 1.06 nM), which is superior to the binding affinity found for the corresponding anti-MSLN scFv antibody PRO1783 $K_D$ = 2.91 nM, domain 54-01-G02-sc01, Table 9). In this SPR setup, bivalent PRO2000 binds to immobilized human recombinant MSLN and thus increased affinity can be explained by avidity effects.

Affinity to human CD3$\epsilon$ in SPR

Affinity of multi-specific anti-MSLN antibodies to recombinant human CD3$\epsilon$ was determined by SPR analysis on a T200 device (Biacore, GE Healthcare). In this experiment, human recombinant CD3$\epsilon$ protein (Sino Biological) was immobilized on a CM5 sensor chip (GE healthcare) by amine-coupling. Serial dilutions of anti-MSLN multi-specific antibodies in HBS-T buffer (10mM HEPES, 150 mM NaCl, and 0.05 % Tween 20, pH 7.4) were injected into the flow cells at a flow rate of 30 $\mu$l/min for 5 min. Dissociation of the antibodies from the CD3$\epsilon$ on the CM5 chip was allowed to proceed for 12 min. After each injection cycle, surfaces were regenerated with one injection of 10 mM Glycine HCl, pH 2. The apparent dissociation ($k_d$) and association ($k_a$) rate constants and the apparent dissociation equilibrium constant ($K_D$) were calculated with the Biacore analysis soft-ware (BIAevaluation, GE Healthcare) using one-to-one Langmuir binding model and quality of the fits was monitored based on Chi2 and U-value, which is a measure for the quality of the curve fitting. Since the fits using the one-to-one Langmuir binding model showed suboptimal quality of curve fitting, the $K_D$ was in addition calculated using a two-state reaction model. This model describes a 1:1 binding of analyte to immobilized ligand followed by a conformational change that stabilizes the complex.

As shown in Table 18, anti-MSLN antibodies PRO1872 and PRO2000 both monovalent for CD3$\epsilon$ and harboring the same anti-CD3 domain (28-21-D09-sc04) bound to recombinant human CD3$\epsilon$ in SPR with a similar binding affinity in nanomolar range (PRO1872, $K_D$ = 12.1 nM; PRO2000, $K_D$ = 20.0 nM).

Affinity to human serum albumin at pH 5.5 in SPR

The binding kinetics to human serum albumin (hSA, Sigma Aldrich, cat. A3782) was assessed by SPR on a T200 device (Biacore, GE Healthcare). HSA was immobilized on a sensor chip (CM5 sensor chip, GE healthcare) by amine-coupling. Serial dilutions of anti-MSLN multi-specific antibodies ranging from 0.7 to 180 nM diluted in running buffer (PBS-Tween20) at pH 5.5 were injected into the flow cells for 5 min. The dissociation time was set to 12 min. The apparent dissociation ($k_d$) and association ($k_a$) rate constants and the apparent dissociation equilibrium constant ($K_D$) were calculated using one-to-one Langmuir binding model as described above.

As shown in Table 19, anti-MSLN antibodies PRO1872 (anti-hSA domain: 23-13-A01-sc02) bound to recombinant hSA in SPR with a binding affinity in sub-nanomolar range ($K_D$ = 0.175 nM).

**Table 15: Architecture of MATCH4 molecules and reference scMATCH3 molecule.**

| PRO ID | Format | Domain 1 | Specificity 1 | Domain 2 | Specificity 2 | Domain 3 | Specificity 3 | Domain 4 | Specificity 4 |
|---|---|---|---|---|---|---|---|---|---|
| PRO2000 | MATCH4 | 54-01-G02-sc01 | MSLN | 23-13-A01-sc02, VL-G141C/VH-G51C* | hSA | 28-21-D09-sc04 | CD3 | 54-01-G02-sc01 | MSLN |
| **PRO2100** | MATCH4 | 54-01-G02-sc01 N66A | MSLN | 23-13-A01-sc02, VL-G141C/VH-G51C* | hSA | 28-21-D09-sc04 | CD3 | 54-01-G02-sc01 N66A | MSLN |
| PRO1872 | scMATCH3 | 28-21-D09-sc04 | CD3 | 23-13-A01-sc02, VL-G141C/VH-G51C3 | hSA | 54-22-H03-sc01 | MSLN | NA | NA |

*AHo numbering

**Table 16: Manufacture of MATCH4 molecules**

| Protein ID | Titer post capture [mg/L] | Polishing step | Final titer [mg/L] | Purity SE-HPLC [% monomer] |
|---|---|---|---|---|
| PRO2000 | 21.3 | SEC | 10.7 | 97.9 |
| PRO2100 | 17.6 | NA | 15.6 | 97.1 |

SEC: size exclusion chromatography
NA: not available

**Table 17: Binding kinetics and affinity of anti-MSLN multi-specific antibodies to human MSLN in SPR.**

| PRO ID | Format | anti-MSLN domain | Affinity to human MSLN by SPR | | | Binding level normalized to theoretical Rmax [%] |
|---|---|---|---|---|---|---|
| | | | $k_a$ [M$^{-1}$ S$^{-1}$] | $k_d$ [S$^{-1}$] | $K_D$ [M] | |
| PRO1872 | scMATCH3 | 54-22-H03-sc01 | 2.65E+05 | 4.96E-05 | 1.87E-10 | 25.3 |
| PRO2000 | MATCH4 | 54-01-G02-sc01 | 3.98E+05 | 4.21 E-04 | 1.06E-09 | 10.5 |
| **PRO2100** | MATCH4 | 54-01-G02-sc01_N62A | not measured | | | |

**Table 18: Binding kinetics and affinity of anti-MSLN multi-specific antibodies to human CD3ε in SPR.**

| PRO ID | Format | anti-MSLN domain | Affinity to human CD3e by SPR | | | | | | | Binding level normalized to theoretical Rmax [%] |
|---|---|---|---|---|---|---|---|---|---|---|
| | | | $k_a1$ [M$^{-1}$ S$^{-1}$] | $k_d1$ [S$^{-1}$] | $K_D1$ [M] | $k_a2$ [M$^{-1}$ S$^{-1}$] | $k_d2$ [S$^{-1}$] | $K_D2$ [M] | $K_D$ [M] | |
| PRO1872 | scMATCH3 | 54-22-H03-sc01 | 2.93 E+05 | 5.08 E-03 | 1.73 E-08 | 2.10 E-03 | 4.84 E-03 | 2.30 E+00 | 1.21 E-08 | 28.7 |
| PRO2000 | MATCH4 | 54-01-G02-SC01 | 1.17 E+05 | 3.91 E-03 | 3.34 E-08 | 1.92 E-03 | 2.87 E-03 | 1.49 E+00 | 2.00 E-08 | 27.6 |
| **PRO2100** | MATCH4 | 54-01-G02-sc01-N62A | not measured | | | | | | | |

**Table 19: Binding kinetics and affinity of anti-MSLN multi-specific antibodies to hSA at pH5.5 in SPR.**

| PRO ID | Format | anti-MSLN domain | Affinity to hSA at pH5.5 by SPR | | | Binding level normalized to theoretical Rmax [%] |
|---|---|---|---|---|---|---|
| | | | $k_a$ [M$^{-1}$ s$^{-1}$] | $k_d$ [s$^{-1}$] | $K_D$ [M] | |
| PRO1872 | scMATCH3 | 54-22-H03-sc01 | 3.66E+05 | 6.39E-05 | 1.75E-10 | 71.2 |
| PRO2000 | MATCH4 | 54-01-G02-sc01 | not measured | | | |
| **PRO2100** | MATCH4 | 54-01-G02-sc01-N62A | not measured | | | |

## Example 5: Determination of mesothelin density on cell surface of target cell lines:

### Introduction

One objective is to compare the ability of the multispecific molecules, monovalent or bivalent for mesothelin binding, to target cell lines exhibiting different levels of mesothelin at their cell surface. Therefore, plasma membranous mesothelin expression was quantified on the different cell lines.

### Method

The Antibody Binding Capacity (ABC) on cancer cell lines expressing various levels of mesothelin and on healthy mesothelial tissue was assessed by FC (flow-cytometry) using Quantum Simply Cellular anti-human IgG kit (Bangs Laboratories). Briefly, 1 mg of anti-mesothelin antibody (7D9.3, Genentech) was conjugated with Alexa Fluor 488 using the Lightning-Link Rapid conjugation kit (Expedeon) following manufacturer's instructions. Receptor density values are reported as the antibody binding capacity (ABC). ABC values were derived from standard curves generated with Quantum Simply Cellular beads anti-human IgG (Bangs Laboratories, Inc.). These beads consist of four populations of microspheres that are each conjugated to a distinct number of anti-human IgG molecules per bead. As a first step, increasing concentrations of Alexa Fluor 488-labelled anti-mesothelin antibody were tested on the bead population with the highest amount of binding sites to determine the saturating antibody concentration, which was used during quantification as described by the manufacturer's protocol. Then, the beads and test samples were stained according to the manufacturer's instructions with the corresponding saturating concentration of Alexa Fluor 488 labelled anti-mesothelin antibody and were run on the same day and at the same photomultiplier tube settings as the test samples. To calculate ABC values, the geometric means for the four Quantum Simply Cellular bead populations were analyzed using the NovoExpress software (ACEA Biosciences). The QuickCal v. 2.3 Excel spreadsheet-based analysis template (Bangs Laboratories, Inc) was used to create a standard curve by linear regression. R square values were typically $\geq 0.99$. ABC values for the Alexa Fluor 488-anti-mesothelin antibody labelled samples were interpolated from the standard curve.

### Results

Mesothelin density on the plasma membrane of three cancer cell lines (H226, H292 and HPAC) and one cell line derived from heathy mesothelial tissue (MeT-5A; (ATCC® CRL-9444™); supplier: ATCC) was determined using the Quantum Simply Cellular beads. Data obtained are presented in Table 20. H226 cells show the highest expression level followed by the HPAC cell line, which exhibit a 4-fold lower expression. A comparable mesothelin expression level was found on H292 and MeT-5A cell lines which was 8 to 10-fold lower than the expression observed on the cell surface of the H226 cells.

**Table 20: Mesothelin density on cancer cells is represented as the average of antibody binding capacity (ABC) of each cell line and was quantified by flow cytometry.**

| Cell line | Mesothelin density (antibody binding capacity) |
|---|---|
| H226 | 41858 |
| H292 | 4660 |
| HPAC | 12825 |
| Met-5A | 5503 |

## Example 6: Cytotoxicity assay (T-cell driven target cell depletion):

### Introduction

To assess the ability of biMSLN$_{high KD}$XCD3xhSA to selectively direct T cells to kill mesothelin expressing cells compared to the MSLN$_{low KD}$XCD3xhSA, a cytotoxicity assay using cell lines expressing different mesothelin densities on their cell surface was performed in the presence of human PBMCs. In addition, the impact of the presence of soluble mesothelin (sMSLN) on the potencies of the molecules was also assessed in this assay. Simultaneous binding to mesothelin on cancer cells and CD3ε by MSLN$_{low KD}$XCD3xhSA tri-specific molecules leads to cross-linking of CD3ε on T cells and activates a signaling cascade that triggers T cell activation (CD69 upregulation, cytokine secretion) and the release of cytotoxic granules, which ultimately results in target cell killing.

**Methods**

Blood cell fractionation

Human peripheral blood mononuclear cells (PBMC) were isolated from fresh blood of healthy volunteers using the lymphocyte separation medium Lymphoprep (Stemcell technologies) according to manufacturer's instructions. In this set of experiments, blood from three different donors (donor #1, donor #2 and donor #3) were used. The properties of the blood of the individual donors differ greatly, in particular with regard to the amount and reactivity of the CD8+ T cells comprised therein. Consequently, the killing potencies among these blood derived CD8+ T cell samples varies over a wide range. This results in different killing potencies and CD8+ T cell activation potencies with the same test molecule in presence of the same target cells, as observed in the examples disclosed herein.

Briefly, blood was diluted 1:2 with human PBMC isolation buffer (PBS, 2 % FCS, 2 mM EDTA) and applied to Leucosep tubes containing recommended amount of Lymphoprep medium. LeucoSep tubes were centrifuged for 30 min at 800 x g without brake at RT. Then, the cell layer containing PBMCs was collected and washed twice with human PBMCs isolation buffer and red blood cells were lysed using red blood cells lysis buffer for 5 min at RT. Isolated human cells were then washed once with their respective isolation buffer and once with assay medium (RPMI-1640, 10 % FCS). After platelet removal, isolated PBMCs were resuspended in assay medium at a density of $3 \times 10^6$ viable cells per ml.

Flow cytometry-based *in vitro* cytotoxicity assay (FC assay) and CD8+ T cell activation:

Three cancer cell lines, H226 cells (high mesothelin density), HPAC cells (intermediate mesothelin density), and H292 cells (low mesothelin density) as well as the MeT-5A cell line derived from healthy mesothelial tissue (low mesothelin density), were used as target cells. 5'000 viable target cells previously labelled with PKH67 and diluted in 75 $\mu$l of assay medium (RPMI-1640, 10 % FCS) were added to 96-well plates. When applicable, assay buffer containing 50, 100 or 500 ng/ml soluble mesothelin was used. 25 $\mu$l of 6-fold concentrated test proteins were diluted in assay medium and added to appropriate wells. 150'000 viable effector cells (PBMCs) diluted in 50 $\mu$l assay medium were added to each well (E:T ratio of 30:1) and plates were mixed on a nutating mixer at RT prior to their incubation at 37°C, 5 % CO2. After 40 h, cells were trypsinized, resuspended in staining buffer (PBS, 2 % BCS, 2 mM EDTA) and transferred into non-binding plates.

Cells were stained for different markers such as CD69, CD8, CD4, CD11c and Annexin-V. For analysis, the focus was on apoptotic and dead target cells and activated CD8+ T cells. Target cells were identified by green fluorescence (PKH67) and their viability was analyzed by Annexin-V APC. Effector cells (CD8+ cells) were identified by detecting CD8 on their surface (anti-CD8 PerCP-Cy5.5). Activation of CD8+ T cells was finally detected by quantification of CD69 expression (anti-CD69 PE). CD4 was used to discriminate between CD8+ and CD4+ T cells. CD11c was used to stain monocytes and dendritic cells and to improve gating of target cells. For all markers, with the exception of Annexin-V, the cells were incubated for 30 min at RT under gentle agitation. Cells were washed once with staining buffer, once with Annexin binding buffer and Annexin-V staining was performed for 30 min at RT under agitation. Cells were washed once with Annexin-V binding buffer and flow cytometry analysis was done on a Novocyte Flow Cytometer.

The percentage of specific target cells lysis was calculated according to the following equation:

$$Specific\ lysis\ of\ target\ cells\ [in\ \%] = \left[1 - \frac{Viability\ target\ cells\ of\ sample}{average\ viability\ of\ control\ samples}\right] x\ 100$$

The percentage of activated CD8+ T cells corresponds to the proportion of CD69+ CD8+ T cells.

Results

Cytotoxic potential and effect on CD8+ T cell activation of MATCH molecules PRO2000 (MATCH-4: biMSLN$_{highKD}$XCD3xhSA) and PRO1872 (scMATCH-3: MSLN$_{lowKD}$XCD3xhSA) was assessed using a flow cytometry based cytotoxicity assay. Data obtained when using the high mesothelin expressing cell line H226 and the low mesothelin expressing MeT-5A cells derived from healthy tissue are presented in Table 21 and 22, and concentration response curves for the MATCH molecules are presented in Figure 6. Both molecules are highly potent on high mesothelin expressing H226 cells. The bivalent mesothelin targeting PRO2000 is 75-fold more potent than the monovalent mesothelin targeting PRO1872. Conversely, on MeT-5A cells expressing low mesothelin levels, the monovalent mesothelin binding molecule PRO1872 shows 16-fold higher potency to kill target cells compared to PRO2000. On high expressing cells, PRO2000 has a killing potency (EC$_{50}$) of 0.07 pM and PRO1872 of 5.31 pM, whereas on MeT-5A cells, PRO2000 shows an EC$_{50}$ of 144.70 pM and PRO1872 of 8.88 pM. Similar data are observed for CD8+ T cells activation in the respective conditions.

Furthermore, cytotoxic activity and effect on CD8+ T cell activation of PRO2000 and PRO1872 were tested on two other target cancer cell lines expressing intermediate and low mesothelin levels, HPAC and H292 cells, respectively (Table 22 and Figure 7). As observed previously PRO1872 is more potent than the bivalent molecule targeting mesothelin, PRO2000, on cells having a low mesothelin expression. In contrast, PRO2000 and PRO1872 have a similar potency on HPAC cells, which exhibit an intermediate mesothelin density. On HPAC, PRO2000 has a killing potency of 40.75 pM and PRO1872 of 30.26 pM, whereas on low mesothelin expressing H292 cells PRO2000 shows an $EC_{50}$ of 652.2 pM and PRO1872 of 91.03 pM. Similar data were observed for CD8+ T cell activation in the respective conditions, except that PRO2000 is 4-fold more potent than PRO1872 in presence of HPAC cells.

Several studies report serum concentrations of soluble mesothelin of several hundreds of ng/ml in cancer patients. Therefore, we evaluated the impact of the presence of soluble mesothelin on the potency of the molecules to kill target cells.

Cytotoxic potential and effect on CD8+ T cell activation of MATCH molecules PRO2000 and PRO1872 were compared using high mesothelin expressing H226 cells in the absence or presence of 50 ng/ml or 500 ng/ml of soluble mesothelin (sMSLN). Data obtained are presented in Table 23 and 24 and concentration response curves of the molecules are presented in Figure 8. Potencies of both molecules were negatively affected by soluble mesothelin in a dose dependent manner. The bivalent mesothelin targeting PRO2000, which has a lower monovalent binding affinity to mesothelin than PRO1872, shows a 17-fold reduction of killing potency in presence of 500 ng/ml sMSLN in comparison to the potency observed in absence of sMSLN. In contrast, the molecule monovalent for mesothelin PRO1872 with a better monovalent affinity for mesothelin, shows a 106-fold lower potency in presence of 500 ng/ml sMSLN as compared to the potency in absence of sMSLN. Similar data are observed for the CD8+ T cell activation in the respective conditions.

In addition, a variant of PRO2000, PRO2100 was characterized in order to show that both molecules have equivalent potency to kill target cells. In PRO2100 a potential glycosylation site has been mutated to prevent glycosylation.

Cytotoxic potential of MATCH4 molecules PRO2000 and PRO2100 was compared using the flow cytometry based cytotoxicity assay in presence of high mesothelin expressing H226 cells and low mesothelin expressing mesothelial cells, MeT-5A. PRO1872 was included as well. Data obtained are presented in Table 25 and concentration response curves are presented in Figure 9. None of the molecules tested show killing of the low mesothelin expressing MeT-5A cells. In the presence of H226 target cells, PRO2000 and PRO2100 show very similar killing potencies in absence or presence of sMSLN. In absence of sMSLN, potencies are 0.7 pM for PRO2000 and 1.54 pM for PRO2100. In presence of 100 ng/ml sMSLN potencies are shifted by a factor of 2 (3.56 pM for PRO2100) and a factor of 5 (3.45 pM for PRO2000). In contrast, in absence of sMSLN the molecule with the better monovalent affinity to mesothelin, PRO1872 is 10- and 20-fold less potent than PRO2100 and PRO2000, respectively ($EC_{50}$ PRO1872 = 13.38 pM). Furthermore, PRO1872 is 10-times less potent in presence of 100 ng/ml sMSLN.

## Example 7: Binding to target cells of MATCH molecules

### Introduction

In order to support the cytotoxicity data obtained using target cells expressing different cell surface densities of mesothelin (H226, HPAC, H292 and MeT-5A), cell binding of the MATCH molecules to all four cell lines was assessed by flow cytometry. MATCH4 PRO2000 and its aglycosylated version PRO2100 were tested in order to confirm that both molecules have similar binding properties. The scMATCH3 PRO1872 was included as well for comparison.

### Method

Cells were washed twice with 100 µl PBS and were incubated with five-fold serial dilutions of PRO1872, PRO2000 or PRO2100 in staining buffer (PBS, 2% BCS heat inactivated, 2 mM EDTA) ranging from 50'000 to 0.005 pM. Cells were washed twice with staining buffer and binding of the MATCH4 molecules was visualized by protein L-PE (2 µg/ml). Plates were incubated 30 min at RT on a nutating mixer, washed twice with staining buffer, centrifuged for 5 min at 200 g and re-suspended in a final volume of 50 µl of staining buffer. PE signal of 20'000 events per well was analyzed by flow cytometry using a Novocyte flow cytometer device and the data were analyzed using the NovoExpress software (ACEA Biosciences). Mean fluorescence intensity (MFI) values of MATCH molecules were corrected for non-specific binding by subtracting blank (zero concentration of antibody). ΔMFI data were analyzed with a four-parameter logistic curve fit using the GraphPad Prism Data Analysis Software (GraphPad Software), and the concentration of molecules of interest required to reach 50 % of target cell binding ($EC_{50}$) was calculated.

**Results**

Binding of PRO2000, PRO2100 and PRO1872 to the different cells lines was assessed by flow cytometry. The concentration at which half maximal binding ($EC_{50}$) was observed and the maximal binding values reached (MFI) are presented in Table 26 and the corresponding titration curves are presented in Figure 10. PRO2000 and PRO2100 show comparable binding data to all cell lines tested. In comparison to PRO1872, PRO2000 and PRO2100 show a 3-fold higher binding to high mesothelin expressing cancer cells H226, 1.5 to 2-fold better binding to intermediate mesothelin expressing cancer cells HPAC and 2-fold lower binding to low mesothelin expressing cancer cells H292. Those data correlate with the data obtained in the cytotoxicity assay. Maximal binding observed for each molecule tested confirm the ranking of the cell lines in terms of cell surface mesothelin expression.

**Table 21: Potencies for target cell killing and CD8+ T cell activation in presence of H226 and Met-5A cells**

| | Target cell killing | | | | CD8+ T cell activation | | | |
|---|---|---|---|---|---|---|---|---|
| Target cell line | H226 | | Met-5A | | H226 | | Met-5A | |
| PRO ID | $EC_{50}$ (pM) | max. killing (%) | $EC_{50}$ (pM) | max. killing (%) | $EC_{50}$ (pM) | max. act. (%) | $Ec_{50}$ (pM) | max. act. (%) |
| PRO2000 | 0.07 | 87.68 | 144.70 | 54.65 | NA | 95.8 | 544.20 | 51.0 |
| PRO1872 | 5.31 | 93.64 | 8.88 | 47.87 | 0.09 | 88.9 | 10.89 | 59.9 |

**Table 22: Potencies for target cell killing and CD8+ T cell activation in presence of H292 and HPAC cells**

| | Target cell killing | | | | CD8+ T cell activation | | | |
|---|---|---|---|---|---|---|---|---|
| Target cell line | H292 | | HPAC | | H292 | | HPAC | |
| PRO ID | $Ec_{50}$ (pM) | max. killing (%) | $EC_{50}$ (pM) | max. killing (%) | $EC_{50}$ (pM) | max. act. (%) | $EC_{50}$ (pM) | max. act. (%) |
| PRO2000 | 652.20 | 89.77 | 40.75 | 79.82 | 114.50 | 53.9 | 12.24 | 70.9 |
| PRO1872 | 91.03 | 91.97 | 30.26 | 83.48 | 34.06 | 61.7 | 47.18 | 76.3 |

**Table 23: Potencies for H226 target cell killing in absence or presence of soluble mesothelin**

| MSLN concentration | no mesothelin | | 50 ng/ml | | 500 ng/ml | |
|---|---|---|---|---|---|---|
| PRO ID | $EC_{50}$ (pM) | max. killing (%) | $EC_{50}$ (pM) | max. killing (%) | $EC_{50}$ (pM) | max. killing (%) |
| PRO2000 | 9.73 | 93.75 | 31.21 | 94.86 | 165.30 | 92.05 |
| PRO1872 | 43.89 | 95.65 | 636.50 | 95.99 | 4653.00 | 97.65 |

**Table 24: Potencies for CD8+ T cell activation in presence of H226 target cell in absence or presence of soluble mesothelin**

| MSLN concentration | no MSLN | | 50 ng/ml | | 500 ng/ml | |
|---|---|---|---|---|---|---|
| PRO ID | $EC_{50}$ (pM) | max. act.(%) | $Ec_{50}$ (pM) | max. act.(%) | $EC_{50}$ (pM) | max. act.(%) |
| PRO2000 | 1.986 | 84.73 | 7.849 | 83.03 | 42.43 | 86.86 |
| PRO1872 | 22.86 | 83.02 | 199.8 | 79.04 | 1115 | 81.39 |

**Table 25: Potencies for target cell killing in absence or presence of soluble mesothelin**

| Target cell line | H226 | | | | Met-5A | | | |
|---|---|---|---|---|---|---|---|---|
| sMSLNconcentration | no sMSLN | | 100 ng/ml | | no sMSLN | | 100 ng/ml | |
| PRO ID | $EC_{50}$ (pM) | max. killing (%) | $EC_{50}$ (pM) | max. killing (%) | $EC_{50}$ (pM) | max. killing (%) | $EC_{50}$ (pM) | max. killing (%) |
| PRO2000 | 0.70 | 49.44 | 3.45 | 58.49 | no lysis | | no lysis | |
| PRO2100 | 1.54 | 46.34 | 3.56 | 58.92 | no lysis | | no lysis | |
| PRO1872 | 13.38 | 54 | 129.80 | 60.66 | no lysis | | no lysis | |

**Table 26: Binding of PRO2000, RPO2100 and PRO1872 to H226, H292, Met-5A and HPAC cells.**

| | H226 | | H292 | | Met-5A | | HPAC | |
|---|---|---|---|---|---|---|---|---|
| PRO ID | $EC_{50}$ (pM) | max. binding (MFI) | $EC_{50}$ (pM) | max. binding (MFI) | $EC_{50}$ (pM) | max. binding (MFI) | $EC_{50}$ (pM) | max. binding (MFI) |
| PRO2000 | 658.6 | 362311 | 1027.0 | 6964 | 128.2 | 7949 | 1476.0 | 17478 |
| PRO2100 | 524.0 | 363091 | 1301.0 | 6253 | 62.8 | 7479 | 1011.0 | 18029 |
| PRO1872 | 1571.0 | 467461 | 708.6 | 12168 | 194.3 | 11190 | 2157.0 | 33329 |

**Example 8: In vivo tumor growth inhibition with PRO2000 (biMSLN$_{high\ KD}$xCD3xhSA):**

**Introduction**

An *in vivo*, mesothelin-expressing cell line xenograft experiment was performed at Charles River Laboratories in order to determine the ability of PRO2000 (biMSLN$_{high\ KD}$xCD3xhSA) to effectively control tumor growth relative to control animals.

**Methods**

*Animals*

Female NCG mice from Charles River Laboratories were bred and housed under conditions suitable for humanized mouse work. Animals were used between 8-12 weeks of age.

*Study design*

Animals in treatment groups (n = 5-6 per group) were subcutaneously co-implanted with 1 x $10^7$ H292 NSCLC tumor cells and 1 x $10^7$ PBMCs in the flank. After 5 days, animals were dosed intravenously with molecules of interest, with additional doses every 5 days until the end of the experiment. During the experiment, animals were monitored at regular intervals for tumor growth using caliper measurements and for weight loss. Animals were euthanized either when the mean tumor volume in the control group was 800 mm$^3$ or at 40 days, whichever came first. Animals were monitored and euthanized according to animal health and welfare regulations at Charles River Laboratories.

**Results**

We assessed the efficacy of PRO2000 (biMSLN$_{high\ KD}$xCD3xhSA) in promoting tumor growth inhibition using a PBMC/H292 co-implantation model, as described in the methods. H292 cells express moderate levels of MSLN and are established from non-small cell lung carcinoma. Multiple dose levels of PRO2000 were administered intravenously, as shown in Figure 11. As comparisons, we used palivizumab (anti-RSV antibody) as a control IgG treatment, as well as tumor cells engrafted in the absence of PBMCs (no treatment). We observed that control conditions resulted in tumor outgrowth (light gray lines, Figure 11A). Treatment with the PRO2000 (biMSLN.CD3) molecule resulted in tumor growth inhibition at 1 and 5 mg/kg relative to controls (black lines and dark gray lines in Figure 11A, respectively). We examined the significance of the treatments using two-way ANOVA, followed by Tukey's multiple comparisons test; day 40 data are shown in Figure 11B, with each point representing an individual animal. The two higher doses (1 mg/kg and 5 mg/kg) resulted in significantly lower tumor volumes relative to palivizumab-treated animals (ctrl) and untreated animals. The lowest dose (0.2 mg/kg) appeared to be suboptimal, as these comparisons were not significant as compared to palivizumab-treated animals. There appeared to be no adverse effects on overall animal health, as animal weights were relatively stable throughout the experiment (data not shown). Taken together, these data indicate that the multispecific antibody PRO2000 (biMSLN$_{high\ KD}$xCD3xhSA) has tumor growth inhibition activity in vivo and is a promising conceptual candidate for cancer immunotherapy.

**Claims**

1. A multispecific antibody comprising:

   a) two antibody-based binding domains, which specifically bind to mesothelin (MSLN-BDs); and
   b) at least one antibody-based binding domain, which specifically binds to CD3 (CD3-BD);

   wherein said multispecific antibody does not comprise an immunoglobulin Fc region polypeptide, and wherein each of said MSLN-BDs binds to mesothelin (MSLN) with a monovalent dissociation constant ($K_D$) in the range of from 0.5 to 20 nM, in particular in the range of from 0.6 to 10 nM when measured by SPR.

2. The multispecific antibody of claim 1, wherein said MSLN-BD comprises

   (ii) the HCDR1, HCDR2, and HCDR3 sequences of SEQ ID NOs: 1, 2 (or 10) and 3, respectively, and the LCDR1, LCDR2, and LCDR3 sequences of SEQ ID NOs: 4, 5 and 6, respectively; or the HCDR1, HCDR2, and HCDR3 sequences of SEQ ID NOs: 11, 12 and 13, respectively, and the LCDR1, LCDR2, and LCDR3 sequences of SEQ ID NOs: 14, 15 and 16, respectively; and
   (iv) VH3 or VH4 domain framework sequences FR1 to FR4; particularly VH3 domain framework sequences FR1 to FR4; and
   (v) a VL domain comprising a VL framework comprising Vκ frameworks FR1, FR2 and FR3, particularly Vκ1 or Vκ3 FR1 to FR3, particularly Vκ1 FR1 to FR3, and a framework FR4, which is selected from a Vκ FR4, and a Vλ FR4, particularly a Vλ FR4 comprising an amino acid sequence having at least 70, 80, or 90 percent identity to any of SEQ ID NO: 66 to SEQ ID NO: 73, more particularly Vλ FR4 selected from any of SEQ ID NO: 66 to SEQ ID NO: 73, particularly Vλ FR4 according to SEQ ID NO: 66 or 73.

3. The multispecific antibody of claim 1 or 2, wherein said MSLN-BD comprises

   a.1) HCDR1, HCDR2, and HCDR3 sequences of SEQ ID NOs: 1, 2 (or 10) and 3, respectively,
   b.1) LCDR1, LCDR2, and LCDR3 sequences of SEQ ID NOs: 4, 5 and 6, respectively,
   c.1) a VH sequence at least 80, 85, 90, 91, 92, 93, 94, 95, 96, 97, 98 or 99 percent identical to the amino acid sequence SEQ ID NO: 7, and
   d.1) a VL sequence at least 80, 85, 90, 91, 92, 93, 94, 95, 96, 97, 98 or 99 percent identical to the amino acid sequence SEQ ID NO: 9;
   or
   a.2) HCDR1, HCDR2, and HCDR3 sequences of SEQ ID NOs: 1, 2 (or 10) and 3, respectively,
   b.2) LCDR1, LCDR2, and LCDR3 sequences of SEQ ID NOs: 4, 5 and 6, respectively,
   c.2) a VH sequence at least 80, 85, 90, 91, 92, 93, 94, 95, 96, 97, 98 or 99 percent identical to the amino acid sequence SEQ ID NO: 8, and
   d.2) a VL sequence at least 80, 85, 90, 91, 92, 93, 94, 95, 96, 97, 98 or 99 percent identical to the amino acid sequence SEQ ID NO: 9;
   or
   a.3) HCDR1, HCDR2, and HCDR3 sequences of SEQ ID NOs: 11, 12 and 13, respectively,
   b.3) LCDR1, LCDR2, and LCDR3 sequences of SEQ ID NOs: 14, 15 and 16, respectively,
   c.3) a VH sequence at least 80, 85, 90, 91, 92, 93, 94, 95, 96, 97, 98 or 99 percent identical to the amino acid sequence SEQ ID NO: 17, and
   d.3) a VL sequence at least 80, 85, 90, 91, 92, 93, 94, 95, 96, 97, 98 or 99 percent identical to the amino acid sequence SEQ ID NO: 18.

4. The multispecific antibody of any one of claims 1 to 3, wherein said CD3-BD is binding to CD3ε.

5. The multispecific antibody of any one of the preceding claims, wherein said antibody comprises one CD3-BD, wherein said CD3-BD binds CD3ε with a monovalent $K_D$ of 0.5 to 50 nM, particularly of 1 to 40 nM, particularly of 2 to 35 nM, particularly of 3 to 30 nM, as measured by SPR.

6. The multispecific antibody of claim 4 or 5, wherein said CD3-BD comprises

   (i) HCDR1, HCDR2, and HCDR3 sequences of SEQ ID NOs: 27, 28 and 29, respectively in a human antibody VH framework, particularly a VH3 framework, and

(ii) LCDR1, LCDR2, and LCDR3 sequences of SEQ ID NOs: 30, 31 and 32, respectively in a human antibody VL framework, wherein the VL framework comprises Vκ frameworks FR1, FR2 and FR3, particularly Vκ1 frameworks, and a framework FR4, which is selected from a Vκ FR4, and a Vλ framework 4,

particularly, wherein said CD3-BD comprises

(i) a VH domain comprising the amino acid sequence of SEQ ID NO: 33, and
(ii) a VL domain comprising the amino acid sequence of SEQ ID NO: 34.

7. The multispecific antibody of any one of the preceding claims, wherein said antibody further comprises at least one human serum albumin binding domain (hSA-BD), particularly one hSA-BD, particularly wherein said hSA-BD comprises

(i) HCDR1, HCDR2, and HCDR3 sequences of SEQ ID NOs: 35, 36 and 37, respectively in a human antibody VH framework, particularly a VH3 framework, and
(ii) LCDR1, LCDR2, and LCDR3 sequences of SEQ ID NOs: 38, 39 and 40, respectively in a human antibody VL framework, wherein the VL framework comprises Vκ frameworks FR1, FR2 and FR3, particularly Vκ1 frameworks, and a framework FR4, which is selected from a Vκ FR4, particularly Vκ1 FR4, and a Vλ framework 4; or

(i) HCDR1, HCDR2, and HCDR3 sequences of SEQ ID NOs: 45, 46 and 47, respectively in a human antibody VH framework, particularly a VH3 framework, and
(ii) LCDR1, LCDR2, and LCDR3 sequences of SEQ ID NOs: 48, 49 and 50, respectively in a human antibody VL framework, wherein the VL framework comprises Vκ frameworks FR1, FR2 and FR3, particularly Vκ1 frameworks, and a framework FR4, which is selected from a Vκ FR4, particularly Vκ1 FR4, and a Vλ framework 4

particularly, wherein wherein said hSA-BD comprises

(i) a VH domain comprising the amino acid sequence of SEQ ID NO: 41 or 51, and
(ii) a VL domain comprising the amino acid sequence of SEQ ID NO: 42 or 52.

8. The multispecific antibody of any one of the preceding claims, wherein said antibody does not comprise CH1 and/or CL regions.

9. The multispecific antibody of any one of the preceding claims, wherein
said first single-chain protein comprises an amino acid sequence having at least 90, 95, 96, 97, 98 or 99 percent identity to the amino acid sequence of SEQ ID NO: 55, more particularly comprises the amino acid sequence of SEQ ID NO: 55, especially consists of the amino acid sequence SEQ ID NO: 55 and
said second single-chain protein comprises an amino acid sequence having at least 90, 95, 96, 97, 98 or 99 percent identity to the amino acid sequence of SEQ ID NO: 56, more particularly comprises the amino acid sequence of SEQ ID NO: 56, especially consists of the amino acid sequence SEQ ID NO: 56; or
said first single-chain protein comprises an amino acid sequence having at least 90, 95, 96, 97, 98 or 99 percent identity to the amino acid sequence of SEQ ID NO: 57, more particularly comprises the amino acid sequence of SEQ ID NO: 57, especially consists of the amino acid sequence SEQ ID NO: 57 and
said second single-chain protein comprises an amino acid sequence having at least 90, 95, 96, 97, 98 or 99 percent identity to the amino acid sequence of SEQ ID NO: 58, more particularly comprises the amino acid sequence of SEQ ID NO: 58, especially consists of the amino acid sequence SEQ ID NO: 58.

10. A nucleic acid sequence or two nucleic acid sequences encoding the multispecific antibody of any one of claims 1 to 9.

11. A vector or two vectors comprising the nucleic acid sequence or the two nucleic acid sequences of claim 10.

12. A host cell or host cells comprising the vector or the two vectors of claim 11.

13. A method for producing the multispecific antibody of any one of claims 1 to 9, comprising (i) providing the nucleic acid sequence or the two nucleic acid sequences of claim 10, or the vector or the two vectors of claim 11, expressing said nucleic acid sequence or nucleic acid sequences, or said vector or vectors, and collecting said multispecific antibody from the expression system, or (ii) providing a host cell or host cells according to claim 12, culturing said host cell or said host cells; and collecting said multispecific antibody from the cell culture.

14. A pharmaceutical composition comprising the multispecific antibody of any one of claims 1 to 9 and a pharmaceutically acceptable carrier.

15. The multispecific antibody of any one of claims 1 to 9 for use in the treatment of a disease, particularly a human disease, more particularly a human disease selected from cancer, particularly a cancer selected from mesothelioma, pancreatic cancer, and ovarian cancer, an inflammatory and an autoimmune disease,
particularly wherein said multispecific antibody is either
a single-chain protein comprising three or four binding domains, or
a hetero-dimeric protein comprising three or four binding domains.

## Figure 1:

## Figure 2:

## Figure 3:

## Figure 4:

**Figure 5**:

MATCH4

scMATCH3 (scDb-scFv)

⊓  Disulfide bond, VL-G141C/VH-G51C
⊂ ↗  Peptide linker
N  N-terminus
C  C-terminus
VLx/VHx  Variable light (VL) and heavy (VH) domain #

**Figure 6**:

**Figure 7**:

Figure 8:

**Figure 9**:

**Figure 10**:

**Figure 11**:

A

B

Europäisches
Patentamt

European
Patent Office

Office européen
des brevets

**EUROPEAN SEARCH REPORT**

Application Number

EP 20 17 7337

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RICHARD AUSTIN ET AL: "HPN536, a T cell-engaging, Mesothelin/CD3-specific TriTAC for the treatment of solid tumors IN VITRO PHARMACOLOGY BACKGROUND", AACR ANNUAL MEETING, 14 April 2018 (2018-04-14), XP55742797, * abstract; compound HPN536 * | 1-15 | INV. A61K39/395 C07K16/28 C07K16/30 |
| A | WO 2017/055391 A1 (F HOFFMANN-LA ROCHE AG [CH]; HOFFMANN-LA ROCHE INC [US]) 6 April 2017 (2017-04-06) * abstract; claims 1, 3, 6; figures 1 0 - Z; example 1 * * page 26, line 16 - page 27, line 4 * * page 84, line 6 - page 85, line 9 * | 1-15 | |
| A | US 2017/029502 A1 (RAUM TOBIAS [DE] ET AL) 2 February 2017 (2017-02-02) * abstract; claims 1-3, 14-18, 20-22; example 3; table 3 * * page 3, paragraph [0032] * * page 10, paragraph [0096] * * page 30, paragraph [0394] * | 1-15 | |
| T | ULRICH BRINKMANN ET AL: "The making of bispecific antibodies", MABS, vol. 9, no. 2, 10 January 2017 (2017-01-10), pages 182-212, XP055531122, US ISSN: 1942-0862, DOI: 10.1080/19420862.2016.1268307 * abstract; figures 1-4; tables 1-3 * * page 182 - page 183 * | | TECHNICAL FIELDS SEARCHED (IPC)  A61K C07K |

-/--

The present search report has been drawn up for all claims

| Place of search | Date of completion of the search | Examiner |
|---|---|---|
| The Hague | 20 January 2021 | Schulz, Regine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 17 7337

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2018/224443 A1 (NUMAB INNOVATION AG [CH]) 13 December 2018 (2018-12-13) * abstract; claims 1, 20 - 26; figures 4-9, 12-15; example 1; tables 2, 3 * * page 7, last paragraph - page 14, line 5 * | 1-15 | |
| A | TEA GUNDE ET AL: "Abstract 1532: A novel, monovalent tri-specific antibody-based molecule that simultaneously modulates PD-L1 and 4-1BB exhibits potent anti-tumoral activity in vivo", IMMUNOLOGY, 1 July 2019 (2019-07-01), pages 1532-1532, XP055766584, GB ISSN: 0019-2805, DOI: 10.1158/1538-7445.AM2019-1532 * the whole document * | 1-15 | |
| A | US 2016/340440 A1 (FANSLOW III WILLIAM CHRISTIAN [US] ET AL) 24 November 2016 (2016-11-24) * abstract; claims 1, 21, 35-42; figures 1-30 * | 1-15 | TECHNICAL FIELDS SEARCHED (IPC) |

The present search report has been drawn up for all claims

| Place of search | Date of completion of the search | Examiner |
|---|---|---|
| The Hague | 20 January 2021 | Schulz, Regine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 20 17 7337

This annex lists the patent family members relating to the patent documents cited in the above-mentioned European search report.
The members are as contained in the European Patent Office EDP file on
The European Patent Office is in no way liable for these particulars which are merely given for the purpose of information.

20-01-2021

| Patent document cited in search report | | | Publication date | Patent family member(s) | | Publication date |
|---|---|---|---|---|---|---|
| WO 2017055391 | A1 | | 06-04-2017 | AR | 106365 A1 | 10-01-2018 |
| | | | | CN | 108026179 A | 11-05-2018 |
| | | | | EP | 3356417 A1 | 08-08-2018 |
| | | | | HK | 1254967 A1 | 02-08-2019 |
| | | | | JP | 2018536389 A | 13-12-2018 |
| | | | | TW | 201726721 A | 01-08-2017 |
| | | | | US | 2017096485 A1 | 06-04-2017 |
| | | | | WO | 2017055391 A1 | 06-04-2017 |
| US 2017029502 | A1 | | 02-02-2017 | AU | 2016302575 A1 | 07-12-2017 |
| | | | | BR | 112018001638 A2 | 18-09-2018 |
| | | | | CA | 2991672 A1 | 09-02-2017 |
| | | | | CL | 2018000268 A1 | 05-10-2018 |
| | | | | CN | 109476736 A | 15-03-2019 |
| | | | | CO | 2018000885 A2 | 19-04-2018 |
| | | | | EA | 201890383 A1 | 29-06-2018 |
| | | | | EP | 3328893 A1 | 06-06-2018 |
| | | | | HK | 1248250 A1 | 12-10-2018 |
| | | | | JP | 2018529317 A | 11-10-2018 |
| | | | | KR | 20180037950 A | 13-04-2018 |
| | | | | PE | 20180798 A1 | 09-05-2018 |
| | | | | PH | 12018500107 A1 | 23-07-2018 |
| | | | | SG | 10202000828T A | 30-03-2020 |
| | | | | TN | 2017000552 A1 | 12-04-2019 |
| | | | | TW | 201708256 A | 01-03-2017 |
| | | | | US | 2017029502 A1 | 02-02-2017 |
| | | | | WO | 2017021356 A1 | 09-02-2017 |
| WO 2018224443 | A1 | | 13-12-2018 | AU | 2018280683 A1 | 17-10-2019 |
| | | | | CA | 3065868 A1 | 13-12-2018 |
| | | | | CN | 110719918 A | 21-01-2020 |
| | | | | EP | 3635014 A1 | 15-04-2020 |
| | | | | JP | 2020522267 A | 30-07-2020 |
| | | | | KR | 20200013230 A | 06-02-2020 |
| | | | | SG | 11201911527Q A | 30-01-2020 |
| | | | | US | 2020181263 A1 | 11-06-2020 |
| | | | | WO | 2018224443 A1 | 13-12-2018 |
| US 2016340440 | A1 | | 24-11-2016 | AR | 091605 A1 | 18-02-2015 |
| | | | | TW | 201420600 A | 01-06-2014 |
| | | | | US | 2014004121 A1 | 02-01-2014 |
| | | | | US | 2016340440 A1 | 24-11-2016 |
| | | | | US | 2019055316 A1 | 21-02-2019 |
| | | | | UY | 34885 A | 31-12-2013 |
| | | | | WO | 2014004549 A2 | 03-01-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6075181 A
- US 6150584 A
- US 5766886 A
- EP 0486525 A
- US 6267964 B
- WO 2004001064 A
- WO 2002076489 A
- WO 200145746 A
- WO 2004003019 A
- WO 2008096158 A
- WO 2005118642 A
- WO 20060591056 A
- WO 2011006915 A
- WO 2009040562 A
- WO 2010035012 A
- WO 2011086091 A
- WO 2019057787 A

- WO 20160202457 A
- EP 404097 A
- WO 9301161 A
- WO 9957150 A
- US 5260203 A
- US 5455030 A
- US 4881175 A
- US 5132405 A
- US 5091513 A
- US 5476786 A
- US 5013653 A
- US 5258498 A
- US 5482858 A
- US 4458066 A
- EP 2018064630 W
- EP 20164913
- EP 19206959

**Non-patent literature cited in the description**

- **WARD et al.** *Nature,* 1989, vol. 341, 544-546
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC, M.-P.** *The Immunologist,* 1999, vol. 7, 132-136
- **LEFRANC, M.-P. et al.** *Dev. Comp. Immunol.,* 2003, vol. 27, 55-77
- **HONEGGER ; PLUCKTHUN.** *J. Mol. Biol.,* 2001, vol. 309, 657-670
- **HOOGENBOOM ; WINTER.** *J. Mol. Biol,* 1991, vol. 227, 381
- **MARKS et al.** *J. Mol. Biol,* 1991, vol. 222, 581
- **COLE et al.** Monoclonal Antibodies and Cancer Therapy. Alan R. Liss, 1985, 77
- **BOEMER et al.** *J. Immunol,* 1991, vol. 147 (I), 86-95
- **VAN DIJK ; VAN DE WINKEL.** *Curr. Opin. Pharmacol,* 2001, vol. 5, 368-74
- **LI et al.** *Proc. Natl. Acad. Sci. USA,* 2006, vol. 103, 3557-3562
- **MORRISON et al.** *Proc. Natl. Acad. Sci. USA,* 1984, vol. 81, 6851-6855
- **MORRISON ; OI.** *Adv. Immunol.,* 1988, vol. 44, 65-92

- **VERHOEYEN et al.** *Science,* 1988, vol. 239, 1534-1536
- **PADLAN.** *Molec. Immun.,* 1991, vol. 28, 489-498
- **PADLAN.** *Molec. Immun.,* 1994, vol. 31, 169-217
- **MEDASAN et al.** *J. Immunol.,* 1997, vol. 158, 2211-2217
- **SUGIO.** *Protein Eng,* 1999, vol. 12, 439-446
- **FANALI.** *Molecular Aspects of Medicine,* 2012, vol. 33, 209-290
- **SMITH et al.** *Bioconjugate Chem.,* 2001, vol. 12, 750-756
- **HOLT et al.** *Protein Engineering, Design & Selection,* vol. 21 (5), 283-288
- **KNAPPIK et al.** *J. Mol. Biol.,* 2000, vol. 296, 57-86
- **EGAN T. et al.** *MABS,* 2017, vol. 9, 68-84
- *MAbs.,* February 2017, vol. 9 (2), 182-212
- **HUDSON et al.** *Nat. Med.,* 2003, vol. 9, 129-134
- **HOLLINGER et al.** *Proc. Natl. Acad. Sci. USA,* 1993, vol. 90, 6444-6448
- **FISCHER, N. ; LEGER, O.** *Pathobiology,* 2007, vol. 74, 3-14
- **HORNIG, N. ; FARBER-SCHWARZ, A.** *Methods Mol. Biol.,* 2012, vol. 907, 713-727

- **LABRIJN et al.** *Proc. Natl. Acad. Sci. USA,* 2013, vol. 110, 5145-5150
- **DE KRUIF et al.** *Biotechnol. Bioeng.,* 2010, vol. 106, 741-750
- **ZHU et al.** *Cancer Lett.,* 1994, vol. 86, 127-134
- **SURESH et al.** *Methods Enzymol.,* 1986, vol. 121, 210-228
- **YOKOYAMA et al.** Curr. Protoc. Immunol. 2006
- **CHAMES ; BATY.** *FEMS Microbiol. Letters,* 2000, vol. 189, 1-8
- **KARPOVSKY et al.** *J. Exp. Med.,* 1984, vol. 160, 1686
- **LIU, M A et al.** *Proc. Natl. Acad. Sci. USA,* 1985, vol. 82, 8648
- **PAULUS.** *Behring Ins. Mitt.,* 1985, 118-132
- **BRENNAN et al.** *Science,* 1985, vol. 229, 81-83
- **GLENNIE et al.** *J. Immunol.,* 1987, vol. 139, 2367-2375
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **NARANG et al.** *Meth. Enzymol.,* 1979, vol. 68, 90
- **BROWN et al.** *Meth. Enzymol.,* 1979, vol. 68, 109
- **BEAUCAGE et al.** *Tetra. Lett.,* 1981, vol. 22, 1859
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 967
- **ECKERT et al.** *PCR Methods and Applications,* 1991, vol. 1, 17
- **HARRINGTON et al.** *Nat Genet.,* 1997, vol. 15, 345
- **SMITH.** *Annu. Rev. Microbiol.,* 1995, vol. 49, 807
- **ROSENFELD et al.** *Cell,* 1992, vol. 68, 143
- **SCHARF et al.** *Results Probl. Cell Differ.,* 1994, vol. 20, 125
- **BITTNER et al.** *Meth. Enzymol.,* 1987, vol. 153, 516
- **WINNACKER.** FROM GENES TO CLONES. VCH Publishers, 1987
- **QUEEN et al.** *Immunol. Rev.,* 1986, vol. 89, 49-68
- **ELLIOT ; O'HARE.** *Cell,* 1997, vol. 88, 223
- Remington: The Science and Practice of Pharmacy. Mack Publishing Co, 2000
- Sustained and Controlled Release Drug Delivery Systems. Marcel Dekker, Inc, 1978
- **EGAN TJ et al.** Novel multi-specific heterodimeric antibody format allowing modular assembly of variable domain fragments. *MABS,* 2017, vol. 9, 68-84

SEQUENCE LISTING

<110> Numab Therapeutics AG

<120> Multispecific Antibody

<130> 116736P877EP

<160> 73

<170> PatentIn version 3.5

<210> 1
<211> 10
<212> PRT
<213> Artificial sequence

<220>
<223> artificial antibody-based sequence

<400> 1

Gly Phe Ser Leu Ser Ser Tyr Ala Met Gly
1               5                   10

<210> 2
<211> 16
<212> PRT
<213> Artificial sequence

<220>
<223> artificial antibody-based sequence

<400> 2

Tyr Ile Ser Thr Ile Asn Asn Thr Tyr Tyr Ala Ser Trp Ala Lys Gly
1               5                   10                  15

<210> 3
<211> 14
<212> PRT
<213> Artificial sequence

<220>
<223> artificial antibody-based sequence

<400> 3

Arg Glu Ile Arg Ser Gly Trp Val Asp Tyr Gly Phe Ser Ile
1               5                   10

<210> 4
<211> 11
<212> PRT
<213> Artificial sequence

<220>
<223> artificial antibody-based sequence

<400> 4

Gln Ala Ser Gln Asn Ile Tyr Ser Asn Leu Ala
1                   5                   10


<210>   5
<211>   7
<212>   PRT
<213>   Artificial sequence

<220>
<223>   artificial antibody-based sequence

<400>   5

Asp Ala Ser Asp Leu Ala Ser
1                   5


<210>   6
<211>   12
<212>   PRT
<213>   Artificial sequence

<220>
<223>   artificial antibody-based sequence

<400>   6

Gln Gln Val Arg Ser Ser Ser Asp Ile Asp Asn Pro
1                   5                   10


<210>   7
<211>   121
<212>   PRT
<213>   Artificial sequence

<220>
<223>   artificial antibody-based sequence

<400>   7

Gln Val Gln Leu Val Glu Ser Gly Gly Gly Leu Val Gln Pro Gly Gly
1                   5                   10                  15


Ser Leu Arg Leu Ser Cys Ala Ala Ser Gly Phe Ser Leu Ser Ser Tyr
            20                  25                  30


Ala Met Gly Trp Val Arg Gln Ala Pro Gly Lys Gly Leu Glu Trp Ile
            35                  40                  45


Gly Tyr Ile Ser Thr Ile Asn Asn Thr Tyr Tyr Ala Ser Trp Ala Lys
            50                  55                  60


Gly Arg Phe Thr Ile Ser Arg Asp Asn Ser Lys Asn Thr Val Tyr Leu
65                  70                  75                  80

```
Gln Met Asn Ser Leu Arg Ala Glu Asp Thr Ala Val Tyr Tyr Cys Ala
                85                  90                  95


Arg Glu Ile Arg Ser Gly Trp Val Asp Tyr Gly Phe Ser Ile Trp Gly
            100                 105                 110


Gln Gly Thr Leu Val Thr Val Ser Ser
        115                 120


<210>   8
<211>   121
<212>   PRT
<213>   Artificial sequence

<220>
<223>   artificial antibody-based sequence

<400>   8

Gln Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Leu Ser Ser Tyr
            20                  25                  30


Ala Met Gly Trp Val Arg Gln Ala Pro Gly Lys Gly Leu Glu Trp Ile
            35                  40                  45


Gly Tyr Ile Ser Thr Ile Ala Asn Thr Tyr Tyr Ala Ser Trp Ala Lys
        50                  55                  60


Gly Arg Phe Thr Ile Ser Arg Asp Asn Ser Lys Asn Thr Val Tyr Leu
65                  70                  75                  80


Gln Met Asn Ser Leu Arg Ala Glu Asp Thr Ala Val Tyr Tyr Cys Ala
                85                  90                  95


Arg Glu Ile Arg Ser Gly Trp Val Asp Tyr Gly Phe Ser Ile Trp Gly
            100                 105                 110


Gln Gly Thr Leu Val Thr Val Ser Ser
        115                 120


<210>   9
<211>   111
<212>   PRT
<213>   Artificial sequence

<220>
<223>   artificial antibody-based sequence

<400>   9
```

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asn Ile Tyr Ser Asn
            20                  25                  30

Leu Ala Trp Tyr Gln Gln Lys Pro Gly Lys Ala Pro Lys Leu Leu Ile
            35                  40                  45

Tyr Asp Ala Ser Asp Leu Ala Ser Gly Val Pro Ser Arg Phe Ser Gly
        50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Arg Ser Ser Ser Asp
                85                  90                  95

Ile Asp Asn Pro Phe Gly Thr Gly Thr Lys Val Thr Val Leu Gly
            100                 105                 110

<210>   10
<211>   16
<212>   PRT
<213>   Artificial sequence

<220>
<223>   artificial antibody-based sequence

<400>   10

Tyr Ile Ser Thr Ile Ala Asn Thr Tyr Tyr Ala Ser Trp Ala Lys Gly
1               5                   10                  15

<210>   11
<211>   10
<212>   PRT
<213>   Artificial sequence

<220>
<223>   artificial antibody-based sequence

<400>   11

Gly Phe Ser Leu Ser Ser Tyr Ala Met Gly
1               5                   10

<210>   12
<211>   16
<212>   PRT
<213>   Artificial sequence

<220>

52

<223> artificial antibody-based sequence

<400> 12

Tyr Ile Ser Lys Ile Gly Thr Thr Tyr Tyr Ala Ser Trp Ala Lys Gly
1               5                   10                  15


<210> 13
<211> 15
<212> PRT
<213> Artificial sequence

<220>
<223> artificial antibody-based sequence

<400> 13

Arg Gly Ser Ser Ser Gly Gly Tyr Leu Asp Asp Gly Phe Asp Pro
1               5                   10                  15


<210> 14
<211> 11
<212> PRT
<213> Artificial sequence

<220>
<223> artificial antibody-based sequence

<400> 14

Gln Ala Ser Gln Ser Ile Ser Asn Tyr Leu Ala
1               5                   10


<210> 15
<211> 7
<212> PRT
<213> Artificial sequence

<220>
<223> artificial antibody-based sequence

<400> 15

Asp Ala Ser Asp Leu Ala Ser
1               5


<210> 16
<211> 12
<212> PRT
<213> Artificial sequence

<220>
<223> artificial antibody-based sequence

<400> 16

Gln Gln Val Tyr Asp Ser Asn Asn Val Glu Asn Val
1               5                   10

<210> 17
<211> 122
<212> PRT
<213> Artificial sequence

<220>
<223> artificial antibody-based sequence

<400> 17

Gln Ser Gln Leu Val Glu Ser Gly Gly Gly Leu Val Gln Pro Gly Gly
1               5                   10                  15

Ser Leu Arg Leu Ser Cys Ala Val Ser Gly Phe Ser Leu Ser Ser Tyr
            20                  25                  30

Ala Met Gly Trp Val Arg Gln Ala Pro Gly Lys Gly Leu Glu Tyr Ile
        35                  40                  45

Gly Tyr Ile Ser Lys Ile Gly Thr Thr Tyr Tyr Ala Ser Trp Ala Lys
    50                  55                  60

Gly Arg Phe Thr Ile Ser Lys Asp Asn Ser Lys Asn Thr Val Tyr Leu
65                  70                  75                  80

Gln Met Asn Ser Leu Arg Ala Glu Asp Thr Ala Val Tyr Phe Cys Ala
                85                  90                  95

Arg Gly Ser Ser Ser Gly Gly Tyr Leu Asp Asp Gly Phe Asp Pro Trp
            100                 105                 110

Gly Gln Gly Thr Leu Val Thr Val Ser Ser
        115                 120

<210> 18
<211> 126
<212> PRT
<213> Artificial sequence

<220>
<223> artificial antibody-based sequence

<400> 18

Ala Leu Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Ser Asn Tyr
            20                  25                  30

Leu Ala Trp Tyr Gln Gln Lys Pro Gly Lys Pro Pro Lys Phe Leu Ile
        35                  40                  45

54

```
Tyr Asp Ala Ser Asp Leu Ala Ser Gly Val Ser Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Tyr Asp Ser Asn Asn
                85                  90                  95

Val Glu Asn Val Phe Gly Thr Gly Thr Lys Val Thr Val Leu Gly Arg
                100                 105                 110

Gly Ser Ser Ser Gly Gly Tyr Leu Asp Asp Gly Phe Asp Pro
        115                 120                 125


<210>  19
<211>  11
<212>  PRT
<213>  Artificial sequence

<220>
<223>  artificial antibody-based sequence

<400>  19

Gly Ile Ser Val Ser Asn Asp Tyr Tyr Met Cys
1               5                   10


<210>  20
<211>  17
<212>  PRT
<213>  Artificial sequence

<220>
<223>  artificial antibody-based sequence

<400>  20

Cys Ile Ser Thr Tyr Ile Gly Asn Thr His Tyr Ala Ser Trp Ala Lys
1               5                   10                  15

Gly


<210>  21
<211>  14
<212>  PRT
<213>  Artificial sequence

<220>
<223>  artificial antibody-based sequence

<400>  21
```

```
Lys Asn Ala Gly Tyr Pro Gly Tyr Arg Tyr Ala Ile Asp Leu
1               5               10
```

```
<210>   22
<211>   11
<212>   PRT
<213>   Artificial sequence

<220>
<223>   artificial antibody-based sequence

<400>   22
```

```
Gln Ala Ser Glu Ser Ile Gly Asn Tyr Leu Ala
1               5               10
```

```
<210>   23
<211>   7
<212>   PRT
<213>   Artificial sequence

<220>
<223>   artificial antibody-based sequence

<400>   23
```

```
Ser Ala Ser Thr Leu Ala Ser
1               5
```

```
<210>   24
<211>   10
<212>   PRT
<213>   Artificial sequence

<220>
<223>   artificial antibody-based sequence

<400>   24
```

```
Gln Ser Thr Asp Tyr Gly Asp Ser Tyr Ile
1               5               10
```

```
<210>   25
<211>   123
<212>   PRT
<213>   Artificial sequence

<220>
<223>   artificial antibody-based sequence

<400>   25
```

```
Glu Val Gln Leu Val Glu Ser Gly Gly Gly Leu Val Gln Pro Gly Gly
1               5               10              15
```

```
Ser Leu Arg Leu Ser Cys Ala Ala Ser Gly Ile Ser Val Ser Asn Asp
            20              25              30
```

Tyr Tyr Met Cys Trp Val Arg Gln Ala Pro Gly Lys Gly Leu Glu Trp
        35                  40                  45

Ile Gly Cys Ile Ser Thr Tyr Ile Gly Asn Thr His Tyr Ala Ser Trp
        50                  55                  60

Ala Lys Gly Arg Phe Thr Ile Ser Arg Asp Asn Ser Lys Asn Thr Val
65                  70                  75                  80

Tyr Leu Gln Met Asn Ser Leu Arg Ala Glu Asp Thr Ala Val Tyr Tyr
            85                  90                  95

Cys Ala Lys Asn Ala Gly Tyr Pro Gly Tyr Arg Tyr Ala Ile Asp Leu
            100                 105                 110

Trp Gly Gln Gly Thr Leu Val Thr Val Ser Ser
        115                 120

<210> 26
<211> 109
<212> PRT
<213> Artificial sequence

<220>
<223> artificial antibody-based sequence

<400> 26

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Ser Ile Gly Asn Tyr
            20                  25                  30

Leu Ala Trp Tyr Gln Gln Lys Pro Gly Lys Ala Pro Lys Leu Leu Ile
        35                  40                  45

Tyr Ser Ala Ser Thr Leu Ala Ser Gly Val Pro Ser Arg Phe Ser Gly
        50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Ser Thr Asp Tyr Gly Asp Ser
            85                  90                  95

Tyr Ile Phe Gly Thr Gly Thr Lys Val Thr Val Leu Gly
            100                 105

<210> 27
<211> 10
<212> PRT
<213> Artificial sequence

<220>
<223> artificial antibody-based sequence

<400> 27

Gly Phe Ser Leu Ser Ser Tyr Asp Met Ser
1               5                   10


<210> 28
<211> 16
<212> PRT
<213> Artificial sequence

<220>
<223> artificial antibody-based sequence

<400> 28

Ala Ser Tyr Ala Ser Gly Pro Thr Tyr Tyr Ala Ser Trp Ala Lys Gly
1               5                   10                  15


<210> 29
<211> 12
<212> PRT
<213> Artificial sequence

<220>
<223> artificial antibody-based sequence

<400> 29

Arg Gly Gly Trp Thr Gly Thr Ser His Ser Asn Ile
1               5                   10


<210> 30
<211> 13
<212> PRT
<213> Artificial sequence

<220>
<223> artificial antibody-based sequence

<400> 30

Gln Ser Ser Gln Ser Val Phe Ser Asn Asn Tyr Leu Ala
1               5                   10


<210> 31
<211> 7
<212> PRT
<213> Artificial sequence

<220>

<223> artificial antibody-based sequence

<400> 31

Ser Ala Ser Thr Leu Ala Ser
1               5


<210> 32
<211> 13
<212> PRT
<213> Artificial sequence

<220>
<223> artificial antibody-based sequence

<400> 32

Leu Gly Ser Tyr Ala Cys Ser Ser Ala Asp Cys Tyr Val
1               5                   10


<210> 33
<211> 119
<212> PRT
<213> Artificial sequence

<220>
<223> artificial antibody-based sequence

<400> 33

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Leu Ser Ser Tyr
            20                  25                  30


Asp Met Ser Trp Val Arg Gln Ala Pro Gly Lys Gly Leu Ala Trp Ile
            35                  40                  45


Gly Ala Ser Tyr Ala Ser Gly Pro Thr Tyr Tyr Ala Ser Trp Ala Lys
    50                  55                  60


Gly Arg Phe Thr Ile Ser Arg Asp Asn Ser Lys Asn Thr Val Tyr Leu
65                  70                  75                  80


Gln Met Asn Ser Leu Arg Ala Glu Asp Thr Ala Thr Tyr Phe Cys Ala
                85                  90                  95


Arg Gly Gly Trp Thr Gly Thr Ser His Ser Asn Ile Trp Gly Gln Gly
            100                 105                 110


Thr Leu Val Thr Val Ser Ser
            115

```
<210>   34
<211>   114
<212>   PRT
<213>   Artificial sequence

<220>
<223>   artificial antibody-based sequence

<400>   34

Asp Ile Gln Met Thr Gln Ser Pro Ser Ser Leu Ser Ala Ser Val Gly
1               5                   10                  15


Asp Arg Val Thr Ile Thr Cys Gln Ser Ser Gln Ser Val Phe Ser Asn
            20                  25                  30


Asn Tyr Leu Ala Trp Phe Gln Gln Lys Pro Gly Gln Ser Pro Lys Arg
        35                  40                  45


Leu Ile Tyr Ser Ala Ser Thr Leu Ala Ser Gly Val Pro Ser Arg Phe
        50                  55                  60


Ser Gly Ser Gly Ser Gly Thr Asp Phe Thr Leu Thr Ile Ser Ser Leu
65                  70                  75                  80


Gln Pro Glu Asp Phe Ala Thr Tyr Tyr Cys Leu Gly Ser Tyr Ala Cys
                85                  90                  95


Ser Ser Ala Asp Cys Tyr Val Phe Gly Thr Gly Thr Lys Val Thr Val
            100                 105                 110


Leu Gly


<210>   35
<211>   10
<212>   PRT
<213>   Artificial sequence

<220>
<223>   artificial antibody-based sequence

<400>   35

Gly Phe Ser Leu Ser Ser Asn Ala Met Gly
1               5                   10


<210>   36
<211>   16
<212>   PRT
<213>   Artificial sequence

<220>
```

<223>  artificial antibody-based sequence

<400>  36

Ile Ile Ser Val Gly Gly Phe Thr Tyr Tyr Ala Ser Trp Ala Lys Gly
1               5                   10                  15


<210>  37
<211>  14
<212>  PRT
<213>  Artificial sequence

<220>
<223>  artificial antibody-based sequence

<400>  37

Arg Asp Arg His Gly Gly Asp Ser Ser Gly Ala Phe Tyr Leu
1               5                   10


<210>  38
<211>  13
<212>  PRT
<213>  Artificial sequence

<220>
<223>  artificial antibody-based sequence

<400>  38

Gln Ser Ser Glu Ser Val Tyr Ser Asn Asn Gln Leu Ser
1               5                   10


<210>  39
<211>  7
<212>  PRT
<213>  Artificial sequence

<220>
<223>  artificial antibody-based sequence

<400>  39

Asp Ala Ser Asp Leu Ala Ser
1               5


<210>  40
<211>  11
<212>  PRT
<213>  Artificial sequence

<220>
<223>  artificial antibody-based sequence

<400>  40

Ala Gly Gly Phe Ser Ser Ser Ser Asp Thr Ala
1               5                   10

```
<210>   41
<211>   121
<212>   PRT
<213>   Artificial sequence

<220>
<223>   artificial antibody-based sequence

<400>   41

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Leu Ser Ser Asn
            20                  25                  30


Ala Met Gly Trp Val Arg Gln Ala Pro Gly Lys Gly Leu Glu Tyr Ile
            35                  40                  45


Gly Ile Ile Ser Val Gly Gly Phe Thr Tyr Tyr Ala Ser Trp Ala Lys
        50                  55                  60


Gly Arg Phe Thr Ile Ser Arg Asp Asn Ser Lys Asn Thr Val Tyr Leu
65                  70                  75                  80


Gln Met Asn Ser Leu Arg Ala Glu Asp Thr Ala Thr Tyr Phe Cys Ala
                85                  90                  95


Arg Asp Arg His Gly Gly Asp Ser Ser Gly Ala Phe Tyr Leu Trp Gly
            100                 105                 110


Gln Gly Thr Leu Val Thr Val Ser Ser
            115                 120


<210>   42
<211>   112
<212>   PRT
<213>   Artificial sequence

<220>
<223>   artificial antibody-based sequence

<400>   42

Asp Ile Gln Met Thr Gln Ser Pro Ser Ser Leu Ser Ala Ser Val Gly
1               5                   10                  15


Asp Arg Val Thr Ile Thr Cys Gln Ser Ser Glu Ser Val Tyr Ser Asn
            20                  25                  30


Asn Gln Leu Ser Trp Tyr Gln Gln Lys Pro Gly Gln Pro Pro Lys Leu
            35                  40                  45
```

```
Leu Ile Tyr Asp Ala Ser Asp Leu Ala Ser Gly Val Pro Ser Arg Phe
    50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Ala Gly Gly Phe Ser Ser
                85                  90                  95

Ser Ser Asp Thr Ala Phe Gly Gly Gly Thr Lys Leu Thr Val Leu Gly
                100                 105                 110


<210>   43
<211>   121
<212>   PRT
<213>   Artificial sequence

<220>
<223>   artificial antibody-based sequence

<400>   43

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Leu Ser Ser Asn
            20                  25                  30

Ala Met Gly Trp Val Arg Gln Ala Pro Gly Lys Cys Leu Glu Tyr Ile
            35                  40                  45

Gly Ile Ile Ser Val Gly Gly Phe Thr Tyr Tyr Ala Ser Trp Ala Lys
    50                  55                  60

Gly Arg Phe Thr Ile Ser Arg Asp Asn Ser Lys Asn Thr Val Tyr Leu
65                  70                  75                  80

Gln Met Asn Ser Leu Arg Ala Glu Asp Thr Ala Thr Tyr Phe Cys Ala
                85                  90                  95

Arg Asp Arg His Gly Gly Asp Ser Ser Gly Ala Phe Tyr Leu Trp Gly
                100                 105                 110

Gln Gly Thr Leu Val Thr Val Ser Ser
            115                 120


<210>   44
<211>   112
<212>   PRT
```

<213> Artificial sequence

<220>
<223> artificial antibody-based sequence

<400> 44

Asp Ile Gln Met Thr Gln Ser Pro Ser Ser Leu Ser Ala Ser Val Gly
1               5                   10                  15

Asp Arg Val Thr Ile Thr Cys Gln Ser Ser Glu Ser Val Tyr Ser Asn
            20                  25                  30

Asn Gln Leu Ser Trp Tyr Gln Gln Lys Pro Gly Gln Pro Pro Lys Leu
        35                  40                  45

Leu Ile Tyr Asp Ala Ser Asp Leu Ala Ser Gly Val Pro Ser Arg Phe
        50                  55                  60

Ser Gly Ser Gly Ser Gly Thr Asp Phe Thr Leu Thr Ile Ser Ser Leu
65                  70                  75                  80

Gln Pro Glu Asp Phe Ala Thr Tyr Tyr Cys Ala Gly Gly Phe Ser Ser
                85                  90                  95

Ser Ser Asp Thr Ala Phe Gly Cys Gly Thr Lys Leu Thr Val Leu Gly
            100                 105                 110

<210> 45
<211> 11
<212> PRT
<213> Artificial sequence

<220>
<223> artificial antibody-based sequence

<400> 45

Gly Phe Ser Phe Ser Ser Ser Tyr Trp Ile Cys
1               5                   10

<210> 46
<211> 17
<212> PRT
<213> Artificial sequence

<220>
<223> artificial antibody-based sequence

<400> 46

Cys Val Phe Thr Gly Asp Gly Thr Thr Tyr Tyr Ala Ser Trp Ala Lys
1               5                   10                  15

Gly


```
<210>  47
<211>  12
<212>  PRT
<213>  Artificial sequence

<220>
<223>  artificial antibody-based sequence

<400>  47

Arg Pro Val Ser Val Tyr Tyr Tyr Gly Met Asp Leu
1               5                   10


<210>  48
<211>  11
<212>  PRT
<213>  Artificial sequence

<220>
<223>  artificial antibody-based sequence

<400>  48

Gln Ala Ser Gln Ile Ile Ser Ser Arg Ser Ala
1               5                   10


<210>  49
<211>  7
<212>  PRT
<213>  Artificial sequence

<220>
<223>  artificial antibody-based sequence

<400>  49

Gln Ala Ser Lys Leu Ala Ser
1               5


<210>  50
<211>  11
<212>  PRT
<213>  Artificial sequence

<220>
<223>  artificial antibody-based sequence

<400>  50

Gln Cys Thr Tyr Ile Asp Ser Asn Phe Gly Ala
1               5                   10


<210>  51
<211>  121
<212>  PRT
```

<210>   52
<211>   110
<212>   PRT
<213>   Artificial sequence

<220>
<223>   artificial antibody-based sequence

<400>   51

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Phe Ser Ser Ser
            20                  25                  30

Tyr Trp Ile Cys Trp Val Arg Gln Ala Pro Gly Lys Gly Leu Glu Trp
        35                  40                  45

Val Gly Cys Val Phe Thr Gly Asp Gly Thr Thr Tyr Tyr Ala Ser Trp
    50                  55                  60

Ala Lys Gly Arg Phe Thr Ile Ser Arg Asp Asn Ser Lys Asn Thr Val
65                  70                  75                  80

Tyr Leu Gln Met Asn Ser Leu Arg Ala Glu Asp Thr Ala Thr Tyr Phe
                85                  90                  95

Cys Ala Arg Pro Val Ser Val Tyr Tyr Tyr Gly Met Asp Leu Trp Gly
            100                 105                 110

Gln Gly Thr Leu Val Thr Val Ser Ser
        115                 120

<210>   52
<211>   110
<212>   PRT
<213>   Artificial sequence

<220>
<223>   artificial antibody-based sequence

<400>   52

Asp Val Val Met Thr Gln Ser Pro Ser Ser Leu Ser Ala Ser Val Gly
1               5                   10                  15

Asp Arg Val Thr Ile Thr Cys Gln Ala Ser Gln Ile Ile Ser Ser Arg
            20                  25                  30

Ser Ala Trp Tyr Gln Gln Lys Pro Gly Gln Pro Pro Lys Leu Leu Ile
        35                  40                  45

Tyr Gln Ala Ser Lys Leu Ala Ser Gly Val Pro Ser Arg Phe Ser Gly
    50                  55                  60

```
Ser Gly Ser Gly Thr Asp Phe Thr Leu Thr Ile Ser Ser Leu Gln Pro
65                  70              75                  80


Glu Asp Phe Ala Thr Tyr Tyr Cys Gln Cys Thr Tyr Ile Asp Ser Asn
                85              90                  95


Phe Gly Ala Phe Gly Gly Gly Thr Lys Leu Thr Val Leu Gly
            100             105             110


<210>  53
<211>  121
<212>  PRT
<213>  Artificial sequence

<220>
<223>  artificial antibody-based sequence

<400>  53

Glu Val Gln Leu Val Glu Ser Gly Gly Gly Leu Val Gln Pro Gly Gly
1               5               10                  15


Ser Leu Arg Leu Ser Cys Ala Ala Ser Gly Phe Ser Phe Ser Ser Ser
            20              25              30


Tyr Trp Ile Cys Trp Val Arg Gln Ala Pro Gly Lys Cys Leu Glu Trp
            35              40                  45


Val Gly Cys Val Phe Thr Gly Asp Gly Thr Thr Tyr Tyr Ala Ser Trp
    50              55                  60


Ala Lys Gly Arg Phe Thr Ile Ser Arg Asp Asn Ser Lys Asn Thr Val
65                  70              75                  80


Tyr Leu Gln Met Asn Ser Leu Arg Ala Glu Asp Thr Ala Thr Tyr Phe
                85              90                  95


Cys Ala Arg Pro Val Ser Val Tyr Tyr Tyr Gly Met Asp Leu Trp Gly
            100             105             110


Gln Gly Thr Leu Val Thr Val Ser Ser
            115             120


<210>  54
<211>  110
<212>  PRT
<213>  Artificial sequence

<220>
<223>  artificial antibody-based sequence
```

<400> 54

Asp Val Val Met Thr Gln Ser Pro Ser Ser Leu Ser Ala Ser Val Gly
1               5                   10                  15

Asp Arg Val Thr Ile Thr Cys Gln Ala Ser Gln Ile Ile Ser Ser Arg
            20                  25                  30

Ser Ala Trp Tyr Gln Gln Lys Pro Gly Gln Pro Pro Lys Leu Leu Ile
        35                  40                  45

Tyr Gln Ala Ser Lys Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Cys Thr Tyr Ile Asp Ser Asn
            85                  90                  95

Phe Gly Ala Phe Gly Cys Gly Thr Lys Leu Thr Val Leu Gly
            100                 105                 110


<210> 55
<211> 510
<212> PRT
<213> Artificial sequence

<220>
<223> artificial antibody-based sequence

<400> 55

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asn Ile Tyr Ser Asn
            20                  25                  30

Leu Ala Trp Tyr Gln Gln Lys Pro Gly Lys Ala Pro Lys Leu Leu Ile
        35                  40                  45

Tyr Asp Ala Ser Asp Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Arg Ser Ser Ser Asp
            85                  90                  95

Ile Asp Asn Pro Phe Gly Thr Gly Thr Lys Val Thr Val Leu Gly Gly
100 105 110

Gly Gly Gly Ser Gly Gly Gly Gly Ser Gly Gly Gly Gly Ser Gly Gly
115 120 125

Gly Gly Ser Gln Val Gln Leu Val Glu Ser Gly Gly Gly Leu Val Gln
130 135 140

Pro Gly Gly Ser Leu Arg Leu Ser Cys Ala Ala Ser Gly Phe Ser Leu
145 150 155 160

Ser Ser Tyr Ala Met Gly Trp Val Arg Gln Ala Pro Gly Lys Gly Leu
165 170 175

Glu Trp Ile Gly Tyr Ile Ser Thr Ile Asn Asn Thr Tyr Tyr Ala Ser
180 185 190

Trp Ala Lys Gly Arg Phe Thr Ile Ser Arg Asp Asn Ser Lys Asn Thr
195 200 205

Val Tyr Leu Gln Met Asn Ser Leu Arg Ala Glu Asp Thr Ala Val Tyr
210 215 220

Tyr Cys Ala Arg Glu Ile Arg Ser Gly Trp Val Asp Tyr Gly Phe Ser
225 230 235 240

Ile Trp Gly Gln Gly Thr Leu Val Thr Val Ser Ser Gly Gly Gly Gly
245 250 255

Ser Gly Gly Gly Gly Ser Glu Val Gln Leu Val Glu Ser Gly Gly Gly
260 265 270

Leu Val Gln Pro Gly Gly Ser Leu Arg Leu Ser Cys Ala Ala Ser Gly
275 280 285

Phe Ser Leu Ser Ser Tyr Asp Met Ser Trp Val Arg Gln Ala Pro Gly
290 295 300

Lys Gly Leu Ala Trp Ile Gly Ala Ser Tyr Ala Ser Gly Pro Thr Tyr
305 310 315 320

Tyr Ala Ser Trp Ala Lys Gly Arg Phe Thr Ile Ser Arg Asp Asn Ser
325 330 335

Lys Asn Thr Val Tyr Leu Gln Met Asn Ser Leu Arg Ala Glu Asp Thr

69

Ala Thr Tyr Phe Cys Ala Arg Gly Gly Trp Thr Gly Thr Ser His Ser
                                360

Asn Ile Trp Gly Gln Gly Thr Leu Val Thr Val Ser Ser Gly Gly Gly
    370                     375                 380

Ser Gly Gly Gly Ser Glu Val Gln Leu Val Glu Ser Gly Gly Gly Leu
385                     390                 395                 400

Val Gln Pro Gly Gly Ser Leu Arg Leu Ser Cys Ala Ala Ser Gly Phe
                405                     410                 415

Ser Phe Ser Ser Ser Tyr Trp Ile Cys Trp Val Arg Gln Ala Pro Gly
            420                     425                 430

Lys Cys Leu Glu Trp Val Gly Cys Val Phe Thr Gly Asp Gly Thr Thr
            435                     440                 445

Tyr Tyr Ala Ser Trp Ala Lys Gly Arg Phe Thr Ile Ser Arg Asp Asn
    450                     455                 460

Ser Lys Asn Thr Val Tyr Leu Gln Met Asn Ser Leu Arg Ala Glu Asp
465                     470                 475                 480

Thr Ala Thr Tyr Phe Cys Ala Arg Pro Val Ser Val Tyr Tyr Tyr Gly
                485                     490                 495

Met Asp Leu Trp Gly Gln Gly Thr Leu Val Thr Val Ser Ser
            500                     505                 510

<210> 56
<211> 492
<212> PRT
<213> Artificial sequence

<220>
<223> artificial antibody-based sequence

<400> 56

Asp Ile Gln Met Thr Gln Ser Pro Ser Ser Leu Ser Ala Ser Val Gly
1                   5                   10                  15

Asp Arg Val Thr Ile Thr Cys Gln Ala Ser Gln Asn Ile Tyr Ser Asn
            20                      25                  30

Leu Ala Trp Tyr Gln Gln Lys Pro Gly Lys Ala Pro Lys Leu Leu Ile
        35                      40                  45

```
Tyr Asp Ala Ser Asp Leu Ala Ser Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Gln Gln Val Arg Ser Ser Ser Asp
            85              90              95

Ile Asp Asn Pro Phe Gly Thr Gly Thr Lys Val Thr Val Leu Gly Gly
        100             105             110

Gly Gly Gly Ser Gly Gly Gly Gly Ser Gly Gly Gly Gly Ser Gly Gly
    115             120             125

Gly Gly Ser Gln Val Gln Leu Val Glu Ser Gly Gly Gly Leu Val Gln
    130             135             140

Pro Gly Gly Ser Leu Arg Leu Ser Cys Ala Ala Ser Gly Phe Ser Leu
145             150             155             160

Ser Ser Tyr Ala Met Gly Trp Val Arg Gln Ala Pro Gly Lys Gly Leu
            165             170             175

Glu Trp Ile Gly Tyr Ile Ser Thr Ile Asn Asn Thr Tyr Tyr Ala Ser
        180             185             190

Trp Ala Lys Gly Arg Phe Thr Ile Ser Arg Asp Asn Ser Lys Asn Thr
    195             200             205

Val Tyr Leu Gln Met Asn Ser Leu Arg Ala Glu Asp Thr Ala Val Tyr
    210             215             220

Tyr Cys Ala Arg Glu Ile Arg Ser Gly Trp Val Asp Tyr Gly Phe Ser
225             230             235             240

Ile Trp Gly Gln Gly Thr Leu Val Thr Val Ser Ser Gly Gly Gly Gly
            245             250             255

Ser Gly Gly Gly Gly Ser Asp Val Val Met Thr Gln Ser Pro Ser Ser
            260             265             270

Leu Ser Ala Ser Val Gly Asp Arg Val Thr Ile Thr Cys Gln Ala Ser
    275             280             285

Gln Ile Ile Ser Ser Arg Ser Ala Trp Tyr Gln Gln Lys Pro Gly Gln
```

290                295                300

Pro Pro Lys Leu Leu Ile Tyr Gln Ala Ser Lys Leu Ala Ser Gly Val
305               310             315             320

Pro Ser Arg Phe Ser Gly Ser Gly Ser Gly Thr Asp Phe Thr Leu Thr
            325             330             335

Ile Ser Ser Leu Gln Pro Glu Asp Phe Ala Thr Tyr Tyr Cys Gln Cys
         340              345             350

Thr Tyr Ile Asp Ser Asn Phe Gly Ala Phe Gly Cys Gly Thr Lys Leu
         355              360             365

Thr Val Leu Gly Gly Gly Ser Gly Gly Ser Asp Ile Gln Met Thr Gln
         370              375             380

Ser Pro Ser Ser Leu Ser Ala Ser Val Gly Asp Arg Val Thr Ile Thr
385                390            395             400

Cys Gln Ser Ser Gln Ser Val Phe Ser Asn Asn Tyr Leu Ala Trp Phe
         405              410             415

Gln Gln Lys Pro Gly Gln Ser Pro Lys Arg Leu Ile Tyr Ser Ala Ser
         420              425             430

Thr Leu Ala Ser Gly Val Pro Ser Arg Phe Ser Gly Ser Gly Ser Gly
         435              440             445

Thr Asp Phe Thr Leu Thr Ile Ser Ser Leu Gln Pro Glu Asp Phe Ala
         450              455             460

Thr Tyr Tyr Cys Leu Gly Ser Tyr Ala Cys Ser Ser Ala Asp Cys Tyr
465                470            475             480

Val Phe Gly Thr Gly Thr Lys Val Thr Val Leu Gly
         485              490

```
<210>  57
<211>  510
<212>  PRT
<213>  Artificial sequence

<220>
<223>  artificial antibody-based sequence

<400>  57
```

Asp Ile Gln Met Thr Gln Ser Pro Ser Ser Leu Ser Ala Ser Val Gly
1          5             10             15

Asp Arg Val Thr Ile Thr Cys Gln Ala Ser Gln Asn Ile Tyr Ser Asn
        20              25              30

Leu Ala Trp Tyr Gln Gln Lys Pro Gly Lys Ala Pro Lys Leu Leu Ile
        35              40              45

Tyr Asp Ala Ser Asp Leu Ala Ser Gly Val Pro Ser Arg Phe Ser Gly
        50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Gln Gln Val Arg Ser Ser Ser Asp
                85              90              95

Ile Asp Asn Pro Phe Gly Thr Gly Thr Lys Val Thr Val Leu Gly Gly
        100             105             110

Gly Gly Gly Ser Gly Gly Gly Gly Ser Gly Gly Gly Ser Gly Gly
        115             120             125

Gly Gly Ser Gln Val Gln Leu Val Glu Ser Gly Gly Gly Leu Val Gln
        130             135             140

Pro Gly Gly Ser Leu Arg Leu Ser Cys Ala Ala Ser Gly Phe Ser Leu
145             150             155             160

Ser Ser Tyr Ala Met Gly Trp Val Arg Gln Ala Pro Gly Lys Gly Leu
                165             170             175

Glu Trp Ile Gly Tyr Ile Ser Thr Ile Ala Asn Thr Tyr Tyr Ala Ser
        180             185             190

Trp Ala Lys Gly Arg Phe Thr Ile Ser Arg Asp Asn Ser Lys Asn Thr
        195             200             205

Val Tyr Leu Gln Met Asn Ser Leu Arg Ala Glu Asp Thr Ala Val Tyr
210             215             220

Tyr Cys Ala Arg Glu Ile Arg Ser Gly Trp Val Asp Tyr Gly Phe Ser
225             230             235             240

Ile Trp Gly Gln Gly Thr Leu Val Thr Val Ser Ser Gly Gly Gly Gly
                245             250             255

Ser Gly Gly Gly Gly Ser Glu Val Gln Leu Val Glu Ser Gly Gly Gly

73

```
                   260                    265                      270


        Leu Val Gln Pro Gly Gly Ser Leu Arg Leu Ser Cys Ala Ala Ser Gly
                275                 280                 285


        Phe Ser Leu Ser Ser Tyr Asp Met Ser Trp Val Arg Gln Ala Pro Gly
                290                 295                 300


        Lys Gly Leu Ala Trp Ile Gly Ala Ser Tyr Ala Ser Gly Pro Thr Tyr
        305                 310                 315                 320


        Tyr Ala Ser Trp Ala Lys Gly Arg Phe Thr Ile Ser Arg Asp Asn Ser
                        325                 330                 335


        Lys Asn Thr Val Tyr Leu Gln Met Asn Ser Leu Arg Ala Glu Asp Thr
                    340                 345                 350


        Ala Thr Tyr Phe Cys Ala Arg Gly Gly Trp Thr Gly Thr Ser His Ser
                355                 360                 365


        Asn Ile Trp Gly Gln Gly Thr Leu Val Thr Val Ser Ser Gly Gly Gly
                370                 375                 380


        Ser Gly Gly Gly Ser Glu Val Gln Leu Val Glu Ser Gly Gly Gly Leu
        385                 390                 395                 400


        Val Gln Pro Gly Gly Ser Leu Arg Leu Ser Cys Ala Ala Ser Gly Phe
                        405                 410                 415


        Ser Phe Ser Ser Ser Tyr Trp Ile Cys Trp Val Arg Gln Ala Pro Gly
                        420                 425                 430


        Lys Cys Leu Glu Trp Val Gly Cys Val Phe Thr Gly Asp Gly Thr Thr
                435                 440                 445


        Tyr Tyr Ala Ser Trp Ala Lys Gly Arg Phe Thr Ile Ser Arg Asp Asn
                450                 455                 460


        Ser Lys Asn Thr Val Tyr Leu Gln Met Asn Ser Leu Arg Ala Glu Asp
        465                 470                 475                 480


        Thr Ala Thr Tyr Phe Cys Ala Arg Pro Val Ser Val Tyr Tyr Tyr Gly
                        485                 490                 495


        Met Asp Leu Trp Gly Gln Gly Thr Leu Val Thr Val Ser Ser
                    500                 505                 510
```

74

```
<210>   58
<211>   492
<212>   PRT
<213>   Artificial sequence

<220>
<223>   artificial antibody-based sequence

<400>   58

Asp Ile Gln Met Thr Gln Ser Pro Ser Ser Leu Ser Ala Ser Val Gly
1               5                   10                  15


Asp Arg Val Thr Ile Thr Cys Gln Ala Ser Gln Asn Ile Tyr Ser Asn
            20                  25                  30


Leu Ala Trp Tyr Gln Gln Lys Pro Gly Lys Ala Pro Lys Leu Leu Ile
        35                  40                  45


Tyr Asp Ala Ser Asp Leu Ala Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Val Arg Ser Ser Ser Asp
            85                  90                  95


Ile Asp Asn Pro Phe Gly Thr Gly Thr Lys Val Thr Val Leu Gly Gly
        100                 105                 110


Gly Gly Gly Ser Gly Gly Gly Gly Ser Gly Gly Gly Gly Ser Gly Gly
        115                 120                 125


Gly Gly Ser Gln Val Gln Leu Val Glu Ser Gly Gly Gly Leu Val Gln
        130                 135                 140


Pro Gly Gly Ser Leu Arg Leu Ser Cys Ala Ala Ser Gly Phe Ser Leu
145                 150                 155                 160


Ser Ser Tyr Ala Met Gly Trp Val Arg Gln Ala Pro Gly Lys Gly Leu
                165                 170                 175


Glu Trp Ile Gly Tyr Ile Ser Thr Ile Ala Asn Thr Tyr Tyr Ala Ser
            180                 185                 190


Trp Ala Lys Gly Arg Phe Thr Ile Ser Arg Asp Asn Ser Lys Asn Thr
        195                 200                 205


Val Tyr Leu Gln Met Asn Ser Leu Arg Ala Glu Asp Thr Ala Val Tyr
```

```
                210                      215                      220


        Tyr Cys Ala Arg Glu Ile Arg Ser Gly Trp Val Asp Tyr Gly Phe Ser
        225                 230                 235                 240


        Ile Trp Gly Gln Gly Thr Leu Val Thr Val Ser Ser Gly Gly Gly Gly
                        245                 250                 255


        Ser Gly Gly Gly Gly Ser Asp Val Val Met Thr Gln Ser Pro Ser Ser
                        260                 265                 270


        Leu Ser Ala Ser Val Gly Asp Arg Val Thr Ile Thr Cys Gln Ala Ser
                        275                 280                 285


        Gln Ile Ile Ser Ser Arg Ser Ala Trp Tyr Gln Gln Lys Pro Gly Gln
                290                 295                 300


        Pro Pro Lys Leu Leu Ile Tyr Gln Ala Ser Lys Leu Ala Ser Gly Val
        305                 310                 315                 320


        Pro Ser Arg Phe Ser Gly Ser Gly Ser Gly Thr Asp Phe Thr Leu Thr
                        325                 330                 335


        Ile Ser Ser Leu Gln Pro Glu Asp Phe Ala Thr Tyr Tyr Cys Gln Cys
                        340                 345                 350


        Thr Tyr Ile Asp Ser Asn Phe Gly Ala Phe Gly Cys Gly Thr Lys Leu
                355                 360                 365


        Thr Val Leu Gly Gly Gly Ser Gly Gly Ser Asp Ile Gln Met Thr Gln
                370                 375                 380


        Ser Pro Ser Ser Leu Ser Ala Ser Val Gly Asp Arg Val Thr Ile Thr
        385                 390                 395                 400


        Cys Gln Ser Ser Gln Ser Val Phe Ser Asn Asn Tyr Leu Ala Trp Phe
                        405                 410                 415


        Gln Gln Lys Pro Gly Gln Ser Pro Lys Arg Leu Ile Tyr Ser Ala Ser
                        420                 425                 430


        Thr Leu Ala Ser Gly Val Pro Ser Arg Phe Ser Gly Ser Gly Ser Gly
                435                 440                 445


        Thr Asp Phe Thr Leu Thr Ile Ser Ser Leu Gln Pro Glu Asp Phe Ala
                450                 455                 460
```

```
Thr Tyr Tyr Cys Leu Gly Ser Tyr Ala Cys Ser Ser Ala Asp Cys Tyr
465             470             475                     480


Val Phe Gly Thr Gly Thr Lys Val Thr Val Leu Gly
                485             490
```

```
<210>   59
<211>   756
<212>   PRT
<213>   Artificial sequence

<220>
<223>   artificial antibody-based sequence

<400>   59
```

```
Asp Val Val Met Thr Gln Ser Pro Ser Ser Leu Ser Ala Ser Val Gly
1               5               10                      15


Asp Arg Val Thr Ile Thr Cys Gln Ala Ser Gln Ile Ile Ser Ser Arg
            20              25                      30


Ser Ala Trp Tyr Gln Gln Lys Pro Gly Gln Pro Pro Lys Leu Leu Ile
            35              40                      45


Tyr Gln Ala Ser Lys Leu Ala Ser Gly Val Pro Ser Arg Phe Ser Gly
    50              55                      60


Ser Gly Ser Gly Thr Asp Phe Thr Leu Thr Ile Ser Ser Leu Gln Pro
65                  70              75                      80


Glu Asp Phe Ala Thr Tyr Tyr Cys Gln Cys Thr Tyr Ile Asp Ser Asn
                85              90                      95


Phe Gly Ala Phe Gly Cys Gly Thr Lys Leu Thr Val Leu Gly Gly Gly
                100             105                     110


Gly Gly Ser Glu Val Gln Leu Val Glu Ser Gly Gly Gly Leu Val Gln
            115             120                     125


Pro Gly Gly Ser Leu Arg Leu Ser Cys Ala Ala Ser Gly Phe Ser Leu
    130             135                     140


Ser Ser Tyr Asp Met Ser Trp Val Arg Gln Ala Pro Gly Lys Gly Leu
145             150                     155                     160


Ala Trp Ile Gly Ala Ser Tyr Ala Ser Gly Pro Thr Tyr Tyr Ala Ser
                165             170                     175


Trp Ala Lys Gly Arg Phe Thr Ile Ser Arg Asp Asn Ser Lys Asn Thr
```

                    180                          185                              190


Val Tyr Leu Gln Met Asn Ser Leu Arg Ala Glu Asp Thr Ala Thr Tyr
        195                 200                 205


Phe Cys Ala Arg Gly Gly Trp Thr Gly Thr Ser His Ser Asn Ile Trp
        210                 215                 220


Gly Gln Gly Thr Leu Val Thr Val Ser Ser Gly Gly Gly Ser Gly
225                 230                 235                 240


Gly Gly Gly Ser Gly Gly Gly Gly Ser Gly Gly Gly Ser Asp Ile
                245                 250                 255


Gln Met Thr Gln Ser Pro Ser Ser Leu Ser Ala Ser Val Gly Asp Arg
                260                 265                 270


Val Thr Ile Thr Cys Gln Ser Ser Gln Ser Val Phe Ser Asn Asn Tyr
            275                 280                 285


Leu Ala Trp Phe Gln Gln Lys Pro Gly Gln Ser Pro Lys Arg Leu Ile
        290                 295                 300


Tyr Ser Ala Ser Thr Leu Ala Ser Gly Val Pro Ser Arg Phe Ser Gly
305                 310                 315                 320


Ser Gly Ser Gly Thr Asp Phe Thr Leu Thr Ile Ser Ser Leu Gln Pro
                325                 330                 335


Glu Asp Phe Ala Thr Tyr Tyr Cys Leu Gly Ser Tyr Ala Cys Ser Ser
                340                 345                 350


Ala Asp Cys Tyr Val Phe Gly Thr Gly Thr Lys Val Thr Val Leu Gly
            355                 360                 365


Gly Gly Gly Gly Ser Glu Val Gln Leu Val Glu Ser Gly Gly Gly Leu
        370                 375                 380


Val Gln Pro Gly Gly Ser Leu Arg Leu Ser Cys Ala Ala Ser Gly Phe
385                 390                 395                 400


Ser Phe Ser Ser Ser Tyr Trp Ile Cys Trp Val Arg Gln Ala Pro Gly
                405                 410                 415


Lys Cys Leu Glu Trp Val Gly Cys Val Phe Thr Gly Asp Gly Thr Thr
        420                 425                 430

Tyr Tyr Ala Ser Trp Ala Lys Gly Arg Phe Thr Ile Ser Arg Asp Asn
435            440            445

Ser Lys Asn Thr Val Tyr Leu Gln Met Asn Ser Leu Arg Ala Glu Asp
450            455            460

Thr Ala Thr Tyr Phe Cys Ala Arg Pro Val Ser Val Tyr Tyr Tyr Gly
465            470            475            480

Met Asp Leu Trp Gly Gln Gly Thr Leu Val Thr Val Ser Ser Gly Gly
485            490            495

Gly Gly Ser Gly Gly Gly Gly Ser Asp Ile Gln Met Thr Gln Ser Pro
500            505            510

Ser Ser Leu Ser Ala Ser Val Gly Asp Arg Val Thr Ile Thr Cys Gln
515            520            525

Ala Ser Glu Ser Ile Gly Asn Tyr Leu Ala Trp Tyr Gln Gln Lys Pro
530            535            540

Gly Lys Ala Pro Lys Leu Leu Ile Tyr Ser Ala Ser Thr Leu Ala Ser
545            550            555            560

Gly Val Pro Ser Arg Phe Ser Gly Ser Gly Ser Gly Thr Asp Phe Thr
565            570            575

Leu Thr Ile Ser Ser Leu Gln Pro Glu Asp Phe Ala Thr Tyr Tyr Cys
580            585            590

Gln Ser Thr Asp Tyr Gly Asp Ser Tyr Ile Phe Gly Thr Gly Thr Lys
595            600            605

Val Thr Val Leu Gly Gly Gly Gly Gly Ser Gly Gly Gly Gly Ser Gly
610            615            620

Gly Gly Gly Ser Gly Gly Gly Gly Ser Glu Val Gln Leu Val Glu Ser
625            630            635            640

Gly Gly Gly Leu Val Gln Pro Gly Gly Ser Leu Arg Leu Ser Cys Ala
645            650            655

Ala Ser Gly Ile Ser Val Ser Asn Asp Tyr Tyr Met Cys Trp Val Arg
660            665            670

Gln Ala Pro Gly Lys Gly Leu Glu Trp Ile Gly Cys Ile Ser Thr Tyr
675            680            685

```
Ile Gly Asn Thr His Tyr Ala Ser Trp Ala Lys Gly Arg Phe Thr Ile
    690                 695             700

Ser Arg Asp Asn Ser Lys Asn Thr Val Tyr Leu Gln Met Asn Ser Leu
705             710             715                 720

Arg Ala Glu Asp Thr Ala Val Tyr Tyr Cys Ala Lys Asn Ala Gly Tyr
                725             730             735

Pro Gly Tyr Arg Tyr Ala Ile Asp Leu Trp Gly Gln Gly Thr Leu Val
            740             745             750

Thr Val Ser Ser
        755


<210>  60
<211>  20
<212>  PRT
<213>  Artificial sequence

<220>
<223>  artificial antibody-based sequence

<400>  60

Gly Gly Gly Gly Ser Gly Gly Gly Gly Ser Gly Gly Gly Gly Ser Gly
1               5                   10                  15

Gly Gly Gly Ser
            20


<210>  61
<211>  5
<212>  PRT
<213>  Artificial sequence

<220>
<223>  artificial antibody-based sequence

<400>  61

Gly Gly Gly Gly Ser
1               5


<210>  62
<211>  3
<212>  PRT
<213>  Artificial sequence

<220>
<223>  artificial antibody-based sequence


<220>
```

```
<221>   Repeat
<222>   (1)..(3)
<223>   generic linker (GmS)n, wherein n is 2, 3, 4, 5 or 6

<220>
<221>   Repeat
<222>   (2)..(2)
<223>   generic linker (GmS)n, wherein m is 2, 3 or 4 (residue 2 in
        sequence listing is present 1, 2 or 3 times)


<400>   62

Gly Gly Ser
1



<210>   63
<211>   122
<212>   PRT
<213>   Artificial sequence

<220>
<223>   artificial antibody-based sequence

<400>   63

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Phe Ser Ala Asn
            20                  25                  30


Tyr Tyr Pro Cys Trp Val Arg Gln Ala Pro Gly Lys Gly Leu Glu Trp
        35                  40                  45


Ile Gly Cys Ile Tyr Gly Gly Ser Ser Asp Ile Thr Tyr Asp Ala Asn
    50                  55                  60


Trp Thr Lys Gly Arg Phe Thr Ile Ser Arg Asp Asn Ser Lys Asn Thr
65                  70                  75                  80


Val Tyr Leu Gln Met Asn Ser Leu Arg Ala Glu Asp Thr Ala Val Tyr
                85                  90                  95


Tyr Cys Ala Arg Ser Ala Trp Tyr Ser Gly Trp Gly Gly Asp Leu Trp
            100                 105                 110


Gly Gln Gly Thr Leu Val Thr Val Ser Ser
            115                 120


<210>   64
<211>   124
<212>   PRT
<213>   Artificial sequence
```

<220>
<223>  artificial antibody-based sequence

<400>  64

Gln Val Gln Leu Gln Glu Ser Gly Pro Gly Leu Val Lys Pro Ser Glu
1                   5                   10                  15


Thr Leu Ser Leu Thr Cys Lys Val Ser Gly Phe Ser Phe Ser Asn Ser
            20                  25                  30


Tyr Trp Ile Cys Trp Ile Arg Gln Pro Pro Gly Lys Gly Leu Glu Trp
        35                  40                  45


Ile Gly Cys Thr Phe Val Gly Ser Ser Asp Ser Thr Tyr Tyr Ala Asn
        50                  55                  60


Trp Ala Lys Gly Arg Val Thr Ile Ser Val Asp Ser Ser Lys Asn Gln
65                  70                  75                  80


Phe Ser Leu Lys Leu Ser Ser Val Thr Ala Ala Asp Thr Ala Val Tyr
                85                  90                  95


Tyr Cys Ala Arg His Pro Ser Asp Ala Val Tyr Gly Tyr Ala Asn Asn
            100                 105                 110


Leu Trp Gly Gln Gly Thr Leu Val Thr Val Ser Ser
            115                 120


<210>  65
<211>  108
<212>  PRT
<213>  Artificial sequence

<220>
<223>  artificial antibody-based sequence

<400>  65

Asp Ile Gln Met Thr Gln Ser Pro Ser Ser Leu Ser Ala Ser Val Gly
1                   5                   10                  15


Asp Arg Val Thr Ile Thr Cys Gln Ala Ser Gln Ser Ile Asn Asn Val
            20                  25                  30


Leu Ala Trp Tyr Gln Gln Lys Pro Gly Lys Ala Pro Lys Leu Leu Ile
        35                  40                  45


Tyr Arg Ala Ser Thr Leu Ala Ser Gly Val Pro Ser Arg Phe Ser Gly
        50                  55                  60

Ser Gly Ser Gly Thr Asp Phe Thr Leu Thr Ile Ser Ser Leu Gln Pro
65              70              75                  80

Glu Asp Phe Ala Thr Tyr Tyr Cys Gln Ser Ser Tyr Gly Asn Tyr Gly
                85              90                  95

Asp Phe Gly Thr Gly Thr Lys Val Thr Val Leu Gly
            100             105

<210> 66
<211> 11
<212> PRT
<213> Artificial sequence

<220>
<223> artificial antibody-based sequence

<400> 66

Phe Gly Thr Gly Thr Lys Val Thr Val Leu Gly
1               5                   10

<210> 67
<211> 11
<212> PRT
<213> Artificial sequence

<220>
<223> artificial antibody-based sequence

<400> 67

Phe Gly Gly Gly Thr Lys Leu Thr Val Leu Gly
1               5                   10

<210> 68
<211> 11
<212> PRT
<213> Artificial sequence

<220>
<223> artificial antibody-based sequence

<400> 68

Phe Gly Gly Gly Thr Gln Leu Ile Ile Leu Gly
1               5                   10

<210> 69
<211> 11
<212> PRT
<213> Artificial sequence

<220>
<223> artificial antibody-based sequence

<400> 69

Phe Gly Glu Gly Thr Glu Leu Thr Val Leu Gly
1               5                   10


<210>  70
<211>  11
<212>  PRT
<213>  Artificial sequence

<220>
<223>  artificial antibody-based sequence

<400>  70

Phe Gly Ser Gly Thr Lys Val Thr Val Leu Gly
1               5                   10


<210>  71
<211>  11
<212>  PRT
<213>  Artificial sequence

<220>
<223>  artificial antibody-based sequence

<400>  71

Phe Gly Gly Gly Thr Gln Leu Thr Val Leu Gly
1               5                   10


<210>  72
<211>  11
<212>  PRT
<213>  Artificial sequence

<220>
<223>  artificial antibody-based sequence

<400>  72

Phe Gly Gly Gly Thr Gln Leu Thr Ala Leu Gly
1               5                   10


<210>  73
<211>  11
<212>  PRT
<213>  Artificial sequence

<220>
<223>  artificial antibody-based sequence

<400>  73

Phe Gly Cys Gly Thr Lys Val Thr Val Leu Gly
1               5                   10